(19) Europäisches Patentamt / European Patent Office / Office européen des brevets

(11) **EP 4 484 422 A1**

(12) **EUROPEAN PATENT APPLICATION**


(43) Date of publication:
**01.01.2025 Bulletin 2025/01**

(21) Application number: **23181344.5**

(22) Date of filing: **26.06.2023**

(51) International Patent Classification (IPC):
***C07D 409/04*** *(2006.01)*

(52) Cooperative Patent Classification (CPC):
**C07D 409/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**



(71) Applicant: **Tallinn University of Technology**
**19086 Tallinn (EE)**

(72) Inventors:
- **AAV, Riina**
  **19086 Tallinn (EE)**
- **SUUT-TUULE, Elina**
  **19086 Talllinn (EE)**
- **JARG, Tatsiana**
  **19086 Tallinn (EE)**
- **NIKONOVICH, Tatsiana**
  **19086 Tallinn (EE)**
- **KANANOVICH, Dzmitry**
  **19086 Tallinn (EE)**
- **USTRNUL, Luka**
  **19086 Tallinn (EE)**

(74) Representative: **Koppel, Mart Enn**
**KOPPEL Patendibüroo OÜ**
**Kajaka 4-10**
**13418 Tallinn (EE)**


(54) **METHOD OF PREPARATION OF CHIMERIC HEMICUCURBIT[N]URILS, DERIVATIVES, AND USES THEREOF**


(57) The invention relates to a solvent free method for producing chimeric hemicucurbit[n]urils. The method of the invention comprises a first step of contacting, under mechanical agitation, at least two different cyclic urea derivatives and a compound that can form methylene bridges in the presence of an acid and templating anions and a second step of aging the products for at least about 30 minutes. The method has a very low process mass intensity (PMI) of 4, making it very attractive from a green chemistry perspective. New chimeric hemicucurbit[n]urils incorporating D-biotin are also described.


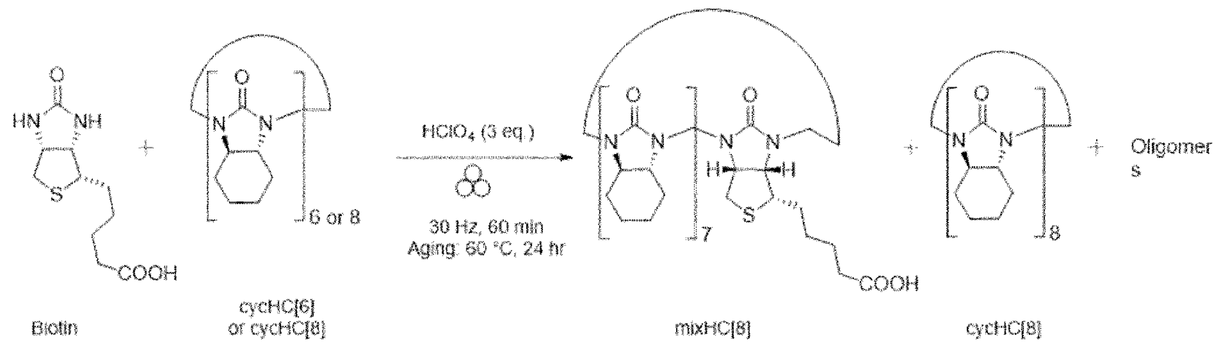


**Figure 1**




Processed by Luminess, 75001 PARIS (FR)

## Description

### *Field of the invention*

The present invention relates to the field of chemical sciences and more particularly to mechanochemistry. In particular, the invention relates to a solvent free method for producing chimeric hemicucurbit[n]urils.

### *Background of the invention*

Hemicucurbiturils are macrocycles usually composed of *N,N'*-(1,2-ethanediyl)-urea derivatives as monomers hinged together by methylene bridges via the nitrogen atoms of the *N*,*N'*-(1,2-ethanediyl)-urea monomers. They are well-known for their anion-binding capability and their solubility in non-polar solvents. They are usually synthesized in solution via an acid mediated, anion templated condensation reaction between the desired *N*,*N'*-(1,2-ethanediyl)-urea monomer and formaldehyde.

However, this classic method of production of hemicucurbiturils is far from ideal. Indeed, the products selectivity toward different homologues is limited, process demands harsh conditions like prolonged heating in concentrated of strong acid and the method has a high process-mass intensity, making it not environmentally friendly. It would thus be desirable to promote a different approach of hemicucurbiturils synthesis.

Mechanochemistry has recently emerged as a versatile, environmentally-friendly alternative to conventional chemical reactivity in solution. Besides offering a cleaner, safer way to conduct chemical transformations, mechanochemistry is also a route to discover new reactions and access materials or molecules that have been difficult to obtain. Indeed, at extreme concentrations in the solid state, when non-covalent interactions are stronger, the formation of products that are less favorable in solution becomes possible (Kwon, T. et al. J. Am. Chem. Soc. 2022, 144 (28), 12595-12601; Dračínský M. et al., Chemical Communications 2021, 57 (17), 2132-2135).

Despite recent advances in understanding the kinetics and thermodynamics of mechanochemical reactions, the factors directing the formation of complex molecular structures under mechanochemical conditions remain largely unexplored. The self-assembly of molecular species is based on the formation of non-covalent interactions and can lead to complex supramolecular aggregates. Molecular interactions also mediate the formation of covalent bonds and are essential for covalent self-assembly. Control over supramolecular self-assembly may be highly sensitive to small changes in external stimuli, resulting in the amplification of various products (Schnitzer, T. et al, Angewandte Chemie 2022, 134 (41)).

Solvent-free mechanochemistry has recently been used to assemble monomers in a one-pot reaction (Kaabel, S. et al., Angewandte Chemie International Edition 2019, 58 (19), 6230-6234). The size of the macrocycle is typically determined by external factors, such as anion templation (Kaabel, S. et al., Israel Journal of Chemistry 2018, 58 (3-4), 296-313). However, only some hemicucurbiturils macrocycles have been synthesized in the solid state via mechanochemistry (Kaabel, S. et al., Angewandte Chemie International Edition 2019, 58 (19), 6230-6234).

Besides their synthesis method, another limitation to a wider use of hemicucurbiturils, is their range of action. Indeed, they are capable of forming complexes only with a limited range of anions, which binding strength is determined by the shape and size of the hemicucurbituril cavity (Andersen, N. et al., Israel Journal of Chemistry 2018, 58 (3-4), 435-448). It would thus be particularly interesting to develop new hemicucurbiturils having a wider range of interaction with anions or being capable to form complexes with different anionic guests.

An interesting option to develop new hemicucurbiturils with such properties is to build macrocycles comprising different monomers. Recently, few teams have managed to synthetize oligomeric hemicucurbiturils incorporating non-uniform monomers (Zeng, Q. et al, Org. Chem. 2021, 17 (1), 2840-2847; Wang, L. et al., Tetrahedron Letters 2022, 101, 153918 Maršálek, K.; et al. Org. Lett. 2020, 22 (4), 1633-1637; De Simone et al. J. Org. Chem. 2022, 87 (15), 9829-9838). It has however been done in solution, utilizing multiple-step synthesis of particularly high process-mass intensity.

There is thus still a need to develop a new method of preparation of chimeric hemicucurbiturils in a simple, cost effective, environmentally friendly and efficient way. The present invention meets these and other needs.

### *Summary of the invention*

The inventors have successfully achieved a liquid assisted mechanochemically-activated, solid-state condensation reaction of (*R,R*)- or (*S*,*S*)-*N*,*N'*-cyclohexa-1,2-diylurea (CU), D-biotin, and formaldehyde. This is the first solid state formation of oligomeric macrocycles from a mixture of different monomers.

The inventors had to overcome negatives preconceptions in the field and quite intimidating odds to reach such an interesting result. Indeed, non-uniform oligomeric macrocycles formation in a single-step of a solvent-free mechanochemically aided synthesis produces a plethora of linear and cyclic intermediates, as well as many side-products. To give an idea, when performing a solvent free reaction with only 1 monomer, only one possible 8-membered macrocycle can be

obtained. In contrast, when adding a second monomer, D-biotin for example, there is theoretically 498 possible 8-membered macrocycles (Weisstein, Eric W. "Necklace." From MathWorld--A Wolfram Web Resource. https://mathworld.wolfram.com/Necklace.html,; where n=8 and a=3 (1 possibility for positioning chiral and $C_2$ symmetric CU and 2 for positioning D-biotin)). When looking now at the number of different possible intermediates of linear and cyclic oligomers, the difference between the two reactions is even greater (ca 6000 combinations). The optimal combination of different starting monomers is governed by statistical probability, as well as chemical selectivity; thus, forcing the organization of a chaotic mixture into a well-organized macrocycle is extremely difficult.

This newly developed covalent self-assembly process was essentially solvent-free and with a remarkably low process mass intensity (PMI = 4), underlying that this single-pot and solvent-free strategy is very attractive from a green chemistry perspective. It is also worth noting that this solid-state self-assembly method is much more efficient than the traditional in solution approach. Indeed, the method of the invention has a yield of 37% when the comparative classic solution approach yield at only 10%.

As a result of this method, the inventors obtained new products, including two enantiopure chimeric mono-biotinylated hemicucurbit[8]urils: (-)-((*S*,*S*,*R*)(*R*,*R*)$_7$)-mono-biotinylated hemicucurbit[8]urils and its diastereoisomer (+)-((*S*,*S*,*R*)(*S*,*S*)$_7$)-mono-biotinylated hemicucurbit[8]urils. Interestingly, D-biotin was never shown to produce 8-membered hemicucurbiturils. It is thus very surprising that the inventors managed to introduce D-Biotin in a hemicucurbit[8]uril macrocycle.

Thus, in a first aspect, the invention relates to a method of preparation of at least one chimeric hemicucurbit[n]uril, comprising the following steps:

- Contacting, under mechanical agitation, at least two different cyclic urea derivatives and a compound that can form methylene bridges in the presence of at least one acid, and at least one solvent;
- Aging the products obtained at the previous step for at least about 30 minutes;

wherein n is an even positive integer comprised between 4 and 14;
wherein the amount of liquid per amount of solid in the reaction media is initially < 1 μL/mg.

In a preferred embodiment, said at least two different cyclic urea derivatives are *N,N'*-(1,2-ethanediyl)-urea derivatives according to formula (I):

(I) 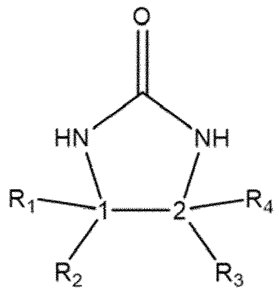

wherein 1 and 2 are carbon atoms and at least one of them is a stereogenic center, preferably both of them are stereogenic centers; wherein at least one of the substituents R1 to R4 bears a stereogenic center; and wherein $R_1$, $R_2$, $R_3$, and $R_4$ can each independently be selected from the group consisting in a hydrogen atom, -OH, -COOH, $-NH_2$, $-NO_2$, $-NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl.

Preferably, said at least two different cyclic urea derivatives are selected from the group consisting in 4,5-Dimethyl-2-imidazolidinone, 4-alkyl-2-imidazolidinone, 2-imidazolidinone, rel-(3aR,6aS)-Hexahydropyrrolo[3,4-d]imidazol-2(1H)-one, 2-Oxo-4-imidazolidinecarboxylic acid, 2-Oxo-4-imidazolidinecarbonitrile, 4-(Aminomethyl)-2-imidazolidinone, 4-(Hydroxymethyl)-2-imidazolidinone, hexahydro-2(1H)-Cyclopentimidazolone, 4-(2-Hydroxyethyl)-2-imidazolidinone, rel-(3aR,6aS)-Tetrahydro-1H-furo[3,4-d]imidazol-2(3H)-one, Octahydroimidazo[4,5-d]azepin-2(1H)-one, 4-(Ethoxymethyl)-2-imidazolidinone, 4,5-Dipropyl-2-imidazolidinone, (4R or 4S)-4-Phenyl-2-imidazolidinone, (4S or 4R)-2-Oxo-4-imidazolidineacetic acid, 8-Methyl-1,3,7-triazaspiro[4.4]nonan-2-one, (4S or 4R)-4-(4-Pyridinyl)-2-imidazolidinone, 4,5-Dialkyl-2-imidazolidinone, 2-Oxo-4-imidazolidinepropanoic acid, 4-(4-Hydroxyphenyl)-2-imidazolidinone, 1H-Thieno[3,4-d]imidazol-2(3H)-one, tetrahydro-, 5,5-dioxide, (3aR,6aR)-Spiro[1-azabicyclo[2.2.2]octane-3,4'-imidazolidin]-2'-one, 2-[(4R)-2-Oxo-4-imidazolidinyl]benzonitrile, ethylene urea, *N,N'*-cyclohexa-1,2-diylurea, D-biotin, their stereoisomers thereof, and their derivatives thereof, more preferably the at least two different cyclic urea derivatives are selected from the group consisting in ethylene urea, *N,N'*-cyclohexa-1,2-diylurea, D-biotin, their stereoisomers thereof, and their derivatives thereof.

In another preferred embodiment, the method according to the invention comprises between 2 and 5 different cyclic urea derivatives, preferably 2 or 3 different cyclic urea derivatives, more preferably 2 different cyclic urea derivatives. Preferably, among the different cyclic urea derivatives, one is the main cyclic urea derivative and the initial ratio of the other cyclic urea derivatives on the main cyclic urea derivative is comprised between about 1/15 and about 1/5, preferably said ratio is of about 1/7.

In another preferred embodiment, one of the cyclic urea derivatives, preferably the main one, is (*S,S*)-*N,N'*-cyclohexa-1,2-diylurea or (*R,R*)-*N,N'*-cyclohexa-1,2-dylurea and the other cyclic urea derivative is D-biotin.

In a particularly preferred embodiment, n is an even positive integer comprised between 6 and 14 and the step of contacting further comprises at least one templating anion.

Preferably, said at least one templating anion is selected from the group consisting in $Cl^-$, $Br^-$, $I^-$, $BF_4^-$, $IO_4^-$, $ClO_4^-$, $ReO_4^-$, $PF_6^-$, $SbF_6^-$, $SO_4^{2-}$, $BO_3^{2-}$, $NO^{3-}$, $PO_3^{3-}$, $PO4^{2-}$, and $RCO_2^-$, R being selected from the group consisting in hydrogen atom, -OH, -COOH, $-NH_2$, $-NO_2$, $-NHNH_2$, nitrile, alkyl, fluoro-alkyl, bromo-alkyl, chloro-alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl, or a combination thereof, more preferably said at least one templating anion is $PF_6^-$.

The initial quantity of said at least one templating anion is preferably comprised between about 0.5 and about 6 eq, more preferably between about 1.2 and about 4 eq, even more preferably it is of about 3 eq.

The method of the invention comprises at least one acid and the initial quantity of said at least one acid is preferably comprised between about 0.5 and about 5 eq, more preferably between about 1.2 and about 4 eq, even more preferably it is of about 3 eq.

In a preferred embodiment, said at least one acid has a pK < 1, preferably said at least one acid is selected from the group consisting in HCl, HBr, HI, $H_2SO_4$, $H_3BO_3$, $HNO_3$, $H_3PO_3$, $H_3PO_4$, $HReO_4$, $HClO_4$, $HPF_6$, $HSbF_6$, $HBF_4$, $HIO_4$, RCOOH, R being selected from or RCOOH, R being selected from the group consisting in hydrogen atom, -OH, -COOH, $-NH_2$, $-NO_2$, $-NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl, or a combination thereof, more preferably said acid is $HPF_6$.

The step of contacting of the method of the invention may also take place in the presence of a salt. Preferably, said salt is selected from the list consisting in the combination of any templating anion as above described with a cation, and a combination thereof, more preferably said salt is selected from the list consisting in $AgPF_6$, $[Cu(CH_3CN)4]PF_6$, $KPF_6$, and a combination thereof, even more preferably said salt is $KPF_6$.

In a preferred embodiment, said at least one solvent according to the method of the invention is selected from the group consisting in apolar or polar solvents, such as alkanes, haloalkanes, arenes, heteroarenes, ethers, nitromethane, acetonitrile, solvent and water or a combination thereof, preferably said at least one solvent is water.

Preferably, the amount of liquid per amount of solid in the reaction media, $\eta$, is initially comprised between about 0.1 $\mu$L/mg and about 1 $\mu$L/mg, preferably between about 0.1 $\mu$L/mg and about 0.5 $\mu$L/mg, more preferably $\eta$ is of about 0.2 $\mu$L/mg.

Preferably $\eta$ remains comprised between about 0.1 $\mu$L/mg and about 1 $\mu$L/mg, preferably between about 0.1 $\mu$L/mg and about 0.5 $\mu$L/mg, more preferably $\eta$ is of about 0.2 $\mu$L/mg during the full length of the method of the invention, including the first step of contacting and the second step of aging.

The compound that can form methylene bridges according to the method of the invention is preferably selected from the group consisting in formaldehyde, paraformaldehyde, formalin, trioxane, or alkenes such as ethene or isoprene, or a combination thereof, more preferably the compound that can form methylene bridges is formaldehyde. The initial ratio between the compound that can form methylene bridges and the at least two cyclic urea derivatives is preferably comprised between about 0.5 and about 2, more preferably between about 0.75 and about 1.5, and even more preferably said ratio is of about 1.

In a preferred embodiment, the contacting step according to the method of the invention last at least about 10 min, more preferably between about 15 min and about 90 min, even more preferably about 20 min.

In a particular embodiment, the frequency of the mechanical agitation of the contacting step according to the method of the invention is comprised between about 10 Hertz and about 50 Hertz in a ball milling, preferably between about 20 Hertz and 40 Hertz in a ball milling, even more preferably about 30 Hertz in a ball milling.

In a preferred embodiment, the aging step according to the method of the invention last at least about 1 h, preferably at least about 1 h 30, more preferably between about 2 h and about 24 h, still preferably between about 2 h 30 and about 16 h, even more preferably between about 3 h and about 12 h.

In another preferred embodiment, said aging step takes place at a temperature comprised between about 35°C and about 75°C, preferably between about 40°C and about 65°C, even more preferably at a temperature of about 60°C.

In still another preferred embodiment, said at least one chimeric hemicucurbit[n]uril is obtained at a yield of at least about 10%, preferably of at least about 20%, more preferably at a yield of at least about 30%, even more preferably at a yield of at least about 35%.

In a second aspect, the invention also relates to a chimeric hemicucurbit[n]uril obtained by the method according to above-described the first aspect of the invention.

In a third aspect, the invention still relates to a chimeric hemicucurbit[n]uril comprising at least two different cyclic urea monomers, wherein n is an even positive integer comprised between 4 and 14 and wherein the at least two different cyclic urea monomers are *N*,*N*'-(1,2-ethanediyl)-urea derivatives according to formula (I):

(I)

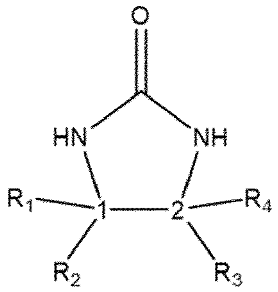

wherein 1 and 2 are carbon atoms and at least one of them is a stereogenic center, preferably both of them are stereogenic centers; wherein at least one of the substituents R1 to R4 bears a stereogenic center; and wherein $R_1$, $R_2$, $R_3$, and $R_4$ can each independently be selected from the group consisting in a hydrogen atom, -OH, -COOH, $-NH_2$, $-NO_2$, $-NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl.

Preferably, said at least two different cyclic urea derivatives are selected from the group consisting in 4,5-Dimethyl-2-imidazolidinone, 4-alkyl-2-imidazolidinone, 2-imidazolidinone, rel-(3aR,6aS)-Hexahydropyrrolo[3,4-d]imidazol-2(1H)-one, 2-Oxo-4-imidazolidinecarboxylic acid, 2-Oxo-4-imidazolidinecarbonitrile, 4-(Aminomethyl)-2-imidazolidinone, 4-(Hydroxymethyl)-2-imidazolidinone, hexahydro-2(1H)-Cyclopentimidazolone, 4-(2-Hydroxyethyl)-2-imidazolidinone, rel-(3aR,6aS)-Tetrahydro-1H-furo[3,4-d]imidazol-2(3H)-one, Octahydroimidazo[4,5-d]azepin-2(1H)-one, 4-(Ethoxymethyl)-2-imidazolidinone, 4,5-Dipropyl-2-imidazolidinone, (4R or 4S)-4-Phenyl-2-imidazolidinone, (4S or 4R)-2-Oxo-4-imidazolidineacetic acid, 8-Methyl-1,3,7-triazaspiro[4.4]nonan-2-one, (4S or 4R)-4-(4-Pyridinyl)-2-imidazolidinone, 4,5-Dialkyl-2-imidazolidinone, 2-Oxo-4-imidazolidinepropanoic acid, 4-(4-Hydroxyphenyl)-2-imidazolidinone, 1H-Thieno[3,4-d]imidazol-2(3H)-one, tetrahydro-, 5,5-dioxide, (3aR,6aR)-Spiro[1-azabicyclo[2.2.2]octane-3,4'-imidazolidin]-2'-one, 2-[(4R)-2-Oxo-4-imidazolidinyl]benzonitrile, ethylene urea, *N*,*N*'-cyclohexa-1,2-diylurea, D-biotin, their stereoisomers thereof, and their derivatives thereof, more preferably said at least two different cyclic urea derivatives are selected from the group consisting in ethylene urea, *N,N'*-cyclohexa-1,2-diylurea, D-biotin, their stereoisomers thereof, and their derivatives thereof.

In a preferred embodiment, the chimeric hemicucurbit[n]uril according to the invention comprises between 2 and 5 different cyclic urea monomers, more preferably 2 or 3 different cyclic urea monomers, even more preferably 2 different cyclic urea monomers.

In a particular embodiment, one of the cyclic urea monomers of the chimeric hemicucurbit[n]uril according to the invention is the main cyclic urea monomer and the ratio of the other cyclic urea monomers on the main cyclic urea monomer is comprised between about 1/14 and about ½, preferably said ratio is of about 1/8.

In a preferred embodiment, the chimeric hemicucurbit[n]uril according to the invention comprises 2 different cyclic urea monomers, one being (*S,S*)-*N,N'*-cyclohexa-1,2-diylurea or (*R*,*R*)-*N*,*N*'-cyclohexa-1,2-dylurea and the other one D-biotin.

In another preferred embodiment, the chimeric hemicucurbit[n]uril according to the invention is according to one of the following formulas, the different monomers present in the cycle of said formula being in any order:

(II) $-[-CH_2-(\text{Cyclic urea derivative 1})_a-CH_2-(\text{Cyclic urea derivative 2})_b-CH_2-]-$

wherein a + b = n;

(III) $-[-CH_2-(\text{ Cyclic urea derivative 1})_a-CH_2-(\text{ Cyclic urea derivative 2})_b-CH_2-]-$

wherein a + b = 6;

(IV) $-[-CH_2-(\text{ Cyclic urea derivative 1})_a-CH_2-(\text{ Cyclic urea derivative 2})_b-CH_2-]-$

wherein a + b = 8;

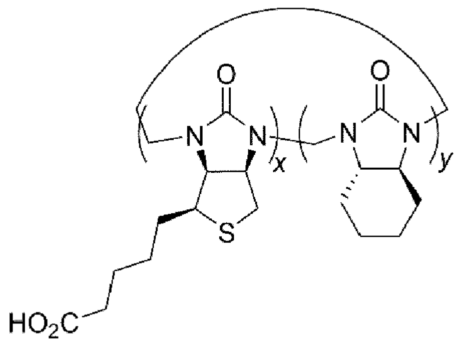

(VIII)

wherein x + y = 6;

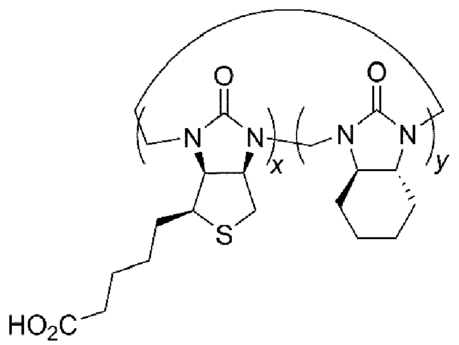

(IX)

wherein x + y = 6;

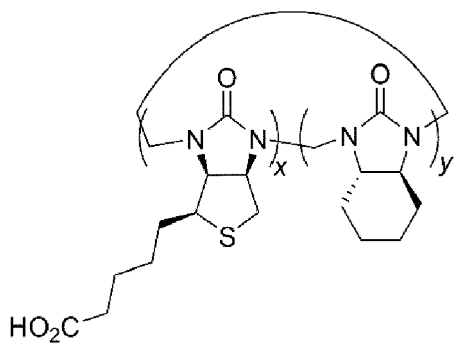

(X)

wherein x + y = 8;

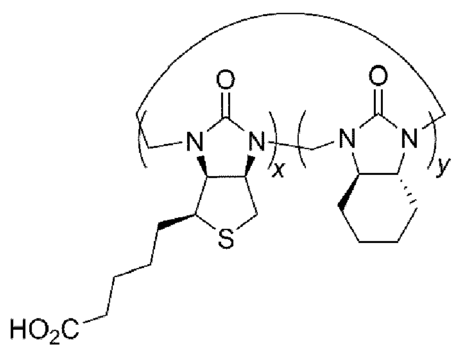

(XI)

wherein x + y = 8;

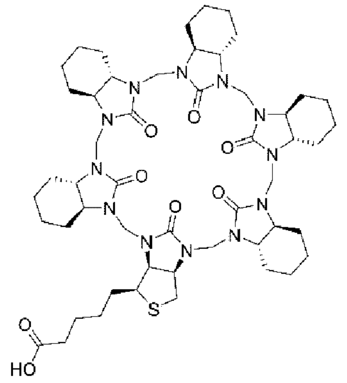

(XII) ;

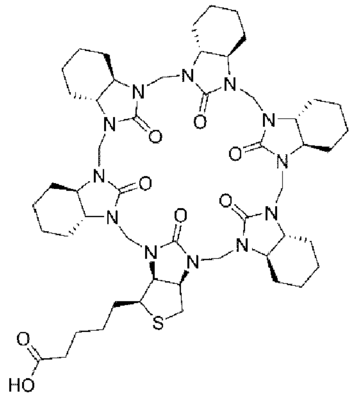

(XIII) ;

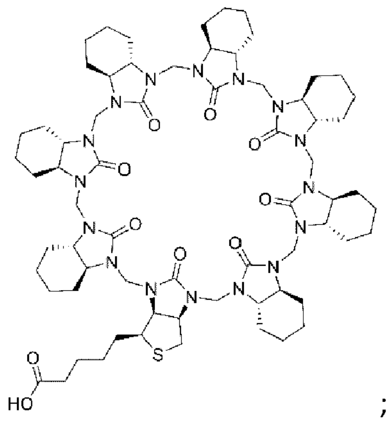

(XIV) ;

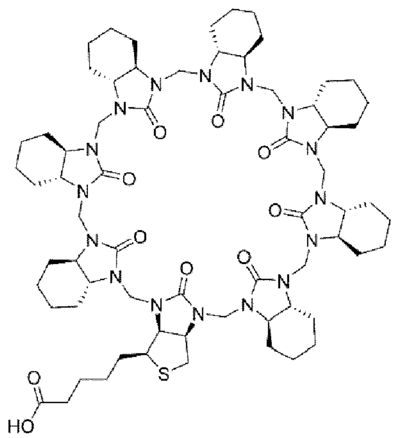

(XV) ;

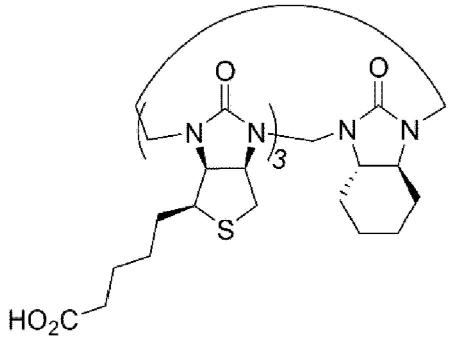

(XVI) ;

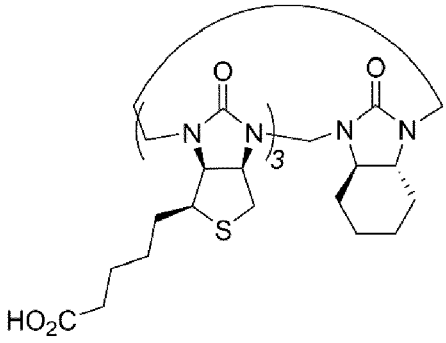

(XVII) ;

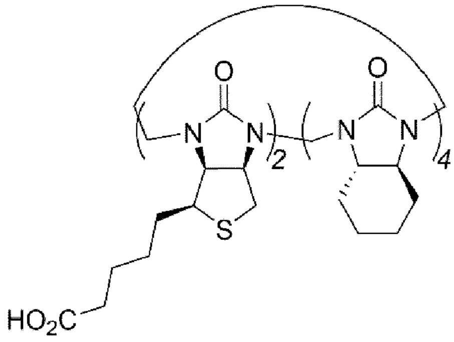

(XVIII) ;

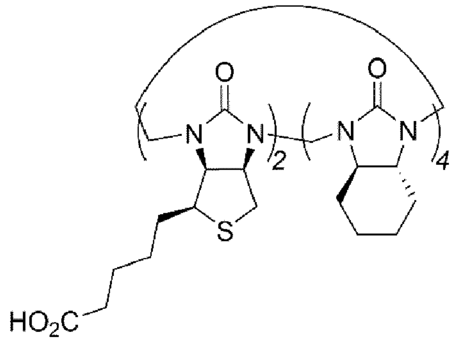

(XIX) ;

(XX) 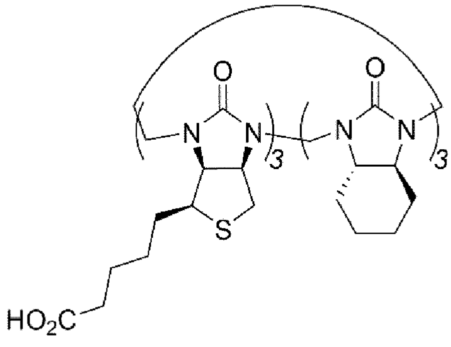 ;

(XXI) 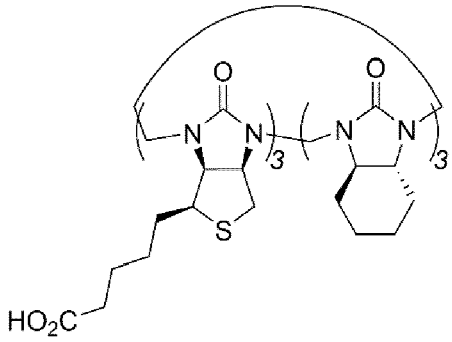 ;

(XXII) 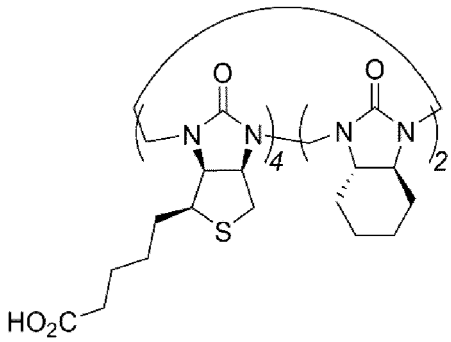 ;

(XXIII) 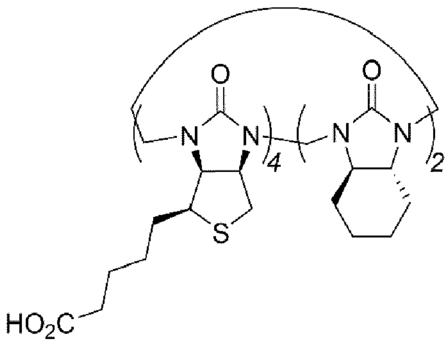 ;

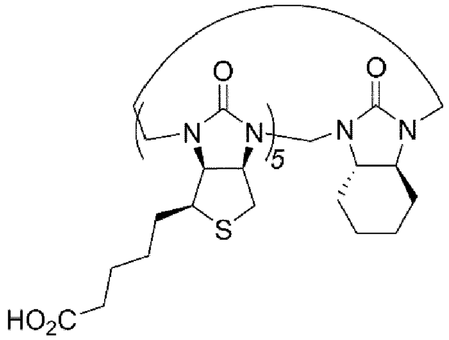

(XXIV) ;

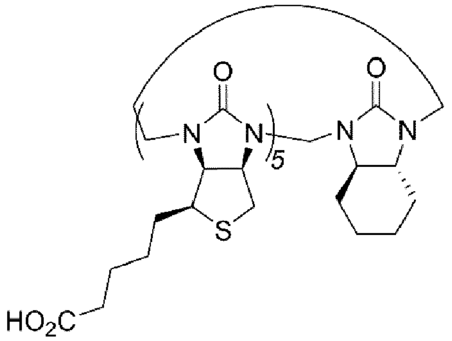

(XXV) ;

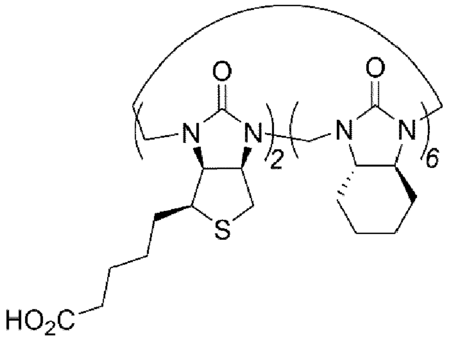

(XXVI) ;

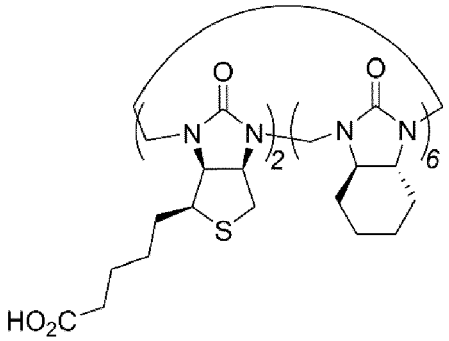

(XXVII) ;

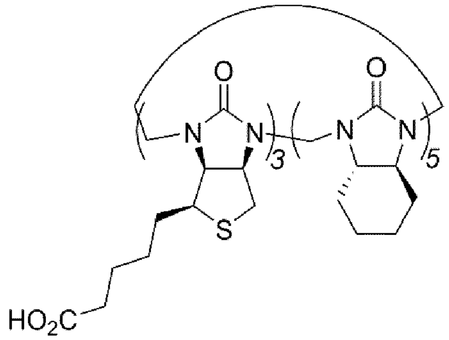

(XXVIII) ;

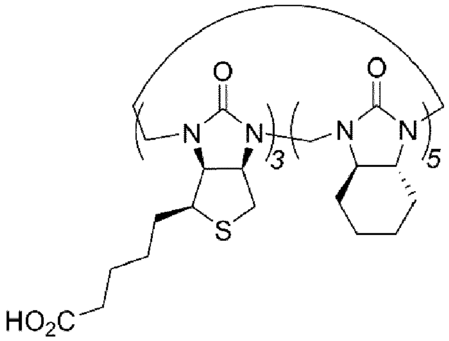

(XXIX) ;

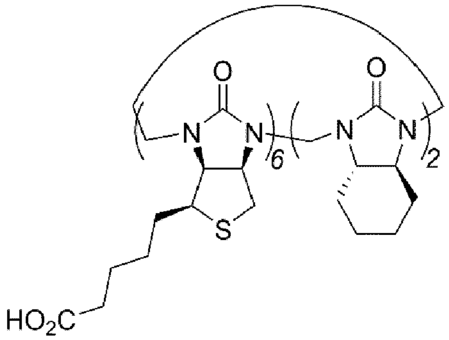

(XXX) ;

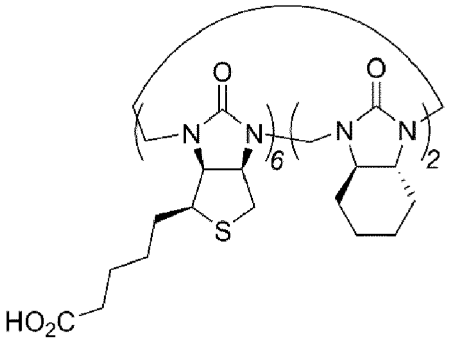

(XXXI) ;

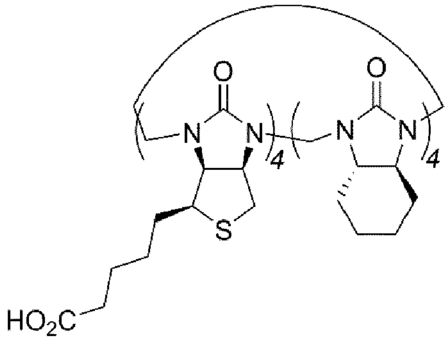

(XXXII) ;

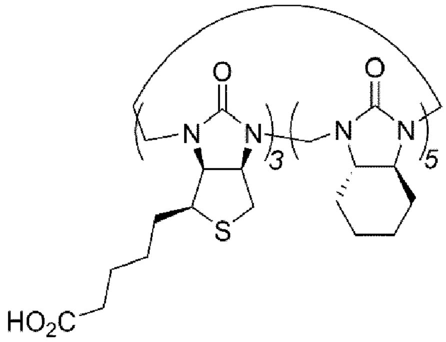

(XXXIV) ;

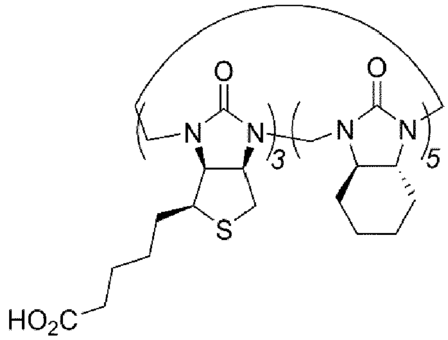

(XXXV) ;

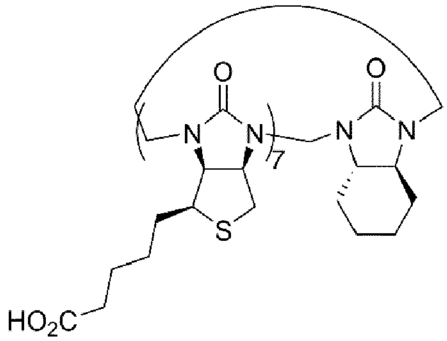

(XXXVI) ;

or

(XXXVII) 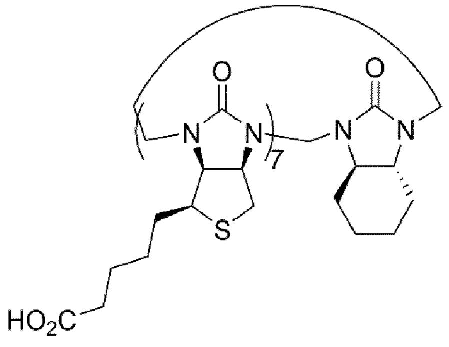 .

In a very preferred embodiment, the chimeric hemicucurbit[n]uril according to the invention is the (-)-((*S*,*S*,*R*)(*R*,*R*)$_7$)-mono-biotinylated cyclohexanohemicucurbit[8]uril according to the following formula:

(XIV) 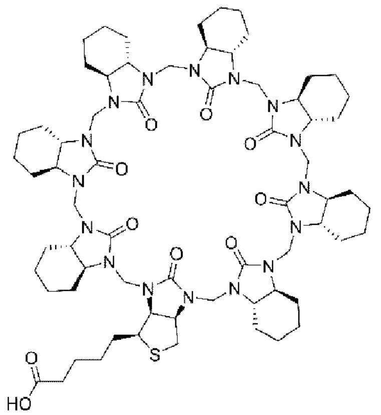

In another very preferred embodiment, the chimeric hemicucurbit[n]uril according to the invention is the (+)-((*S*,*S*,*R*)(*S*,*S*)$_7$)-mono-biotinylated cyclohexanohemicucurbit[8]uril according to the following formula:

(XV) 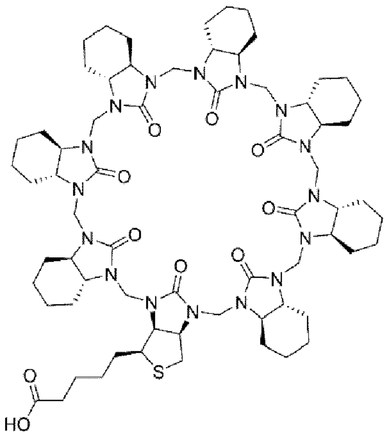

In a particular embodiment, the chimeric hemicucurbit[n]uril according to the invention comprises at least one additional group attached to at least one of its monomers, preferably D-biotin monomers, more preferably the carboxylic side chain of D-biotin monomers. Said additional group can be for example selected from the group consisting in fluorescent dyes such as free-base and metallo porphyrins, for example - tetraphenylporphyrin ($H_2$TPP), metallo-tetraphenylporphyrin, said metallo group can be any metal, for example Zinc (ZnTPP), 4,4-difluoro-4-bora-3a,4a-diaza-s-indacene (BODIPY) derivatives, amines derivatives fluorescent dyes such as benzyl amines fluorescent dyes and alcohol derivatives fluorescent dyes.

In a fourth aspect, the invention further relates to a chimeric hemicucurbit[n]uril-modified material, wherein chimeric hemicucurbit[n]uril according to the above-described second or third aspects of the invention are attached to a material. Preferably, said material is selected from a solid support or an insoluble compound, preferably a metal support, a

silica support, an alumina support and a polymer support, preferably a silica support, even more preferably a 3-aminopropyl silico gel.

Finally, in a fifth aspect, the invention also concerns the use of the chimeric hemicucurbit[n]uril(s) according to the above metnionned second and theird aspects of the invention or the chimeric hemicucurbit[n]uril-modified material according to the fourth aspect of the invention for binding anions, cations or organic molecules.

***Brief Description of the drawings***

**Figure 1:** General synthesis procedure for screening reaction with cyclohexanohemicucurbit[8]uril (cycHC[n]) as starting material.
**Figure 2:** Screening of reaction conditions using response surface methodology (RSM).
**Figure 3:** Removal percentage upon extraction of perchlorate and sulfate anions from methanol by mono-biotinylated cyclohexanohemicucurbit[8]uril-3-aminopropyl silica gel (mixHC[8]-APS) and non-modified APS. The error bars represent standard deviation between parallel experiments (n≥2).
**Figure 4:** General scheme of mixHC[8] synthesis, highlighting the best yields from routes a and b and screened templates.
**Figure 5:** 3D response surfaces displaying the effect of the yields of **A)** mixHC[8] and **B)** cycHC[8] on the ratio of monomers (factor i) and loadings of aq. $HClO_4$ (factor ii) and also correlation between other factors **(C** to **L).**
**Figure 6:** Effect of the acid templating anion (a), hexafluorophosphate salt additive (b), and the ratio of monomers (c) on the yields of cycHC[8] and mixHC[8].
**Figure 7:** The distribution of biotin and mutual interconversion of intermediates during the synthesis of mixHC[8] (General conditions: 1:7 mixture of monomers, $HPF_6$ / $KPF_6$; Table 7). CU = (*R*,*R*)-*N*,*N'*-cyclohexa-1,2- diylurea, mixOLIGO = linear oligomers composed of different units, homoOLIGO = homomeric linear oligomers.
**Figure 8:** Content of homodimers $(B)_2$ and $(CU)_2$ and products mixHC[8] and cycHC[8], during milling, prior to and after the aging stage in reaction with $HPF_6$ / $KPF_6$, and 1:7 ratio of monomers; (a, b) homodimers during milling and after aging; (c, d) macrocycles during milling and after aging; (e) content of macrocycles during aging; (f) a representative HPLC total ion chromatogram of homodimers. The content of the dimers is reported as the average normalized peak area for triplicate measurements (n = 3) ± standard deviation; the yields of macrocycles are presented as mean values obtained in a series of replicated experiments (2 ≤ n ≤ 4) ± standard deviation.
**Figure 9:** Density Functional Theory (DFT) structures of mixHC[8] pseudo-enantiomers with the S-atom pointing away from (left) and inward toward (right) the structures.
**Figure 10:** Upper (left) and side view (right) of single crystal X-ray diffraction (SC-XRD) of the $PF_6^-$@mixHC[8] inclusion complex (Note: the tetrabutylammonium (TBA) cation and $PF_6^-$@cycHC[8] are omitted for clarity).
**Figure 11:** Immobilization of mixHC[8] on APS (a). DIC = *N*,*N'*-diisopropylcarbodiimide, DCM (dichloromethane) and perchlorate removal from spiked Martian Global Simulant (MGS-1) using mixHC[8]-APS and non-modified APS, determined by ion chromatography based on the concentration change before and after solid-phase extraction (SPE) (b). The error bars represent the standard deviation between the parallel experiments (*n* ≥ 2).
**Figure 12:** Procedure for the synthesis of mixHC[8]-$H_2$TPP derivative.
**Figure 13:** HPLC chromatogram of mixHC[8]-$H_2$TPP derivative.
**Figure 14:** Procedure for the synthesis of mixHC[8]-ZnTPP derivative.
**Figure 15:** HPLC chromatogram of mixHC[8]-ZnTPP derivative.
**Figure 16:** Procedure for the synthesis of mixHC[8]-BODIPY derivative.
**Figure 17:** HPLC chromatogram of mixHC[8]-BODIPY derivative.
**Figure 18:** Procedure for the synthesis of mixHC[8]-Benzyl amine derivative.
**Figure 19:** Procedure for the synthesis of mixHC[8]-Amine derivative.
**Figure 20:** HPLC chromatogram of mixHC[8]-Amine derivative.

***Detailed Description of the Invention***

The inventors have developed a Method of preparation of enantiopure chimeric hemicucurbit[n]urils. It is a solvent-free/solid state method, mechanochemically aided and anion templated.

***Definitions***

It is to be understood that the terminology used herein is for the purpose of describing particular embodiments only and is not intended to be limiting.

Unless defined otherwise, all technical and scientific terms used herein have the same meaning as commonly understood to one of ordinary skill in the art to which the present application belongs. Although any methods and materials similar or equivalent to those described herein can be used in the practice or testing of the present application, representative methods and materials are herein described.

As used herein, the terms "a", "an", and "the" refer to "one or more" when used in this application, including the claims. Thus, for example, reference to "a compound that forms methylene bridges" includes mixtures of one or more such compounds, two or more such compounds, and the like.

Unless otherwise indicated, all numbers expressing quantities of ingredients, reaction conditions, and so forth used in the specification and claims are to be understood as being modified in all instances by the term "about". As used herein, the term "about" when referring to a measurable value such as an amount of weight, time, dose, etc. is meant to encompass in one example variations of $\pm$ 20%, preferably $\pm$ 10%, more preferably $\pm$ 5%, alternatively $\pm$ 2%, still alternatively $\pm$ 1% and yet alternatively $\pm$ 0,1% from the specified amount. For example, "about 20" include all the values ranging from $\pm$ 5% of 20, i.e. a range of values from 19 to 21.

As used herein, the terms "initial" or initially" refer to conditions at the beginning of the first step of the method of the invention, the contacting step.

As used herein, the terms "mechanochemistry" and "mechanochemically aided" refer to chemical transformations initiated or sustained by mechanical force.

As used herein the terms "milling" and "grinding" are equivalent and refer to a process of mixing of materials, applying a mechanical force to said materials, and mechanically reducing said materials to a powder of fine or very fine size, preferably < about 10 $\mu$m.

As used herein, the term "$\eta$" refers to the amount of liquid (in $\mu$L) per amount of solid (mg) in the reaction media and describes liquid assistance.

As used herein, the terms "solid state", "solvent free", "solventless" and "essentially solvent free" are equivalent and refer to reactions in which $\eta$ is comprised between 0 and 1. In this range, liquid assisted grinding takes place. As used herein, the term "Liquid Assisted Grinding" refers to the use of a small amount of a liquid to accelerate a reaction, as well as to enable and direct transformations that do not take place by neat grinding ($\eta$ = 0). Such a liquid can, for example, facilitate proton transfer, delivery of template, and promote conformational flexibility.

As used herein, the term "Process Mass Intensity" refers to the total mass of materials used to produce a specified mass of product. Materials include reactants, reagents, solvents used for reaction and purification, as well as catalysts.

As used herein, the term "self-assembly" refers to a process in which molecules spontaneously form ordered aggregates.

As used herein, the term "Dynamic Covalent Chemistry" (DCC) refers to a synthetic strategy to make complex supramolecular assemblies from discrete molecular building blocks. In DCC, a template is used to select a specific molecule among others with which it is in equilibrium through covalent bounds, for example after self-assembly.

As used herein, the term "template" refers to molecules capable to establish reversible noncovalent bonding interactions with molecular building blocks in order to organize them into a certain relative geometry.

As used herein, the term "single pot reaction", "single step reaction", "single step synthesis" or "single pot synthesis" are equivalent and refer to synthesis in which the reactants are subjected to successive chemical reactions in just one reactor. This kind of synthesis avoid lengthy separation process and purification of the intermediate chemical compounds.

As used herein, the terms "yield" and "reaction yield" are equivalent and refer to a measure of the quantity of moles, expressed as a percentage, of a product formed in relation to the reactant consumed, obtained in a chemical reaction.

As used herein, the term "monomer" refers to a molecule that has the ability to chemically bond with other monomers in a chain. This chain is an oligomer or a polymer.

As used herein, the term "macrocycle" refers to a molecule that contains a cyclic framework of at least twelve atoms. A macrocycle can be made of several monomers. In that case, the macrocycle is oligomeric (number of monomers < 10) or polymeric (number of monomers > 10).

As used herein, the term "chimeric", refers to a polymeric macrocycle in which the monomers are not identical. A monochimeric macrocycle has 2 types of monomers, a dichimeric macrocycle has 3 types of monomers, a trichimeric macrocycle has 4 types of monomers, a tetrachimeric macrocycle has 5 types of monomers, etc.

As used herein, the term "chiral" refers to a molecule that is distinguishable from its mirror image, that is, it cannot be superimposed onto it.

As used herein, the term "stereoisomer" refers to molecules that have the same molecular formula and sequence of bonded atoms, but differ in the three-dimensional orientations of their atoms in space.

As used herein, the term "enantiomer" refers to stereoisomers that are mirror images of each other and that are non-superimposable.

As used herein, the term diastereoisomer" refers to stereoisomers that are not mirror images of each other's.

As used herein, the term "pseudoenantiomer" refers to diastereomers that, in the case of chiral induction, behave as if they were enantiomers.

As used herein, the term "chiral induction" refers to the process of generation of non-racemic chiral product from achiral or racemic starting material(s).

As used herein, the terms "stereocenter" or "stereogenic center" are equivalent and refer to an atom that is bound to at least three different groups in such a way that interchanging any two different groups creates a new stereoisomer.

As used herein, the term "enantiopure" refers to a molecule that is available in one specific enantiomeric form.

As used herein, the terms "equivalent" and "eq" are equivalent and refer to the amount of a substance in moles in relation to 1 mole of monomeric urea derivative.

***Product***

In a first aspect, the invention relates to a chimeric hemicucurbit[n]uril.

As used herein the term "hemicucurbit[n]uril" refers to a macrocycle composed of monomers of cyclic urea derivatives hinged together by methylene bridges via the nitrogen atoms of their cyclic urea derivatives.

As used herein, the term "urea derivative" refers to a class of organic compounds derived from urea ($(H_2N)_2CO$).

As used herein, the term "cyclic urea derivative", "*N,N'*-(1,2-ethanediyl)-urea monomer" or "N,N'-(1,2-ethanediyl)-urea derivative" are equivalent and refer to molecules according to the following formula (I):

(I) 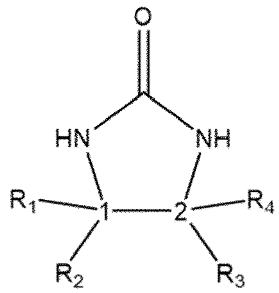

wherein:

1 and 2 are carbon atoms and at least one of them is a stereogenic center, preferably both of them are stereogenic centers;

at least one of the substituents R1 to R4 bears a stereogenic center;

$R_1$, $R_2$, $R_3$, and $R_4$ can each independently be selected from the group consisting in a hydrogen atom, -OH, -COOH, $-NH_2$, $-NO_2$, $-NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl;

As used herein, the term "alkyl" refers to linear or branched alkyl chain having 1 to 30 carbon atoms, and in which any of the $-CH_2-$ groups can be replaced by -O-, -S- or - NH- group, while the alkyl can be unsubstituted or substituted by 1 to 5 groups selected from the group consisting of -OH, -SH, =O, halogen, aryl, -NH2 and -COORc, where Rc is hydrogen atom or alkyl group having 1 to 6 carbon atoms;

As used herein, the term "alkenyl" refers to a linear or branched chain containing 2 to 30 carbon atoms, and containing at least one double bond, and in which any of the $-CH_2-$groups can be replaced by -O-, -S- or -NH- group, and any =CH-group can be replaced by =N-, while the alkenyl can be unsubstituted or substituted by 1 to 5 groups selected from the group consisting of -OH, -SH, =O, halogen, aryl, $-NH_2$, and -COORc, wherein Rc is hydrogen atom or alkyl group having 1 to 6 carbon atoms;

As used herein, the term "alkynyl" refers to a linear or branched chain having 2 to 30 carbon atoms, and containing at least one triple bond, and which may contain also a double bond, in this alkynyl chain any of the $-CH_2-$ groups can be replaced by -O-, -S- or - NH- group, and any =CH- group can be replaced by =N-, while the alkynyl can be unsubstituted or substituted by 1 to 7 groups selected from the group consisting of -OH, - SH, =O, halogen, aryl, $-NH_2$, and -COORc, wherein Rc is hydrogen atom or alkyl group having 1 to 6 carbon atoms;

As used herein, the term "cycloalkyl" refers to a linear or branched group having 4 to 10 carbon atoms, and containing at least one cycle, in which any of the $-CH_2-$ groups can be replaced by -O-, -S- or -NH- group, while the cycloalkyl can be unsubstituted or substituted by 1 to 5 groups consisting of -OH, -SH,=O, halogen, aryl, $-NH_2$, and -COORc, wherein Rc is hydrogen atom or alkyl group having 1 to 6 carbon atoms;

As used herein, the term "cycloalkenyl" refers to a linear or branched group having 4 to 10 carbon atoms, and containing at least one double bond and at least one cycle, in which any of the $-CH_2-$ groups can be replaced by -O-, -S- or -NH- group, and any =CH-group can be replaced by =N-, while the cycloalkenyl can be unsubstituted or substituted

by 1 to 5 groups consisting of -OH, -SH, =O, halogen, aryl, $-NH_2$, and -COORc, where Rc is hydrogen atom or alkyl group having 1 to 6 carbon atoms;
As used herein, the term "alkoxy" refers to a group -ORa, where Ra is selected from the group consisting of alkyl, alkenyl, alkynyl, cycloalkyl, and cycloalkenyl;
As used herein, the term "hydrocarbylthio" refers to a group -SRa, where Ra is selected from the group consisting of alkyl, alkenyl, alkynyl, cycloalkyl, and cycloalkenyl;
As used herein, the term "hydrocarbylamino" refers to a group -NHRa, where Ra is selected from the group consisting of alkyl, alkenyl, alkynyl, cycloalkyl, and cycloalkenyl;
As used herein, the term "dihydrocarbylamino" refers to a group -NRaRb, where Ra and Rb are each independently selected from the group consisting of alkyl, alkenyl, alkynyl, cycloalkyl, and cycloalkenyl;
As used herein, the term "carboxyl" refers to a group -COORa, where Ra is selected from the group consisting of alkyl, alkenyl, alkynyl, cycloalkyl, and cycloalkenyl;
As used herein, the term "aryl" refers to a hydrocarbon group having 6 to 30 carbon atoms, and containing at least one aromatic ring, and the aryl can be unsubstituted or substituted by 1 to 5 groups selected independently from the group consisting of -OH, -SH, =O, halogen, $-NH_2$, and -COORc, where Rc is hydrogen atom or alkyl group having 1 to 6 carbon atoms;
As used herein, the term "heteroaryl" refers to hydrocarbon group having 4 to 30 carbon atoms, and containing at least one aromatic ring, containing at least one heteroatom selected from the group comprising O, S, N, and the heteroaryl can be unsubstituted or substituted by 1 to 5 groups selected independently from -OH, -SH, =O, halogen, $-NH_2$, and -COORc, where Rc is hydrogen atom or alkyl group having 1 to 6 carbon atoms;
As used herein, the term "halogen" refers to the group consisting in fluorine (F), chlorine (Cl), bromine (Br), iodine (I), astatine (At), and tennessine (Ts), preferably the halogen of the invention is selected from the group consisting in fluorine, chlorine, bromine and iodine.

In a preferred embodiment, the monomers of cyclic urea derivative of the chimeric hemicucurbit[n]uril of the invention are selected from the group consisting in 4,5-Dimethyl-2-imidazolidinone, 4-alkyl-2-imidazolidinone, 2-imidazolidinone, rel-(3aR,6aS)-Hexahydropyrrolo[3,4-d]imidazol-2(1H)-one, 2-Oxo-4-imidazolidinecarboxylic acid, 2-Oxo-4-imidazolidinecarbonitrile, 4-(Aminomethyl)-2-imidazolidinone, 4-(Hydroxymethyl)-2-imidazolidinone, hexahydro-2(1H)-Cyclopentimidazolone, 4-(2-Hydroxyethyl)-2-imidazolidinone, rel-(3aR,6aS)-Tetrahydro-1H-furo[3,4-d]imidazol-2(3H)-one, Octahydroimidazo[4,5-d]azepin-2(1H)-one, 4-(Ethoxymethyl)-2-imidazolidinone, 4,5-Dipropyl-2-imidazolidinone, (4R or 4S)-4-Phenyl-2-imidazolidinone, (4S or 4R)-2-Oxo-4-imidazolidineacetic acid, 8-Methyl-1,3,7-triazaspiro[4.4]nonan-2-one, (4S or 4R)-4-(4-Pyridinyl)-2-imidazolidinone, 4,5-Dialkyl-2-imidazolidinone, 2-Oxo-4-imidazolidinepropanoic acid, 4-(4-Hydroxyphenyl)-2-imidazolidinone, 1H-Thieno[3,4-d]imidazol-2(3H)-one, tetrahydro-, 5,5-dioxide, (3aR,6aR)-Spiro[1-azabicyclo[2.2.2]octane-3,4'-imidazolidin]-2'-one, 2-[(4R)-2-Oxo-4-imidazolidinyl]benzonitrile, ethylene urea, *N*,*N*'-cyclohexa-1,2-diylurea, D-biotin, their stereoisomers thereof, and their derivatives thereof, preferably ethylene urea, *N*,*N*'-cyclohexa-1,2-diylurea, D-biotin, their stereoisomers thereof, and their derivatives thereof.

In a most preferred embodiment, the cyclic urea derivatives of the invention are in an enantiopure form. The man skilled in the art know how to select enantiopure forms of the cyclic urea derivatives. For example, biotin can be D-biotin or L-biotin.

In the chimeric hemicucurbit[n]uril of the invention, the monomers of cyclic urea derivatives are hinged together by methylene bridges.

As used herein, the terms "methylene bridge", "methylene spacer", or "methanediyl group" are equivalent and refer to any part of a molecule with formula "$-CH_2-$", namely a carbon atom bound to two hydrogen atoms and connected by single bonds to two other distinct atoms in the rest of the molecule.

The hemicucurbit[*n*]uril of the invention is a macrocycle comprising n monomers of cyclic urea derivatives, *n* being an even positive integer. Preferably *n* is at least superior or egal to 4. Preferably n is inferior or egal to 20. Thus, in a preferred embodiment, *n* is comprised between 4 and 20. Preferably, n is comprised between 4 and 14, more preferably between 4 and 12, even more preferably between 4 and 10. In a most preferred embodiment *n* is 6 or 8. For example, the hemicucurbit[*n*]uril of the invention can be a hemicucurbit[4]uril, a hemicucurbit[6]uril, a hemicucurbit[8]uril, a hemicucurbit [10]uril, a hemicucurbit[12]uril or a hemicucurbit[14]uril. Preferably, the hemicucurbit[*n*]uril of the invention is a hemicucurbit[6]uril or a hemicucurbit[8]uril. Even more preferably, the hemicucurbit[*n*]uril of the invention is a hemicucurbit[8] uril.

The hemicucurbit[*n*]uril of the invention is a chimeric hemicucurbit[*n*]uril. The chimeric hemicucurbit[*n*]uril according to the invention comprises at least 2 different monomers of cyclic urea derivatives. The hemicucurbit[*n*]uril of the invention may have between 2 and 5 different monomers of cyclic urea derivatives, preferably between 2 and 4 different monomers of cyclic urea derivatives, more preferably 2 or 3 different monomers of cyclic urea derivatives. In a most preferred embodiment, the chimeric hemicucurbit[n]uril of the invention has 2 different monomers of cyclic urea derivatives. For example, the chimeric hemicucurbit[*n*]uril according to the invention can be monochimeric (2 different

monomers), dichimeric (3 different monomers), trichimeric (4 different monomers), or tetrachimeric (5 different monomers). Preferably, the chimeric hemicucurbit[n]uril according to the invention is monochimeric or dichimeric, even more preferably monochimeric. A monochimeric hemicucurbit[n]uril according to the invention is of the following formula (II):

(II) -[-$CH_2$-(Cyclic urea derivative $1)_a$-$CH_2$-(Cyclic urea derivative $2)_b$-$CH_2$-]-

wherein a + b = n

In a preferred embodiment, the chimeric hemicucurbit[n]uril according to the invention is a monochimeric hemicucurbit[6]uril of the following formula (III):

(III) -[-$CH_2$-( Cyclic urea derivative $1)_a$-$CH_2$-( Cyclic urea derivative $2)_b$-$CH_2$-]-

wherein a + b = 6

In an even preferred embodiment, the chimeric hemicucurbit[n]uril according to the invention is a monochimeric hemicucurbit[8]uril of the following formula (IV):

(IV) -[-$CH_2$-( Cyclic urea derivative $1)_a$-$CH_2$-( Cyclic urea derivative $2)_b$-$CH_2$-]-

wherein a + b = 8

In a preferred embodiment, the first cyclic urea derivative is the (*S,S*)-*N,N'*-cyclohexa-1,2-diylurea (V) or the (*R*, *R*)-*N*,*N*'-cyclohexa-1,2-dylurea (VI) of the following formula:

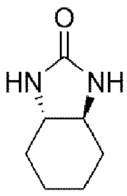
(V)

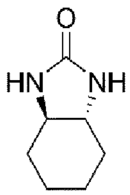
(VI)

In another preferred embodiment, the second cyclic urea derivative is the D-biotin of the following formula (VII):

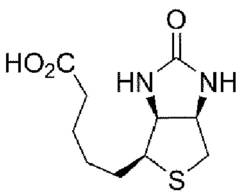
(VII).

In an even preferred embodiment, the chimeric hemicucurbit[*n*]uril of the invention is a monochimeric hemicucurbit[n]uril with (*S*,*S*)-*N*,*N*'-cyclohexa-1,2-diylurea or the (*R,R*)-*N*,*N*'-cyclohexa-1,2-dylurea as a first cyclic urea derivative and D-biotin as a second cyclic urea derivative.

In preferred embodiment, the chimeric hemicucurbit[n]uril of the invention is a monochimeric hemicucurbit[6]uril with (*S,S*)-*N,N'*-cyclohexa-1,2-diylurea or the (*R,R*)-*N,N'*-cyclohexa-1,2-dylurea as a first cyclic urea derivative and D-biotin as a second cyclic urea derivative. Such monochimeric hemicucurbit[6]urils are biotinylated cyclohexanohemicucurbit[6]urils of the following formula (VIII and IX):

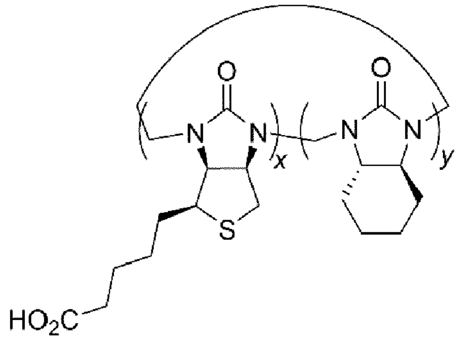

(VIII);

or

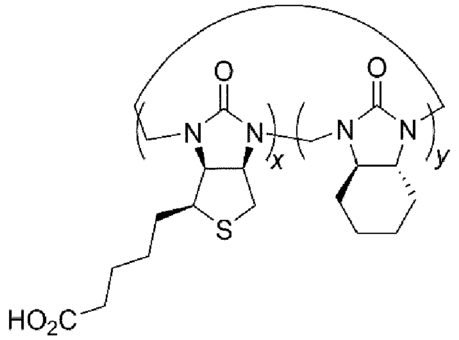

(IX);

wherein x + y = 6

In a most preferred embodiment, the chimeric hemicucurbit[n]uril of the invention is a monochimeric hemicucurbit[8]uril with (*S*,*S*)-*N*,*N*'-cyclohexa-1,2-diylurea or the (*R,R*)-*N,N'*-cyclohexa-1,2-dylurea as a first cyclic urea derivative and D-biotin as second cyclic urea derivative. Such monochimeric hemicucurbit[8]urils are biotinylated cyclohexanohemicucurbit[8]urils of the following formula (X and XI):

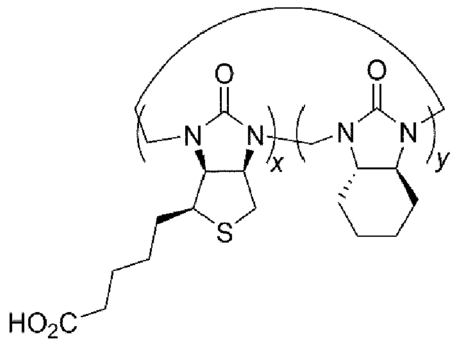

(X);

or

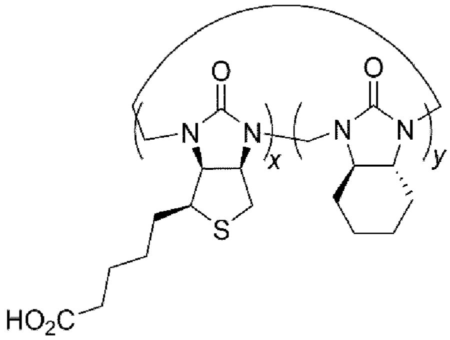

(XI);

wherein x + y = 8

In the chimeric hemicucurbit[n]uril of the invention, one of the cyclic urea derivatives is the main cyclic urea derivative and the initial ratio of the other cyclic urea derivatives on the main cyclic urea derivative is comprised between about 1/20 and about 3/4, preferably between about 1/14 and about 1/2, more preferably between 1/10 and 1/4. For example, the hemicucurbit[n]uril of the invention may have 1, 2, 3, 4, 5 or 6 monomers of said other cyclic urea derivatives in its composition, preferably 1, 2, 3 or 4, more preferably, 1, 2 or 3, still preferably 1 or 2. Even more preferably, the hemicucurbit[n]uril of the invention has 1 monomer of said other cyclic urea derivative in its composition.

As used herein, the term "main cyclic urea derivatives" refers to the monomer of cyclic urea derivative present in the higher proportion in the hemicucurbit[*n*]uril.

In a preferred embodiment, the chimeric hemicucurbit[n]uril of the invention is a monochimeric hemicucurbit[n]uril having a ratio of monomers of the second/other cyclic urea derivative on the first/main cyclic urea derivative comprised between about 1/20 and about 3/4, preferably between about 1/14 and about 1/2, more preferably between 1/10 and 1/4. For example, the monochimeric hemicucurbit[n]uril of the invention may have 1, 2, 3, 4, 5 or 6 monomers of said second/other cyclic urea derivative in its composition, preferably 1, 2, 3 or 4, more preferably, 1, 2 or 3, still preferably 1 or 2. Even more preferably, the monochimeric hemicucurbit[n]uril of the invention has 1 monomer of said second/other cyclic urea derivative in its composition.

In a more preferred embodiment, the chimeric hemicucurbit[n]uril of the invention is a monochimeric hemicucurbit[6]uril or monochimeric hemicucurbit[8]uril, preferably a monochimeric hemicucurbit[8]uril, having a ratio of monomers of the second/other cyclic urea derivative comprised between about 1/8 and about 2/3, preferably between about 1/8 and about 1/2, more preferably between 1/8 and 1/3. For example, the monochimeric hemicucurbit[6]uril or monochimeric hemicucurbit[8]uril, preferably monochimeric hemicucurbit[8]uril, of the invention may have 1, 2, 3, 4, 5 or 6 monomers of said second/other cyclic urea derivative in its composition, preferably 1, 2, 3 or 4, more preferably, 1, 2 or 3, still preferably 1 or 2. Even more preferably, the monochimeric hemicucurbit[6]uril or monochimeric hemicucurbit[8]uril, preferably monochimeric hemicucurbit[8]uril, of the invention has 1 monomer of said second/other cyclic urea derivative in its composition.

In an even preferred embodiment, the chimeric hemicucurbit[n]uril of the invention is a monochimeric hemicucurbit[6]uril with (*S,S*)-*N,N'*-cyclohexa-1,2-diylurea or the (*R,R*)-*N,N'*-cyclohexa-1,2-dylurea as a first/main cyclic urea derivative and D-biotin as a second/other cyclic urea derivative or a monochimeric hemicucurbit[8]uril with (*S,S*)-*N,N'*-cyclohexa-1,2-diylurea or the (*R,R*)-*N,N'*-cyclohexa-1,2-dylurea as a first/main cyclic urea derivative and D-biotin as a second/other cyclic urea derivative, preferably a monochimeric hemicucurbit[8]uril with (*S,S*)-*N,N'*-cyclohexa-1,2-diylurea or the (*R,R*)-*N,N'*-cyclohexa-1,2-dylurea as a first/main cyclic urea derivative and D-biotin as a second/other cyclic urea derivative, having a ratio of monomers of D-biotin on (*S,S*)-*N,N'*-cyclohexa-1,2-diylurea or (*R,R*)-*N,N'*-cyclohexa-1,2-dylurea comprised between about 1/8 and about 2/3, preferably between about 1/8 and about 1/2, more preferably between 1/8 and 1/3. For example, 1, 2, 3, 4, 5 or 6 monomers of D-biotin may be present in its composition, preferably 1, 2, 3 or 4, more preferably, 1, 2 or 3, still preferably 1 or 2.

In a most preferred embodiment, the chimeric hemicucurbit[n]uril of the invention is a monochimeric hemicucurbit[6]uril with five monomers of (*S,S*)-*N,N'*-cyclohexa-1,2-diylurea or (*R,R*)-*N,N'*-cyclohexa-1,2-dylurea and one monomer of D-biotin. Such monochimeric hemicucurbit[6]urils are (+)-((*S,S,R*)(*S,S*)$_5$)-mono-biotinylated cyclohexanohemicucurbit[6]uril and (-)-((*S,S,R*)(*R,R*)$_5$)-mono-biotinylated cyclohexanohemicucurbit[6]uril of the respective formulas (XII, XIII):

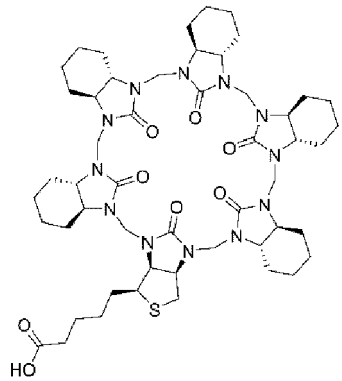

(XII);

and

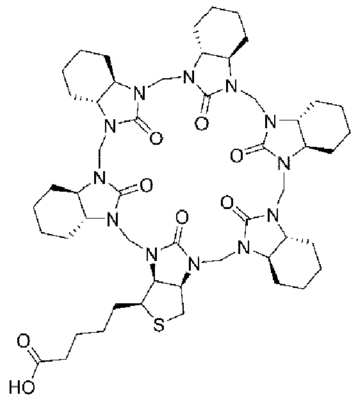

(XIII).

In an alternative most preferred embodiment, the chimeric hemicucurbit[*n*]uril of the invention is a monochimeric hemicucurbit[8]uril with seven monomer of (*S,S*)-*N,N'*-cyclohexa-1,2-diylurea or (*R*,*R*)-*N*,*N*'-cyclohexa-1,2-dylurea and one monomer of D-biotin. Such monochimeric hemicucurbit[8]urils are and (+)-((*S*,*S*,*R*)(*S*,*S*)$_7$)-mono-biotinylated cyclohexanohemicucurbit[8]uril and (-)-((*S*,*S*,*R*)(*R*,*R*)$_7$)-mono-biotinylated cyclohexanohemicucurbit[8]uril of the respective formulas (XIV, XV):

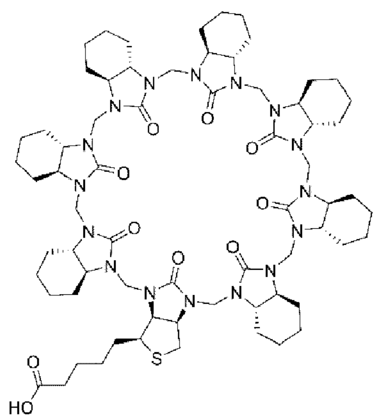

(XIV);

and

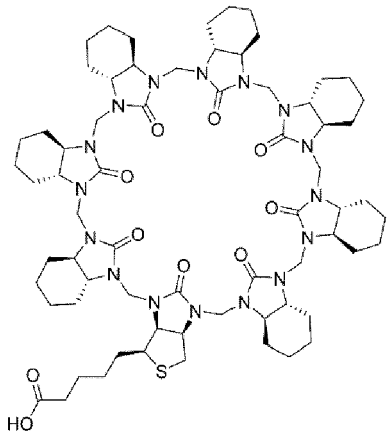

(XV).

In another preferred embodiment, the chimeric hemicucurbit[n]uril of the invention is a chimeric hemicucurbit[4] uril, preferably a monochimeric hemicucurbit[4]uril with D-biotin as a first/main cyclic urea derivative and (*S*,*S*)-*N*,*N*'-cyclohexa-1,2-diylurea or the (*R*,*R*)-*N*,*N*'-cyclohexa-1,2-dylurea as a second/other cyclic urea derivative, more preferably the chimeric hemicucurbit[*n*]uril of the invention is a monochimeric hemicucurbit[4]uril with 3 monomers of D-biotin and 1 monomers of (*S*,*S*)-*N*,*N*'-cyclohexa-1,2-diylurea or (*R,R*)-*N,N'*-cyclohexa-1,2-dylurea and. Such monochimeric hemicucurbit[4]urils are (+)-(($S,S,R)_3(S,S)$)-tri-biotinylated cyclohexanohemicucurbit[4]uril and (-)-(($S,S,R)_3(R,R)$)-tri-biotinylated cyclohexanohemicucurbit[4]uril of the respective formulas (XVI, XVII):

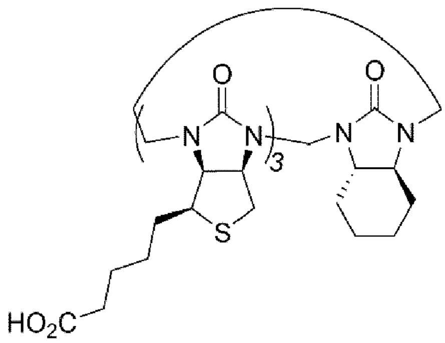

monomers can adapt any order (XVI);

and

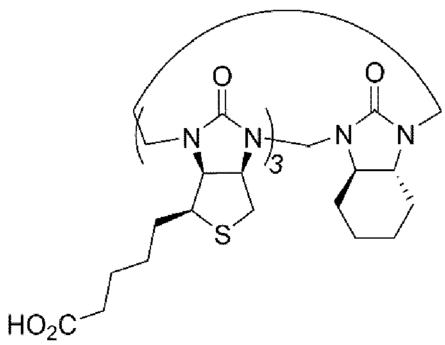

monomers can adapt any order (XVII).

In another preferred embodiment, the invention also concern alternative chimeric hemicucurbit[6]urils such as:

- chimeric hemicucurbit[6]urils with four monomers of (*S,S*)-*N,N'*-cyclohexa-1,2-diylurea or (*R*,*R*)-*N*,*N*'-cyclohexa-1,2-dylurea and two monomers of D-biotin. Such dichimeric hemicucurbit[6]urils are (+)-(($S,S,R)_2(S,S)_4$)-di-biotinylated cyclohexanohemicucurbit[6]uril and (-)-(($S,S,R)_2(R,R)_4$)-di-biotinylated cyclohexanohemicucurbit[6]uril of the respective formulas (XVIII, XIX):

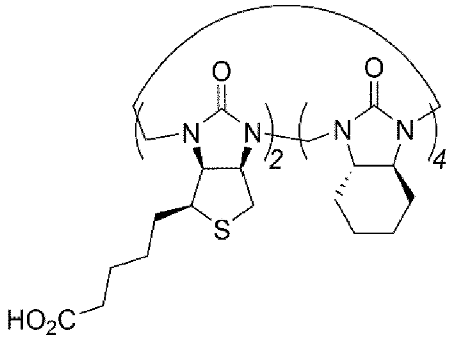

monomers can adapt any order (XVIII);

and

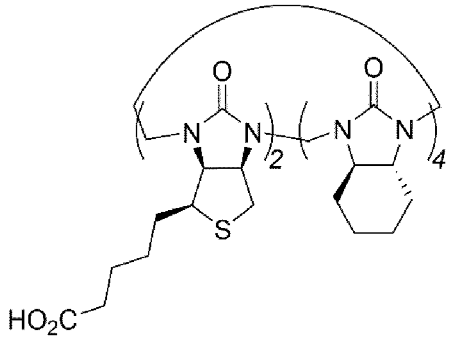

monomers can adapt any order (XIX).

- chimeric hemicucurbit[6]urils with 3 monomers of (*S,S*)-*N,N'*-cyclohexa-1,2-diylurea or (*R,R*)-*N,N'*-cyclohexa-1,2-dylurea and 3 monomers of D-biotin. Such trichimeric hemicucurbit[6]urils are (+)-((*S,S,R*)$_3$(*S,S*)$_3$)-tri-biotinylated cyclohexanohemicucurbit[6]uril and (-)-((*S,S,R*)$_3$(*R,R*)$_3$)-tri-biotinylated cyclohexanohemicucurbit[6]uril of the respective formulas (XX, XXI):

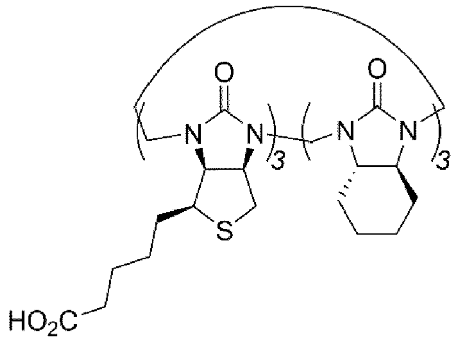

monomers can adapt any order (XX);

and

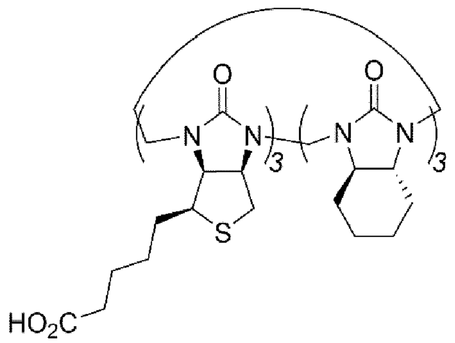

monomers can adapt any order (XXI).

- chimeric hemicucurbit[6]urils with 2 monomers of (*S,S*)-*N,N'*-cyclohexa-1,2-diylurea or (*R*,*R*)-*N*,*N'*-cyclohexa-1,2-dylurea and 4 monomers of D-biotin. Such dichimeric hemicucurbit[6]urils are (+)-((*S*,*S*,*R*)$_4$(*S*,*S*)$_2$-tetra-biotinylated cyclohexanohemicucurbit[6]uril and (-)-((*S*,*S*,*R*)$_4$(*R*,*R*)$_2$-tetra-biotinylated cyclohexanohemicucurbit[6]uril of the respective formulas (XXII, XXIII):

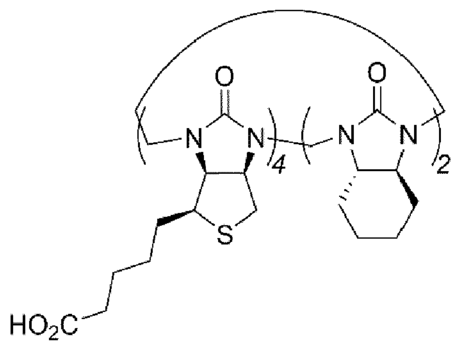

monomers can adapt any order (XXII);

and

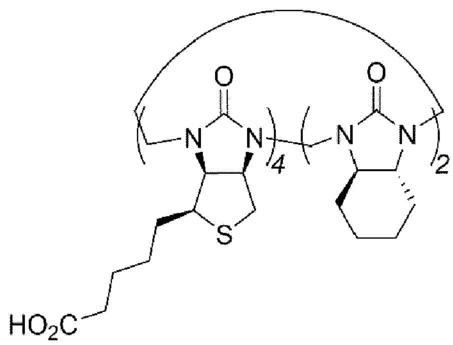

monomers can adapt any order (XXIII).

- chimeric hemicucurbit[6]urils with 1 monomer of (*S*,*S*)-*N*,*N'*-cyclohexa-1,2-diylurea or (*R*,*R*)-*N*,*N'*-cyclohexa-1,2-dylurea and 5 monomers of D-biotin. Such monochimeric hemicucurbit[6]urils are (+)-((*S*,*S*,*R*)$_5$(*S*,*S*))-penta-biotinylated cyclohexanohemicucurbit[6]uril and (-)-((*S*,*S*,*R*)$_5$(*R*,*R*))-penta-biotinylated cyclohexanohemicucurbit[6]uril of the respective formulas (XXIV, XXV):

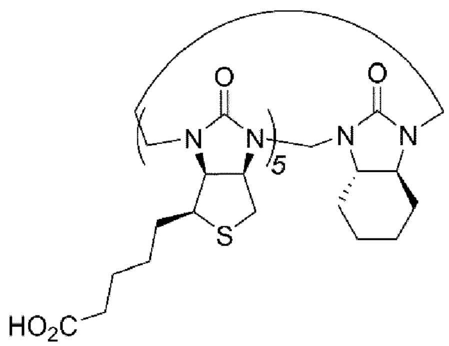

(XXIV);

and

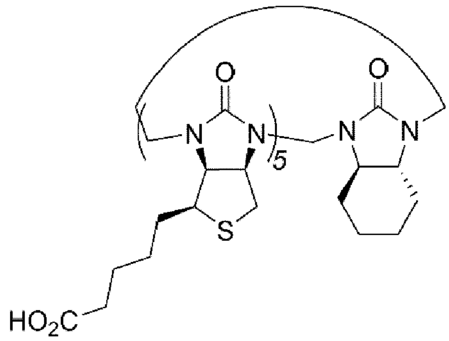

(XXV).

In another preferred embodiment, the invention also concern alternative chimeric hemicucurbit[8]urils such as:

- chimeric hemicucurbit[8]urils with six monomers of (*S,S*)-*N,N'*-cyclohexa-1,2-diylurea or (*R,R*)-*N,N'*-cyclohexa-1,2-dylurea and two monomers of D-biotin. Such dichimeric hemicucurbit[8]urils are (+)-((*S,S,R*)$_2$(*S,S*)$_6$)-di-biotinylated cyclohexanohemicucurbit[8]uril and (-)-((*S,S,R*)$_2$(*R,R*)$_6$)-di-biotinylated cyclohexanohemicucurbit[8]uril of the respective formulas (XXVI, XXVII):

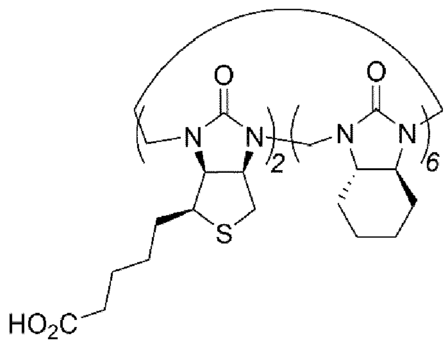

monomers can adapt any order (XXVI);

and

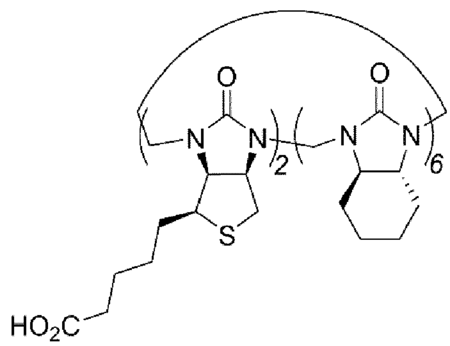

monomers can adapt any order (XXVII).

- chimeric hemicucurbit[8]urils with 5 monomers of (*S,S*)-*N,N'*-cyclohexa-1,2-diylurea or (*R,R*)-*N,N'*-cyclohexa-1,2-dylurea and 3 monomers of D-biotin. Such trichimeric hemicucurbit[8]urils are (+)-((*S,S,R*)$_3$(*S,S*)$_5$)-tri-biotinylated cyclohexanohemicucurbit[8]uril and (-)-((*S,S,R*)$_3$(*R,R*)$_5$)-tri-biotinylated cyclohexanohemicucurbit[8]uril of the respective formulas (XXVIII, XXIX):

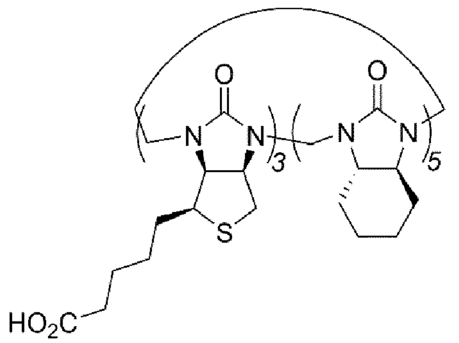

monomers can adapt any order (XXVIII);

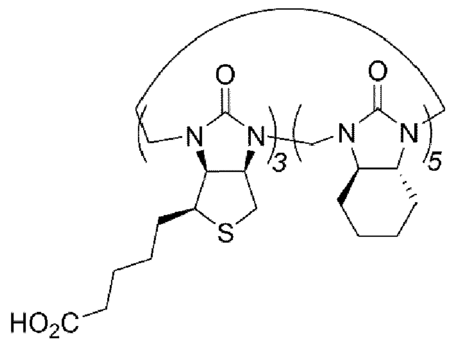

monomers can adapt any order (XXIX).

- chimeric hemicucurbit[8]urils with 2 monomers of (*S,S*)-*N,N'*-cyclohexa-1,2-diylurea or (*R*,*R*)-*N*,*N*'-cyclohexa-1,2-dylurea and 6 monomers of D-biotin. Such dichimeric hemicucurbit[8]urils are (+)-((*S*,*S*,*R*)$_6$(*S*,*S*)$_2$-hexa-biotinylated cyclohexanohemicucurbit[8]uril and (-)-((*S*,*S*,*R*)$_6$(*R*,*R*)$_2$)-hexa-biotinylated cyclohexanohemicucurbit[8]uril of the respective formulas (XXX, XXXI):

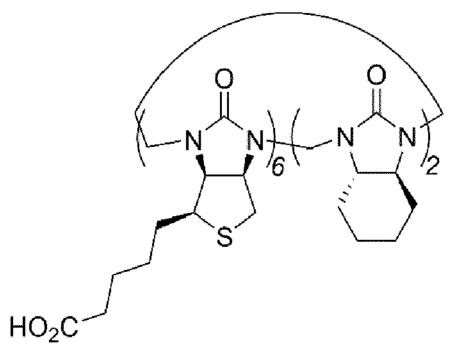

monomers can adapt any order (XXX);

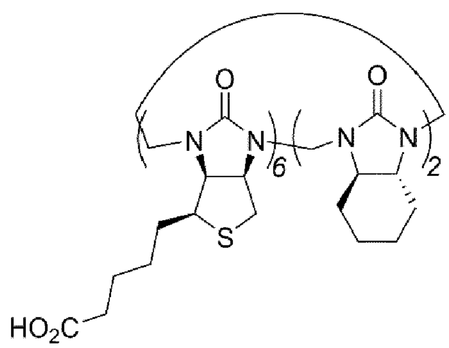

monomers can adapt any order (XXXI).

- chimeric hemicucurbit[8]urils with 4 monomers of (*S,S*)-*N,N'*-cyclohexa-1,2-diylurea or (*R*,*R*)-*N*,*N*'-cyclohexa-1,2-dylurea and 4 monomers of D-biotin. Such tetrachimeric hemicucurbit[8]urils are (+)-((*S,S,R*)$_4$(*S,S*)$_4$)-tetra-biotinylated cyclohexanohemicucurbit[8]uril and (-)-((*S*,*S*,*R*)$_4$(*R*,*R*)$_4$)-tetra-biotinylated cyclohexanohemicucurbit[8]uril of

the respective formulas (XXXII, XXXIII):

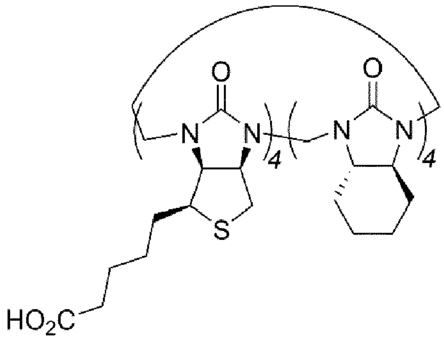

monomers can adapt any order (XXXII);

and

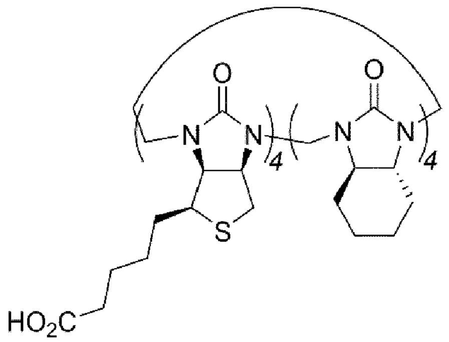

monomers can adapt any order (XXXIII).

- chimeric hemicucurbit[8]urils with 3 monomers of (*S,S*)-*N,N'*-cyclohexa-1,2-diylurea or (*R,R*)-*N,N'*-cyclohexa-1,2-dylurea and 5 monomers of D-biotin. Such trichimeric hemicucurbit[8]urils are (+)-((*S,S,R*)$_3$(*S,S*)$_5$)-penta-biotinylated cyclohexanohemicucurbit[8]uril and (-)-((*S,S,R*)$_3$(*R,R*)$_5$)-penta-biotinylated cyclohexanohemicucurbit[8]uril of the respective formulas (XXXIV, XXXV):

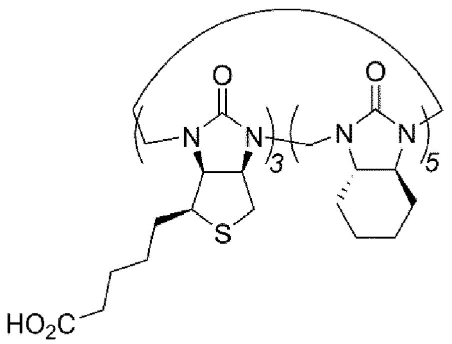

monomers can adapt any order (XXXIV);

and

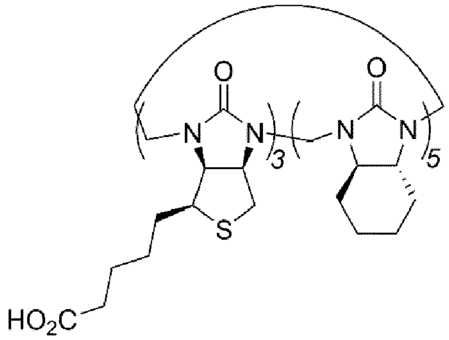

monomers can adapt any order (XXXV).

- chimeric hemicucurbit[8]urils with 1 monomers of (*S,S*)-*N,N'*-cyclohexa-1,2-diylurea or (*R,R*)-*N,N'*-cyclohexa-1,2-dylurea and 7 monomers of D-biotin. Such monochimeric hemicucurbit[8]urils are (+)-((*S,S,R*)$_7$(*S,S*)$_1$)-hepta-biotinylated cyclohexanohemicucurbit[8]uril and (-)-((*S,S,R*)$_7$(*R,R*)$_1$-hepta-biotinylated cyclohexanohemicucurbit [8]uril of the respective formulas (XXXVI, XXXVII):

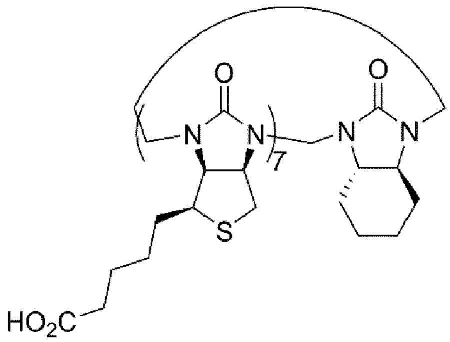

(XXXVI);

and

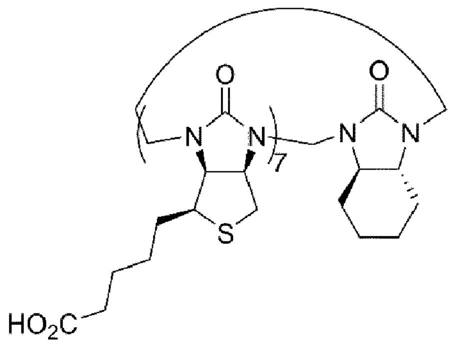

(XXXVII).

In a preferred embodiment, the chimeric hemicucurbit[n]uril according to the invention further comprises at least one additional group attached to at least one of its monomers, preferably D-biotin monomers, more preferably -COOH tails of D-biotin monomers. For example, the chimeric hemicucurbit[n]uril according to the invention may comprise between 1 and 7 additional groups, preferably between 1 and 4 additional group, more preferably 1 or 2 additional groups, even more preferably the chimeric hemicucurbit[n]uril according to the invention comprises 1 additional group.

The additional group according to the invention is preferably selected from the group consisting of fluorescent dyes such as free-base and metallo porphyrins, for example - tetraphenylporphyrin ($H_2$TPP), metallo-tetraphenylporphyrin, said metallo group can be any metal, for example Zinc (ZnTPP), 4,4-difluoro-4-bora-3a,4a-diaza-s-indacene (BODIPY) derivatives, amines derivatives fluorescent dyes such as benzyl amines fluorescent dyes and alcohol derivatives fluorescent dyes.

In a most preferred embodiment, the chimeric hemicucurbit[*n*]uril according to invention is a mono-biotinylated cyclohexanohemicucurbit[8]uril comprising an additional group attached to its D-biotin monomer, preferably the -COOH tail of its D-biotin monomer.

***Method***

In a second aspect, the invention also concerns a method of preparation of at least one chimeric hemicucurbit[n] uril comprising the following steps:

- Contacting, under mechanical agitation, at least two different cyclic urea derivatives and a compound that can form methylene bridges in the presence of at least one acid, and at least one solvent;
- Aging the products obtained at the previous step for at least about 30 minutes;

wherein n is an even positive integer comprised between 4 and 14
wherein the amount of liquid per amount of solid in the reaction media is initially < 1 μL/mg.

In the first step of the method of the invention, at least two different cyclic urea derivatives and a compound that can form methylene bridges are contacted.

The number of different cyclic urea derivatives to be contacted in the first step of the method of the invention is comprised between 2 and 5, preferably between 2 and 4, more preferably 2 or 3 different cyclic urea derivatives are contacted. In a most preferred embodiment, 2 different cyclic urea derivatives are contacted in the first step of the method of the invention.

The at least two different cyclic urea derivatives are *N*,*N*'-(1,2-ethanediyl)-urea derivatives according to formula (I):

(I) 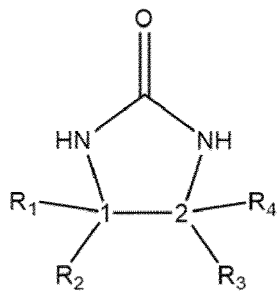

wherein 1 and 2 are carbon atoms and at least one of them is a stereogenic center, preferably both of them are stereogenic centers; wherein at least one of the substituents R1 to R4 bears a stereogenic center; and wherein $R_1$, $R_2$, $R_3$, and $R_4$ can each independently be selected from the group consisting in a hydrogen atom, -OH, -COOH, $-NH_2$, $-NO_2$, $-NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl.

Preferably, the at least two different cyclic urea derivatives are selected from a group consisting in 4,5-Dimethyl-2-imidazolidinone, 4-alkyl-2-imidazolidinone, 2-imidazolidinone, rel-(3aR,6aS)-Hexahydropyrrolo[3,4-d]imidazol-2(1H)-one, 2-Oxo-4-imidazolidinecarboxylic acid, 2-Oxo-4-imidazolidinecarbonitrile, 4-(Aminomethyl)-2-imidazolidinone, 4-(Hydroxymethyl)-2-imidazolidinone, hexahydro-2(1H)-Cyclopentimidazolone, 4-(2-Hydroxyethyl)-2-imidazolidinone, rel-(3aR,6aS)-Tetrahydro-1H-furo[3,4-d]imidazol-2(3H)-one, Octahydroimidazo[4,5-d]azepin-2(1H)-one, 4-(Ethoxymethyl)-2-imidazolidinone, 4,5-Dipropyl-2-imidazolidinone, (4R or 4S)-4-Phenyl-2-imidazolidinone, (4S or 4R)-2-Oxo-4-imidazolidineacetic acid, 8-Methyl-1,3,7-triazaspiro[4.4]nonan-2-one, (4S or 4R)-4-(4-Pyridinyl)-2-imidazolidinone, 4,5-Dialkyl-2-imidazolidinone, 2-Oxo-4-imidazolidinepropanoic acid, 4-(4-Hydroxyphenyl)-2-imidazolidinone, 1H-Thieno[3,4-d]imidazol-2(3H)-one, tetrahydro-, 5,5-dioxide, (3aR,6aR)-Spiro[1-azabicyclo[2.2.2]octane-3,4'-imidazolidin]-2'-one, 2-[(4R)-2-Oxo-4-imidazolidinyl]benzonitrile, ethylene urea, *N*,*N*'-cyclohexa-1,2-diylurea, D-biotin, their stereoisomers thereof, and their derivatives thereof, preferably ethylene urea, *N*,*N*'-cyclohexa-1,2-diylurea, D-biotin, their stereoisomers thereof, and their derivatives thereof.

Preferably, the cyclic urea derivatives of the invention are enantiopure.

In a preferred embodiment, 2 different cyclic urea derivatives are contacted in the first step of the method of the invention and a first cyclic urea derivative is (*S,S*)-*N,N'*-cyclohexa-1,2-diylurea or (*R*,*R*)-*N*,*N*'-cyclohexa-1,2-dylurea.

In another preferred embodiment, 2 different cyclic urea derivatives are contacted in the first step of the method of the invention and a second cyclic urea derivative is the D-biotin.

In an even preferred embodiment, 2 different cyclic urea derivatives are contacted in the first step of the method of the invention and the first cyclic urea derivative is (*S,S*)-*N,N'*-cyclohexa-1,2-diylurea or (*R*,*R*)-*N*,*N*'-cyclohexa-1,2-dylurea and the second cyclic urea derivative is D-biotin.

One of the cyclic urea derivatives contacted in the first step of the method of the invention is the main cyclic urea derivative and the initial ratio of the other cyclic urea derivatives on the main cyclic urea derivative is comprised between about 1/50 and about 1, preferably about 1/30 and about 3/4, more preferably between about 1/20 and about 1/2, still

preferably between about 1/15 and about 1/5, even more preferably the initial ratio of the other cyclic urea derivatives on the main cyclic urea derivative is of about 1/7.

Preferably, 2 different cyclic urea derivatives are contacted in the first step of the method of the invention and the initial ratio of the second/other cyclic urea derivative on the first/main cyclic urea derivative is comprised between about 1/50 and about 1, preferably about 1/30 and about 3/4, more preferably between about 1/20 and about 1/2, still preferably between about 1/15 and about 1/5, even more preferably the initial ratio of the second/other cyclic urea derivative on the first/main cyclic urea derivative is of about 1/7.

More preferably, the first/main cyclic urea derivative is (*S,S*)-*N,N'*-cyclohexa-1,2-diylurea or (*R*,*R*)-*N*,*N'*-cyclohexa-1,2-dylurea, the second/other cyclic urea derivative is D-biotin and the initial ratio of the D-biotin on (*S*,*S*)-*N*,*N'*-cyclohexa-1,2-diylurea or (*R,R*)-*N,N'*-cyclohexa-1,2-dylurea is comprised between about 1/50 and about 1, preferably about 1/30 and about 3/4, more preferably between about 1/20 and about 1/2, still preferably between about 1/15 and about 1/5, even more preferably the initial ratio of D-biotin on (*S,S*)-*N,N'*-cyclohexa-1,2-diylurea or (*R*,*R*)-*N*,*N'*-cyclohexa-1,2-dylurea is of about 1/7.

In the first step of the method of the invention, a compound that can form methylene bridges is contacted with at least two different cyclic urea derivatives.

As used herein, the term "compound that can form methylene bridges" refers to a compound selected from the list consisting in formaldehyde, paraformaldehyde, formalin, trioxane, or alkenes such as ethene or isoprene, preferably the compound that can form methylene bridges is formaldehyde.

The initial ratio between the compound that can form methylene bridges and the monomers of cyclic urea derivatives is comprised between about 0.5 and about 2, preferably between about 0.7 and about 1.5, more preferably between about 0.85 and about 1.25 and even more preferably the ratio between the compound that can form methylene bridges and the monomers of cyclic urea derivatives is of about 1.

The first step of contacting of the method of the invention takes place in the presence of at least one acid, and at least one solvent.

In the method of the invention n is an even positive integer comprised between 4 and 14, preferably n is comprised between 6 and 14. When n is comprised between 6 and 14, the method of the invention further comprises at least one templating anion.

The method of the invention may comprise one or several templating anions, preferably one templating anion. If the method of the invention comprises several templating anions, they are preferably templating for the same chimeric hemicucurbit[n]uril. The templating anion according to the invention is preferably selected from the group consisting in $Cl^-$, $Br^-$, $I^-$, $BF_4^-$, $IO_4^-$ , $ClO_4^-$ , $ReO_4^-$, $PF_6^-$, $SbF_6^-$, $SO_4^{2-}$, $BO_3^{2-}$, $NO^{3-}$, $PO_3^{3-}$, $PO4^{2-}$, and $RCO_2^-$, R being selected from the group consisting in hydrogen atom, -OH, -COOH, $-NH_2$, $-NO_2$, $-NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl, or a combination thereof, preferably the templating anion according to the invention is $PF_6^-$.

The total amount of templating anions according to the method of the invention is initially comprised between about 0.2 and about 10 eq, preferably between about 0.5 and about 6 eq, more preferably between about 1.2 eq and about 5 eq, still preferably between about 1.5 eq and about 4 eq, even more preferably the total amount of templating anions is initially of about 3 eq.

Templating anions in the reaction mixture result from the dissociation of acid(s) and/or salt(s) in the presence of the solvent.

In the method according to the invention, one or a combination of different acids can be used. The acid(s) is (are) initially present in a quantity comprised between about 0.2 and about 5 eq, preferably between about 0.5 and about 4.5 eq, more preferably between about 1.2 eq and about 3.5 eq, even more preferably the acid(s) is(are) initially present in a quantity of about 3 eq. The acid(s) according to the invention can be any acid, such as organic acids, mineral acids or a combination thereof, preferably mineral acids.

As used herein, the terms "mineral acid" or "inorganic acid" are equivalent and refer to any acid derived from an inorganic compound that dissociates to produce protons ($H^+$).

The acid(s) according to the invention preferably have a $pK < 3$, more preferably a $pK < 2$, even more preferably a $pK < 1$.

In a preferred embodiment, the acid(s) according to the method of the invention is (are) source(s) of templating anions.

As used herein, the term "source of templating anion" refers to a molecule that, upon dissociation in the presence of a solvent, free templating anions.

When the method of the invention comprises several acids, preferably only one type of templating anion is freed, meaning that either they are freeing the same templating anion or some acids are not freeing any templating anion. Alternatively, different templating anions can be freed that are templating for the same chimeric hemicucurbit[n]uril.

In a preferred embodiment, the method of the invention comprises only 1 acid. Preferably that acid is a mineral acid. Alternatively, that acid is a source of templating anions. In a most preferred embodiment, the method of the invention

comprises only 1 acid that is a mineral acid and a source of templating anions.

The acid(s) according to the method of the invention may be selected from any acid comprising one of the above-mentioned templating anions. In a preferred embodiment, the acid(s) according to the method of the invention is (are) selected from the group consisting in HCl, HBr, HI, $H_2SO_4$, $H_3BO_3$, $HNO_3$, $H_3PO_3$, $H_3PO_4$, $HReO_4$, $HClO_4$, $HPF_6$, $HSbF_6$, $HBF_4$, $HIO_4$, RCOOH, R being selected from or RCOOH, R being selected from the group consisting in hydrogen atom, -OH, -COOH, $-NH_2$, $-NO_2$, $-NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl, or a combination thereof, preferably the acid according to the method of the invention is $HPF_6$.

In a particular embodiment, the at least one acid according to the invention is the only source of templating anions. However, the method of the invention may comprise an additional source of templating anions, when the at least one acid of the method of the method of the invention comprises a source of templating anions, or alternative source of templating anions, when the at least one acid of the method of the invention is not a source of templating anion. For example, the method of the invention may comprise salt(s) as an additional or alternative source of templating anions.

The method according to the invention may comprise at least one salt, i.e. one or several sats. When the method of the invention comprises several salts, they are preferably freeing the same templating anion or at least different templating anions that are templating for the same chimeric hemicucurbit[n]uril. Preferably, the method of the invention comprises only one salt. The salt(s) according to the method of the invention may be selected from any salt comprising one of the above-mentioned templating anions. Preferably, the salt(s) according to the method of the invention are ionic compounds comprising a templating anion as above described and a cation, for example, a cation selected from the group consisting in $Al^{3+}$, $Am^{3+}$, $NH^{4+}$, $Ag^+$, $Ag(NH_3)^{2+}$, $Ba^{2+}$, $Be^{2+}$, $Cd^{2+}$, $Ca^{2+}$, $Cs^+$, $Cr^{3+}$, $Co^{2+}$, $Co^{3+}$, $Cu^+$, $Cu^{2+}$, $Cu(CH_3CN)_4{}^+$, $Sn^{2+}$, $Sn^{4+}$, $Fe^{2+}$, $Fe^{3+}$,$H_3O^+$, $Li^+$, $Mg^{2+}$, $Mn^{2+}$, $Mn^{4+}$, $Hg^+$, $Hg^{2+}$, $CH_5{}^+$, $CH^{3+}$,$Ni^{2+}$,$NO_2{}^+$, $NO^+$, $Au^{3+}$, $K^+$, $Pb^{2+}$, $Na^+$, $Sr^{2+}$, $U^{3+}$, $U^{4+}$, $UO_2{}^{2+}$, or $Zn^{2+}$.

A salt according to the invention can thus be, for example $AgPF_6$, $[Cu(CH_3CN)4]PF_6$ or $KPF_6$.

According to the choice of templating anions, a hemicucurbituril with a specific number of monomers per macrocycle, *n*, can be produced. For a hemicucurbit[6]uril, the at least one templating anion according to the method of the invention is preferably selected from the group consisting in $Cl^-$, $Br^-$, $I^-$, $BF_4{}^-$, and a combination thereof, more preferably from $Cl^-$ or $Br^-$, even more preferably $Cl^-$. For a hemicucurbit[8]uril, the at least one templating anion according to the method of the invention is preferably selected from the group consisting in $IO_4{}^-$ $ClO_4{}^-$, $ReO_4{}^-$ $PF_6{}^-$, $SbF_6{}^-$, and a combination thereof, more preferably from $ClO_4{}^-$ and $PF_6{}^-$, even more preferably $PF_6{}^-$. For a hemicucurbit[10]uril, a hemicucurbit[12]uril or hemicucurbit[14]uril, the templating anion is preferably selected from the group consisting in anions with a diameter larger than 0.8 nm such as nano-anions, for example the boron-rich cobaltabisdicarbollide (COSAN) or polyoxametallates. For hemicucurbit[12]uril, carboxylate, such as fluorinated carboxylate are preferred.

As used herein, the term "nano-ion" refers to multi-atomic entities which diameter is in the nanometric range, i.e. comprised between about 1 and about 10 nm, preferably about 10 nm.

In a most preferred embodiment, the method of the invention comprises $HPF_6$ as an acid and $KPF_6$ as a salt. Preferably, the HPF6/KPF6 ratio is of about 2/1.

In the method according to the invention, the acid and eventually the salt are reacted in the presence of the at least one solvent that act as an assisting liquid during grinding. The at least one solvent is selected from the group consisting in apolar or polar solvents like alkanes, haloalkanes, arenes, heteroarenes, ethers, nitromethane, acetonitrile, solvent and water or a combination thereof, preferably water.

In the method of the invention, the amount of liquid per amount of solid in the reaction media, $\eta$, is initially comprised between about 0.1 $\mu$L/mg and about 1 $\mu$L/mg, preferably between about 0.1 $\mu$L/mg and about 0.5 $\mu$L/mg, more preferably $\eta$ is of about 0.2 $\mu$L/mg.

Preferably $\eta$ remains comprised between about 0.1 $\mu$L/mg and about 1 $\mu$L/mg, preferably between about 0.1 $\mu$L/mg and about 0.5 $\mu$L/mg, more preferably $\eta$ is of about 0.2 $\mu$L/mg during the full length of the method of the invention, including the first step of contacting and the second step of aging.

The first step of contacting of the method of the invention takes place under mechanical agitation. There are different ways to provide mechanical agitation that are well known from the man skilled in the art. Apart from manual grinding, which is open and susceptible to a range of environmental factors, ball or planetary milling, extrusion or resonance accusting milling offers an enclosed solvent-free reaction environment with well-defined parameters for optimizing reactivity, such as frequency, medium-to-sample weight ratio, etc. (Stolle, A. et al, Faraday Discuss. 2014, 170, 267- 286; Schmidt, R. et al, Org. Process Res. Dev. 2015, 19, 427- 436). Several type of ball milling devices are well known from the man skilled in the art, including, without limitation, shaker mills, planetary mills and roller mills such as industrial-scale roller mills and extruders.

Preferably, the mechanical agitation of the method of the invention is performed by ball milling. Preferably, the ball milling mechanical agitation frequency of the method of the invention is comprised within the range of about 10 Hertz and about 50 Hertz, more preferably within the range of about 20 Hertz and about 40 Hertz, even more preferably the ball milling mechanical agitation frequency of the method of the invention is of about 30 Hertz.

Alternatively, when not performed by ball milling, the mechanical agitation is performed at a mechanical force equivalent to the mechanical force generated by ball milling mechanical agitation at a frequency comprised within the range of about 10 Hertz and about 50 Hertz, more preferably within the range of about 20 Hertz and about 40 Hertz, even more preferably of about 30 Hertz.

In the method according to the invention, the first step of contacting last at least about 10 minutes, preferably between about 15 minutes and about 90 minutes, more preferably between about 15 minutes and about 60 minutes, still preferably between about 15 minutes and about 40 minutes, even more preferably the first step of contacting last about 20 min.

Preferably, the first step of contacting last for a time period equivalent to at least about 10 minutes at a mechanical force of about 30Hz with ball milling, preferably between about 15 minutes at a mechanical force of about 30Hz with ball milling and about 90 minutes at a mechanical force of about 30Hz with ball milling, more preferably between about 15 minutes at a mechanical force of about 30Hz with ball milling and about 60 minutes at a mechanical force of about 30Hz with ball milling, still preferably between about 15 minutes at a mechanical force of about 30Hz with ball milling and about 40 minutes at a mechanical force of about 30Hz with ball milling, even more preferably the first step of contacting last about 20 min at a mechanical force of about 30Hz with ball milling.

In a second step according to the method of the invention, the products obtained at the contacting steps are aging for at least about 30 minutes.

As used herein, the term "aging" refers to the storage of crude reaction mixture without any mechanical agitation. Self-assembly of crude reaction mixture occurs during aging, which can result in the formation of new products and/or stabilization and increase of content of specific products.

The step of aging of the method of the invention last at least about 30 minutes, preferably at least about 1 hour, more preferably at least about 1 hour and 30 minutes, still preferably between about 1 hour and 30 minutes and about 24 hours, yet preferably between about 2 hours and about 16 hours, even more preferably between about 2 hours and 30 minutes about 12 hours. In a most preferred embodiment, the aging of the method of the invention last about 3 hours.

In the method of the invention, the aging step takes place at a temperature comprised between about 0°C and about 100°C, preferably between about 20°C and about 85°C, more preferably between about 35°C and about 75°C, still preferably between about 40°C and about 65°C, and even more preferably at a temperature of about 60°C.

The method of the invention is producing at least one chimeric hemicucurbit[n]uril. The method of the invention may produce between 1 and 20 different chimeric hemicucurbit[n]urils, preferably between 1 and 15 different chimeric hemicucurbit[n]urils, still preferably between 1 and 10 different chimeric hemicucurbit[n]urils, even more preferably between 1 and 5 different chimeric hemicucurbit[n]urils.

The chimeric hemicucurbit[n]urils produced by the method of the invention can be as above described in the first aspect of the invention.

Preferably, the at least one chimeric hemicucurbit[n]uril is obtained at a yield of at least about 10%, preferably of at least about 20%, more preferably at a yield of at least about 30%, even more preferably at a yield of at least about 35%.

The method of the invention may further comprise a step of sealing the container in which the contacting step took place prior to the aging step. The seal can be a parafilm seal.

The method of the invention may further comprise an additional step of washing at the end of the aging step. Methods for washing the products of a solid-state reaction are well known from the man skilled in the art. For example, the resulting crude mixture resulting from the aging step can be washed with distilled water, for example on a glass filter, until neutral pH is reached. By washing the products of the reaction, the reaction mixture is quenched, i.e. there will be no more evolution in the products composition.

The method of the invention may further comprise an additional step of drying after the washing step. Methods for drying the products of a solid-state reaction are well known from the man skilled in the art. For example, the washed products of reaction can be dried in open air at, room temperature.

The method of the invention may comprise several cycles of washing/drying as above described. The method of the invention may thus comprise between 2 and 12 cycles of washing/drying, for example 8 cycles of washing/drying.

The method of the invention may further comprise an additional step of purification of a the at least one chimeric hemicucurbit[n]uril produced by said method. Methods of purification like extraction and chromatography are well known by the man skilled in the art. For example, the chimeric hemicucurbit[n]uril according to the invention can be converted into a potassium salt. For that purpose, a solution of KOH/MeOH at about 3% to about 10%, preferably about 5%, can be added to the dry quenched reaction mixture, followed by stirring for about 30 minutes to about 1 hour, preferably about 45 minutes. MeOH and the water can then be evaporated via azeotropic removal with toluene (for example 50 mL, 3 times). Additional steps can be added to remove unwanted macrocycles, for example non chimeric ones. The example part provides examples of such a step. The remaining mixture is then dissolved in about 200 mL to about 500 mL, preferably about 350 mL, of distilled water. The mixture is then acidified to a pH ranging from about 2 to about 3. The precipitated obtained at the previous step is then filtered and dried. The dried solid is then purified by flash column chromatography (for example with a gradient 0-50% iPrOH (1% acetic acid)/$CH_2Cl_2$).

The method of the invention may produce additional non-chimeric hemicucurbit[n]urils as side-products. Therefore, in order to increase the overall yield of production of chimeric hemicucurbit[n]uril, after separation of the non-chimeric hemicucurbit[n]uril from the chimeric hemicucurbit[n]uril, the method of the invention may further comprise a step of crossover reaction between the non-chimeric hemicucurbit[n]uril and the other cyclic urea derivatives according to the invention, in order to produced additional chimeric hemicucurbit[n]uril. An example of such a crossover reaction is further developed in the example section with cyclohexanohemicucurbit[8]uril and D-biotin in order to produce mono-biotinylated cyclohexanohemicucurbit[8]uril.

The method of the invention may further comprise a step of contacting the at least one chimeric hemicucurbit[n]uril obtained at the end of the aging step with an additional group selected from the group consisting in fluorescent dyes such as free-base and metallo porphyrins, for example -tetraphenylporphyrin ($H_2$TPP), metallo-tetraphenylporphyrin, said metallo group can be any metal, for example Zinc (ZnTPP), 4,4-difluoro-4-bora-3a,4a-diaza-s-indacene (BODIPY) derivatives, amines derivatives fluorescent dyes such as benzyl amines fluorescent dyes and alcohol derivatives fluorescent dyes. This additional contacting step will be performed under conditions compatible with adjacent multifunctional hemicucubituril core structure or will contain pretreatment step for example for protection of sulphide fragment of biotin.

### *Product obtained by the method*

In a third aspect, the invention also concerns a chimeric hemicucurbit[*n*]uril obtained by the method of the invention, as above- described in the second aspect of the invention.

### *Other products*

In a fourth aspect, the invention also concerns a chimeric hemicucurbit[*n*]uril-modified material wherein the chimeric hemicucurbit[*n*]uril is as above-described in the first or third aspects of the invention.

As used herein, the term "chimeric hemicucurbit[n]uril-modified material" refers to any material, such as a solid surface or an insoluble compound, on which chimeric hemicucurbit[*n*]urils according to the method of the invention can be attached, for example covalently. Suitable materials for attaching chimeric hemicucurbit[*n*]urils according to the invention are well known from the man skilled in the art. Such material can be, for example as described in the following publication, the content of which being thereby incorporated by reference: Polym. Chem., 2019,10, 3674-3711, ACS Nano 2020, 14, 10, 12491-12521. Such a material include, without limitations, a metal surface, a silica support, an alumina support and a polymer support, preferably a silica support. For example, chimeric hemicucurbit[n]urils of the invention can be attached to the 3-aminopropyl silico gel.

### *Use of the Product*

In a fifth aspect, the invention also concerns the use of a chimeric hemicucurbit[*n*]uril, as described above in the first or third aspect of the invention, as well as a chimeric hemicucurbit[n]uril-modified material, as described above in the fourth aspect of the invention, for binding anions, cations or organic molecules.

The chimeric hemicucurbit[*n*]uril of the invention are particularly effective in binding anions selected from the group consisting in $F_6^-$, $SbF_6^-$, $ClO_4^-$, $I^-$, $NO_3^-$, $Cl^-$, $ReO_4^-$, $SCN^-$, $H_2PO_4^-$, $Br^-$, $HSO_4^-$, $CF_3SO_3^-$, $NO_2^-$, and $BF_4^-$.

The chimeric hemicucurbit[*n*]uril of the invention can also bind cations through strong electrostatic interactions between said cation and a first anion bound to the chimeric hemicucurbit[*n*]uril.

The chimeric hemicucurbit[*n*]uril of the invention can also bind organic molecules, especially organic molecules that are hydrophobic and/or electron rich, for example organic compounds with S and O- atom, heterocycles and/or haloalkanes containing chloride and bromide.

For example, the chimeric hemicucurbit[n]urils-modified materials of the invention can be used as a stationary phase (solid phase) in chromatography, such as HPLC, for laboratory, industrial or pharmaceutical use, allowing in particular:

a. purifying a solvent phase from anions or cations and organic compounds, including purification of organic compounds according to their chirality (industrial purification, waste management, pharmaceutical purification, dialysis of biological fluids).

b. extracting anions, cations or organic compounds from the solvent or gas phase for further analysis, sensing or use.

c. trapping anions, cations or organic compounds and thereby inhibiting a reaction in a solvent phase that is dependent on said anions, cations or organic compounds.

For example, the chimeric hemicucurbit[n]urils of the invention can be immobilized on the surface of 3-aminopropylsilico gel. It can be used, for example, to remove perchlorate from contaminated soil.

Chimeric hemicucurbit[*n*]urils of the invention can be also be immobilized on the surface of dextran matrix such as a Biacore Sensor Chip CM5 and use in sensing studies.

The chimeric hemicucurbit[*n*]uril(s) of the invention can also be dissolved in a solvent and thus serve for anion, cation or organic compounds caption for laboratory, industrial or pharmaceutical use. They can for example be used for:

a. inhibiting specific anion dependent chemical or enzymatic reactions in solution.
b. trapping toxic anions, cations or organic compounds for the purpose of treatment or drug.
c. trapping anions, cations or organic compounds for the purpose of sensing.

For their ability to selectively bind some anions and cations, chimeric hemicucurbit[n]urils of the invention can as well be used as ion-exchange materials.

Chimeric hemicucurbit[*n*]urils of the invention can be also used for the preparation of sensors for anion and cation detection and for the extraction, transportation, and regulation of an anion.

For example, the chimeric hemicucurbit[n]urils of the invention can be covalently functionalized with chromophore like porphyrin, BODYPY, fluorescein and other optically signalling compounds and used as supramolecular optical sensors.

Thanks to their ability to bind various compounds, chimeric hemicucurbit[*n*]urils of the invention may also be used for drug transportation and targeting in organisms, or in food and pharmaceutical industry as a matrix bearing aromatic or curative compounds.

Chimeric hemicucurbit[*n*]urils of the invention can be used for water purification, desalination, removal of various ions and/or compounds from the solution (for example polar and non-polar organic solvents, water) separation of liquid mixtures and mixtures of gases and organic solvent vapors. Water can be purified by chimeric hemicucurbit[*n*]urils according to the invention in powder form or incorporated as nanoadditive into polymeric membranes. Polymeric membranes containing chimeric hemicucurbit[*n*]urils can be also used for the separation of liquid mixtures, and mixtures of gases and organic solvent vapors using the pervaporation, permeation, and sorption techniques.

Further aspects and advantages of this invention are disclosed in the following experimental section, which should be regarded as illustrative and not limiting the scope of this application.

## *Examples*

### Example 1 - mixHC[8] synthesis

Material & Method

*General information*

Unless otherwise stated, all reagents were purchased from commercial suppliers and used without further purification. (*R,R*)- and (*S,S*)-*N,N'*-cyclohexa-1,2-diylurea monomers, (*R,R*)-cyclohexanohemicucurbit[6]uril (*R,R*)-cycHC[6]) and (*R,R*)-cyclohexanohemicucurbit[8]uril ((*R,R*)-cycHC[8]) were synthesized in the inventors laboratory following the procedures described in literature.[1-3]

Mechanochemical experiments were carried out in FTS-1000 ball mill, at a frequency of 30 Hz in 14 mL $ZrO_2$-coated grinding jars charged with two 10 mm $ZrO_2$ milling balls. For aging reaction mixtures at elevated temperatures VWR Incu-line Digital Incubator was used.

$^1H$, $^{13}C$ and 2D nuclear magnetic resonance (NMR) spectra were acquired using a Triple Resonance Probe (TXI) on a Bruker AVANCE III 800 MHz spectrometer at 298.15 K. The samples were dissolved in $CDCl_3$ and the chemical shifts were referenced to CDCl3 residual peak. Optical rotation was measured with an Anton Paar MCP 500 polarimeter. IR spectra were recorded on a Bruker Tensor 27 FT spectrometer. High-resolution mass spectrometry (HRMS) data was collected on Agilent 6540 Accurate-Mass Q-TOF and Agilent 6560 UHD LC-IMQTOF mass spectrometers. Thermodynamic measurements by isothermal titration calorimetry (ITC) were performed on MicroCal PEAQ-ITC calorimeter using a 200 μL calorimetric cell and a 40 μL syringe.

Silica gel 40-63 μm was used for column chromatography, silica gel 60 $F_{254}$ plates were used for TLC. Visualization of TLC plates was performed using phosphomolybdic acid (PMA) stain. To perform automatic column chromatography Biotage Isolera Prime machine was used. HPLC-UV-MS analysis was performed on Agilent 1200 Series System equipped with multiple wavelength detector (MWD) and single quadrupole mass detector (MSD), using Phenomenex Kinetex XB-C18 column (150 mm × 4.6 mm, 2.6 μm). Ion chromatographic analysis was carried out on Metrohm Ltd 761 Compact IC with chemical suppression of eluent conductivity.

*General procedure for screening reaction conditions starting from monomeric units*

*D*-biotin (46 mg, 0.19 mmol, 1 eq.), (*R,R*)- or (*S*,*S*)-*N*,*N*'-cyclohexa-1,2-diylurea (183 mg, 1.30 mmol, 7 eq.), paraformaldehyde (45 mg, 1.50 mmol, 8 eq.) and triphenylmethane (10 mg, internal standard for HPLC yield estimation) were placed into a 14 mL $ZrO_2$-coated jar charged with two 10 mm $ZrO_2$ balls (3.5 g). The template consisting of aqueous acid or acid and corresponding salt (0.56 mmol, 3 eq.) was added to the mixture, which was then set to mill at 30 Hz for 60 minutes. After milling the jar was sealed with parafilm and the reaction mixture was aged at 60 °C for 24 hours. The resulting crude mixture was further washed on a glass filter with distilled water until neutral pH, which was determined by test strips. The quenched mixture was dried in open air at room temperature.

*General conditions for HPLC-UV-MS (High-Performance Liquid Chromatography-UV-Mass Spectrometry) analysis*

*System A* (estimation of HPLC yield). Eluents A (water / 0.1% formic acid) and B (acetonitrile / 0.1% formic acid) were used in a gradient mode from A:B 50:50 (v/v) to A:B 10:90 (v/v) with the flow rate of 0.75 mL/min. The column temperature was set at 30 °C, injection volume at 2 μL and detection wavelength at 210 nm. The peaks of the macrocycles were identified by ESI-MS.

HPLC yields were calculated based on calibration graphs for isolated macrocycles and internal standard (triphenylmethane) introduced into reaction mixture before milling. The sampling was performed in triplicate, ~1 mg of crude mixture after aging was dissolved in chloroform: isopropanol 1:1 (v/v). If necessary, the solution was filtered through 0.2 μm PTFE syringe filter.

*System B* (MS analysis of oligomers). Eluents A (water / 0.1% formic acid) and B (acetonitrile / 0.1% formic acid) were used in a gradient mode from A:B 90:10 (v/v) to A:B 0:100 (v/v), followed by the isocratic stage at A:B 0:100 (v/v). The flow rate was set at 0.5 mL/min, column temperature at 30 °C and injection volume at 2-5 μL. The general oligomeric profile was studied by ESI-HRMS in scan mode with positive polarity. The quantitative analysis was carried out using SIM methodology, following the target m/z values corresponding to the chosen oligomeric species $[M+H]^+$ and $[M+Na]^+$. The samples were prepared as described above and diluted if necessary for SIM analysis.

Optimization studies

*1.1.1 Synthesis using cycHC[n] as a starting material*

General synthesis procedure for screening reaction with cycHC[6] as starting material (cf. Figure 1): D-biotin (56 mg, 0.23 mmol, 1 eq.) and (R,R)-cyclohexanohemicucurbit[6]uril (249 mg, 0.270 mmol, 1.2 eq.) and 70% aq. $HClO_4$ (60 μl, 0.69 mmol, 3 eq.) were milled for 60 minutes and aged at 60 °C for 24 hours.

General synthesis procedure for screening reaction with cycHC[8] as starting material (cf. Figure 1): D-biotin (55 mg, 0.23 mmol, 1 eq.) and (R,R)-cyclohexanohemicucurbit[8]uril (240 mg, 0.196 mmol, 0.9 eq.) and 70% aq. $HClO_4$ (58 μl, 0.68 mmol, 3 eq.) were milled for 60 minutes and aged at 60 °C for 24 hours.

**Table 1.** Screening reactions using cycHC[n] macrocycles as starting material

| Entr y | Biotin:cycHC [*n*] molar ratio[a] | HCl $O_4$, eq. | HPLC yield[b], % | |
|---|---|---|---|---|
| | | | mixHC[8] | cycHC[8] |
| 1 | 1:1.2 (cycHC[6]) | 3 | 9.8±0.2 | 47±1 |
| 2 | 1:0.9 (cycHC[8]) | 3 | 9.3±0.2 | 51±1 |

[a] *Ratio of starting materials was chosen to satisfy 1:7 ratio of monomers in mixHC[8].*
[b] *Each reaction was performed once, the HPLC yields are provided as the mean value ± standard deviation between triplicate measurements.*

*1.1.2 Synthesis from monomers*

Reaction conditions for screening using RSM: D-biotin (cf. Figure 2): (25.9-65.2 mg, 0.106-0.267 mmol, 1 eq.), (R,R)-N,N'-cyclohexa-1,2-diylurea (186.7-224.7 mg, 1.3319-1.6029 mmol, 5-15 eq.), paraformaldehyde (48.0-52.1 mg, 1.60-1.74 mmol, 8 eq.) and 70% aq. $HClO_4$ (8.6-75 μl, 0.10 - 0.87 mmol, 0.5-4 eq.) were milled for 10-60 minutes and aged at 35-75 °C for 24 hours.

***Screening different templates***

**Table 2.** Comparison of various acids and salt additives

| Entry | Acid (eq.) | Salt (eq.) | Template anion | HPLC yield, % | |
|---|---|---|---|---|---|
| | | | | mixHC[8] | cycHC[8] |
| 1[a] | $HClO_4$ (3)[4] | - | $ClO_4^-$ | 20±2 | 4113 |
| 2[b] | $HPF_6$ (3/2.4) | - | $PF_6^-$ | 27.6±0.3 | 34±2 |
| 3[c] | $H_2SO_4$ (3) | $KPF_6$ (3/1.5) | $PF_6^-$ | 15±1 | 22±3 |
| 4[d] | $HPF_6$ (2) | $KPF_6$ (1) | $PF_6^-$ | 37±2 | 38±2 |
| 5[e] | $HPF_6$ (2) | $AgPF_6$ (1) | $PF_6^-$ | 29±2 | 42±6 |
| 6[f] | $HPF_6$ (2) | $[Cu(CH_3CN)_4]PF_6$ (1) | $PF_6^-$ | 31.1±0.4 | 44±1 |

*Reaction conditions: D-biotin (38-52 mg, 0.15-0.21 mmol, 1 eq.), (R,R)-N,N'-cyclohexa-1,2-diylurea (151-208 mg, 1.08-1.48 mmol, 7 eq.), paraformaldehyde (38-52 mg, 1.3-1.7 mmol, 8 eq.) and template (3 eq.) were milled for 60 minutes and aged at 60 °C for 24 hours. The acid /salt combinations tested as the suitable template: [a] 70% aq. $HClO_4$ (50-55 $\mu$l, 0.58-0.59 mmol, 3 eq.), [b] 55% aq. $HPF_6$ (72-89 $\mu$l, 0.45-0.56 mmol, 2.4-3 eq.), [c] aqueous 25% and 50% $H_2SO_4$ (65-75 $\mu$l, 0.46-0.54 mmol, 1.5-3 eq.) and $KPF_6$ (51-87 mg, 0.28-0.47 mmol, 3 eq.), [d] 55% $HPF_6$ (60$\mu$l, 0.37 mmol, 2 eq.) and $KPF_6$ (34-35 mg, 0.19-0.19 mmol, 1 eq.), [e] 55% $HPF_6$ (55-57 $\mu$l, 0.34-0.36 mmol, 2 eq.) and $AgPF_6$ (43-46 mg, 0.17-0.18 mmol, 1 eq.), [f] 55% $HPF_6$ (53 $\mu$l, 0.33 mmol, 2 eq.) and $[Cu(CH_3CN)4]PF_6$ (61-61 mg, 0.16-0.165 mmol, 1 eq.). The HPLC yields are provided as the mean value ± standard deviation for the reaction reproduced [a] 8, [d] 4 or [b, c, e, f] 2 times.*

**Table 3.** Screening ratios of starting monomers

| Entry | Monomer molar ratio | HPLC yield, % | |
|---|---|---|---|
| | | mixHC[8] | cycHC[8] |
| 1[a] | 1:5 | 34±1 | 30±1 |
| 2[b] | 1:7 | 37±2 | 38±2 |
| 3[c] | 1:15 | 27±1 | 61.8±0.4 |

*Reaction conditions: [a] D-biotin (60 mg, 0.25 mmol, 1 eq.), (R,R)-N,N'-cyclohexa-1,2-diylurea (172 mg, 1.23 mmol, 5 eq.), paraformaldehyde (44 mg, 1.5 mmol, 6 eq.), $KPF_6$ (34 mg, 0.19 mmol, 1 eq.) and 55% aq. $HPF_6$ (59 $\mu$l, 0.37 mmol, 2 eq.) as LAG additive; [b] D-biotin (45-46 mg, 0.19 mmol, 1 eq.), (R,R)-N,N'-cyclohexa-1,2-diylurea (182-184 mg, 1.30-1.31 mmol, 7 eq.), paraformaldehyde (44-45 mg, 1.5 mmol, 8 eq.), $KPF_6$ (35 mg, 0.19 mmol, 1 eq.) and 55% aq. $HPF_6$ (60$\mu$l, 0.37 mmol, 2 eq.) as LAG additive; c D-biotin (23 mg, 0.094 mmol, 1 eq.), (R,R)-N,N'-cyclohexa-1,2-diylurea (198 mg, 1.41 mmol, 15 eq.), paraformaldehyde (45 mg, 1.19 mmol, 16 eq.), $KPF_6$ (35 mg, 0.19 mmol, 1 eq.) and 55% aq. $HPF_6$ (61 $\mu$l, 0.34 mmol, 2 eq.) as LAG additive were milled at 30 Hz for 60 minutes and aged at 60 °C for 24 hours. Reactions 1 and 3 were performed once, reaction 2 was performed 4 times; the HPLC yields are provided as the mean value ± standard deviation for the triplicate measurement.*

**Table 4.** Screening milling time

| Entry | Milling time, min | HPLC yield, % | |
|---|---|---|---|
| | | mixHC[8] | cycHC[8] |
| 1[a] | 5 | 16±1 | 55±3 |
| 2[a] | 10 | 30±1 | 39±2 |
| 3[a] | 20 | 37±1 | 40±1 |
| 4[b] | 30 | 33±4 | 32±3 |
| 5[b] | 45 | 26±4 | 29±4 |

(continued)

| Entry | Milling time, min | HPLC yield, % | |
|---|---|---|---|
| | | mixHC[8] | cycHC[8] |
| 6[a] | 60 | 36±1 | 38±2 |
| 7[C] | 90 | 34±1 | 32±1 |

*Reaction conditions: D-biotin (45-46 mg, 0.19 mmol, 1 eq.), (R,R)-N,N'-cyclohexa-1,2-diylurea (182-184 mg, 1.30-1.31 mmol, 7 eq.), paraformaldehyde (44-46 mg, 1.5-1.5 mmol, 8 eq.) and $KPF_6$ (34-35 mg, 0.19 mmol, 1 eq.) and 55% aq. $HPF_6$ (60 μl, 0.37 mmol, 2 eq.) were milled for 5-90 minutes and aged at 60 °C for 24 hours. The HPLC yields are provided as the mean value ± standard deviation for the reactions repeated [a] 3, [b] 4 or [c] 2 times.*

**Table 5.** Screening aging time

| Entry | Aging time, h | HPLC yield, % | |
|---|---|---|---|
| | | mixHC[8] | cycHC[8] |
| 1 | 0 | 13±2 | 13±2 |
| 2 | 3 | 38±1 | 34±1 |
| 3 | 6 | 37±1 | 35±1 |
| 4 | 12 | 31±1 | 33±2 |
| 5 | 24 | 36.4±0.4 | 39±3 |

*Reaction conditions: D-biotin (45-46 mg, 0.19 mmol, 1 eq.), (R,R)-N,N'-cyclohexa-1,2-diylurea (183- mg, 1.31 mmol, 7 eq.), paraformaldehyde (45 mg, 1.5 mmol, 8 eq.), $KPF_6$ (34-35 mg, 0.19 mmol, 1 eq.) and 55% aq. $HPF_6$ (60 μl, 0.37 mmol, 2 eq.) were milled for 60 minutes and aged at 60 °C for 0, 3, 6, 12 or 24 hours. The HPLC yields are provided as the mean value ± standard deviation for each reaction performed twice.*

<u>Analysis of Oligomers and Side-Products</u>

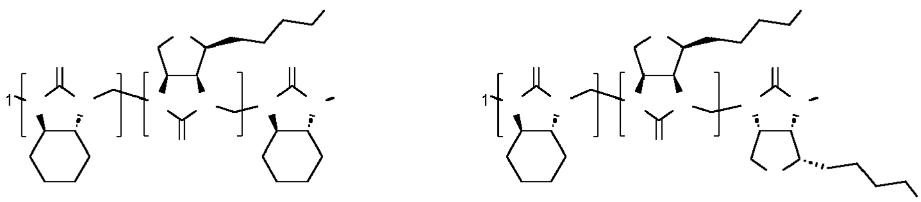

x, = +

1, : CHO, CH CH CH ,

The terminal groups of oligomers formed during synthesis can react with isopropanol used in the solvent mixture for sample preparation (chloroform : isopropanol 1 : 1), which results in $-CH_2OC_3H_7$ substituent forming upon analysis.

General oligomeric and macrocyclic profile of crude reaction mixtures was studied on Agilent 6540 Accurate-Mass Q-TOF mass-spectrometer, using the HPLC setup from system B. The mass spectra were acquired in positive polarity, mass range *m*/*z* 50 - 3200 and fragmentor voltage 175 V. The AJS-ESI source parameters were set as follows: gas temperature 200 °C, drying gas flow 6 L/min, nebulizer pressure 30 psi, sheath gas temperature 200 °C, sheath gas flow 8 L/min, capillary voltage 3500 V, nozzle voltage 1000 V, skimmer voltage 65 V, octopole peak-peak voltage 750 V. Prior to analysis the instrument was tuned in *m*/*z* 50-3200 mass range.

**General oligomeric profile (HPLC-HRMS, scan mode)**

| | | | |
|---|---|---|---|
| **A** | 5 min milling | **G** | 5 min milling + 24 h aging |
| **B** | 10 min milling | **H** | 10 min milling + 24 h aging |
| **C** | 20 min milling | **I** | 20 min milling + 24 h aging |
| **D** | 30 min milling | **J** | 30 min milling + 24 h aging |
| **E** | 45 min milling | **K** | 45 min milling + 24 h aging |
| **F** | 60 min milling | **L** | 60 min milling + 24 h aging |

Reaction conditions: *D*-biotin (45-46 mg, 0.19 mmol, 1 eq.), (*R,R*)-*N,N'*-cyclohexa-1,2-diylurea (183 mg, 1.31 mmol, 7 eq.), paraformaldehyde (45 mg, 1.50 mmol, 8 eq.) and $KPF_6$ (34-35 mg, 0.19 mmol, 1 eq.) and 55% aq. $HPF_6$ (60 µl, 0.37 mmol, 2 eq.) were milled for 5-60 minutes and aged 60 °C for 24 hours. The samples for analysis were taken after milling and after aging.

**Table 6**. Identification of oligomers and macrocycles in crude reaction mixtures after different milling time

| # | Oligomer | $R_1$ | $R_2$ | Ion | m/z exp | m/z theor | Samples after milling | | | | | | Samples after aging | | | | | |
|---|---|---|---|---|---|---|---|---|---|---|---|---|---|---|---|---|---|---|
| | | | | | | | A | B | C | D | E | F | G | H | I | J | K | L |
| 1 | $(CU)_2$ | H | H | $[M+H]^+$<br>$[M+Na]^+$ | 293.1970±0.0008<br>315.1797±0.0003 | 293.1972<br>315.1791 | | | | | | | | | | | | |
| 2 | $(CU)_2$ | H | $=CH_2$ | $M^+$ | 305.1972±0.0028 | 305.1972 | | | | | | | | | | | | |
| 3 | $(CU)_2$ | H | CHO | $[M+Na]^+$ | 343.1749±0.0002 | 343.1740 | | | | | | | | | | | | |
| 4 | $(CU)_2$ | H | $CH_2OH$ | $[M+H]^+$<br>$[M+Na]^+$ | 323.2052<br>345.1920±0.0003 | 323.2078<br>345.1897 | | | | | | | | | | | | |
| 5 | $(CU)_2$ | $CH_2OH$ | $=CH_2$ | $M^+$ | 335.2078 | 335.2077 | | | | | | | | | | | | |
| 6 | $(CU)_2$ | CHO | CHO | $[M+Na]^+$ | 371.1694±0.0002 | 371.1689 | | | | | | | | | | | | |
| 7 | $(CU)_2$ | CHO | $CH_2OH$ | $[M+Na]^+$ | 373.1869±0.0005 | 373.1846 | | | | | | | | | | | | |
| 8 | $(CU)_2$ | $CH_2OH$ | $CH_2OH$ | $[M+H]^+$ | 353.2181 | 353.2183 | | | | | | | | | | | | |
| 9 | $(CU)_2$ | H | $CH_2OC_3H_7$ | $[M+H]^+$<br>$[M+Na]^+$ | 365.2556±0.0019<br>387.2368±0.0020 | 365.2547<br>387.2366 | | | | | | | | | | | | |
| 10 | $(CU)_2$ | $CH_2OC_3H_7$ | $=CH_2$ | $M^+$ | 377.2564±0.0017 | 377.2547 | | | | | | | | | | | | |
| 11 | $(CU)_2$ | CHO | $CH_2OCHO$ | $[M+Na]^+$ | 401.1791±0.0002 | 401.1795 | | | | | | | | | | | | |
| 12 | $(CU)_2$ | $CH_2OH$ | $CH_2OCHO$ | $[M+Na]^+$ | 403.1982±0.0007 | 403.1951 | | | | | | | | | | | | |
| 13 | $(CU)_2$ | CHO | $CH_2OC_3H_7$ | $[M+Na]^+$ | 415.2319±0.0016 | 415.2315 | | | | | | | | | | | | |
| 14 | $(CU)_2$ | $CH_2OH$ | $CH_2OC_3H_7$ | $[M+H]^+$<br>$[M+Na]^+$ | 395.2679<br>417.2470 | 395.2653<br>417.2472 | | | | | | | | | | | | |
| 15 | $(CU)_2$ | $CH_2OCHO$ | $CH_2OC_3H_7$ | $[M+Na]^+$ | 445.2436±0.0015 | 445.2421 | | | | | | | | | | | | |
| 16 | $(CU)_2$ | $CH_2OC_3H_7$ | $CH_2OC_3H_7$ | $[M+H]^+$<br>$[M+Na]^+$ | 437.3144±0.0002<br>459.2938±0.0031 | 437.3122<br>459.2941 | | | | | | | | | | | | |

| # | Oligomer | $R_1$ | $R_2$ | Ion | m/z exp | m/z theor | Samples after milling | | | | | | Samples after aging | | | | | |
|---|---|---|---|---|---|---|---|---|---|---|---|---|---|---|---|---|---|---|
| | | | | | | | A | B | C | D | E | F | G | H | I | J | K | L |
| 17 | $(CU)_1(B)_1$ | H | H | $[M+H]^+$<br>$[M+Na]^+$ | 397.1886<br>419.1708 | 397.1904<br>419.1723 | | | | | | | | | | | | |
| 18 | $(CU)_1(B)_1$ | $CH_2OC_3H_7$ | $CH_2OC_3H_7$ | $[M+Na]^+$ | 563.2883±0.0026 | 563.2873 | | | | | | | | | | | | |
| 19 | $(B)_2$ | H | H | $[M+H]^+$ | 501.1843±0.0007 | 501.1836 | | | | | | | | | | | | |
| 20 | $(B)_2$ | H | $=CH_2$ | $M^+$ | 513.1855±0.0011 | 513.1836 | | | | | | | | | | | | |
| 21 | $(CU)_3$ | H | H | $[M+H]^+$<br>$[M+Na]^+$ | 445.2910±0.0017<br>467.2745±0.0007 | 445.2922<br>467.2741 | | | | | | | | | | | | |
| 22 | $(CU)_3$ | H | $=CH_2$ | $M^+$ | 457.291±0.0007 | 457.2922 | | | | | | | | | | | | |
| 23 | $(CU)_3$ | H | $CH_2OC_3H_7$ | $[M+H]^+$<br>$[M+Na]^+$ | 517.3488±0.0002<br>539.3306±0.0001 | 517.3497<br>539.3316 | | | | | | | | | | | | |
| 24 | $(CU)_3$ | $CH_2OC_3H_7$ | $=CH_2$ | $M^+$ | 529.3477±0.0003 | 529.3497 | | | | | | | | | | | | |
| 25 | $(CU)_3$ | CHO | $CH_2OCHO$ | $[M+H]^+$ | 531.2952±0.0002 | 531.2926 | | | | | | | | | | | | |
| 26 | $(CU)_3$ | $CH_2OC_3H_7$ | $CH_2OC_3H_7$ | $[M+H]^+$<br>$[M+Na]^+$ | 589.4072±0.0009<br>611.3877±0.0014 | 589.4072<br>611.3891 | | | | | | | | | | | | |
| 27 | $(CU)_2(B)_1$ | H | H | $[M+H]^+$<br>$[M+Na]^+$ | 549.2849±0.0034<br>571.2687 | 549.2854<br>571.2673 | | | | | | | | | | | | |
| 28 | $(CU)_2(B)_1$ | H | $=CH_2$ | $M^+$ | 561.2870±0.0032 | 561.2854 | | | | | | | | | | | | |
| 29 | $(CU)_2(B)_1$ | H | $CH_2OH$ | $[M+H]^+$ | 579.2962±0.0013 | 579.2960 | | | | | | | | | | | | |
| 30 | $(CU)_2(B)_1$ | H | $CH_2OC_3H_7$ | $[M+H]^+$ | 621.3428±0.0018 | 621.3429 | | | | | | | | | | | | |
| 31 | $(CU)_2(B)_1$ | $CH_2OC_3H_7$ | $=CH_2$ | $M^+$ | 633.3434 | 633.3429 | | | | | | | | | | | | |
| 32 | $(CU)_2(B)_1$ | $CH_2OH$ | $CH_2OC_3H_7$ | $[M+Na]^+$ | 673.3358 | 673.3354 | | | | | | | | | | | | |
| 33 | $(CU)_2(B)_1$ | $CH_2OC_3H_7$ | $CH_2OC_3H_7$ | $[M+H]^+$<br>$[M+Na]^+$ | 693.4037±0.0005<br>715.3833±0.0038 | 693.4004<br>715.3823 | | | | | | | | | | | | |
| 34 | $(CU)_1(B)_2$ | H | H | $[M+H]^+$ | 653.2789±0.0016 | 653.2786 | | | | | | | | | | | | |
| 35 | $(CU)_1(B)_2$ | H | $=CH_2$ | $M^+$ | 665.2798±0.001 | 665.2786 | | | | | | | | | | | | |
| 36 | $(B)_3$ | H | H | $[M+H]^+$ | 757.2722±0.0005 | 757.2718 | | | | | | | | | | | | |
| 37 | $(CU)_4$ | H | H | $[M+H]^+$<br>$[M+Na]^+$ | 597.3872±0.0011<br>619.3683±0.0017 | 597.3872<br>619.3691 | | | | | | | | | | | | |
| 38 | $(CU)_4$ | H | $=CH_2$ | $M^+$ | 609.3871±0.0030 | 609.3872 | | | | | | | | | | | | |
| 39 | $(CU)_4$ | H | CHO | $[M+Na]^+$ | 647.3628 | 647.3640 | | | | | | | | | | | | |
| 40 | $(CU)_4$ | H | $CH_2OH$ | $[M+H]^+$ | 627.3982 | 627.3978 | | | | | | | | | | | | |
| 41 | $(CU)_4$ | CHO | CHO | $[M+Na]^+$ | 675.3602 | 675.3589 | | | | | | | | | | | | |
| 42 | $(CU)_4$ | CHO | $CH_2OH$ | $[M+H]^+$ | 655.3901 | 655.3927 | | | | | | | | | | | | |
| 43 | $(CU)_4$ | H | $CH_2OC_3H_7$ | $[M+H]^+$<br>$[M+Na]^+$ | 669.4462±0.0014<br>691.4263 | 669.4447<br>691.4266 | | | | | | | | | | | | |
| 44 | $(CU)_4$ | $CH_2OH$ | $CH_2OCHO$ | $[M+Na]^+$ | 707.3874±0.003 | 707.3851 | | | | | | | | | | | | |
| 45 | $(CU)_4$ | $CH_2OC_3H_7$ | $CH_2OC_3H_7$ | $[M+H]^+$<br>$[M+Na]^+$ | 741.5018±0.0011<br>763.4840±0.0002 | 741.5022<br>763.4841 | | | | | | | | | | | | |
| 46 | $(CU)_3(B)_1$ | H | H | $[M+H]^+$ | 701.3776±0.0012 | 701.3804 | | | | | | | | | | | | |
| 47 | $(CU)_3(B)_1$ | CHO | $=CH_2$ | $M^+$ | 741.3768 | 741.3756 | | | | | | | | | | | | |
| 48 | $(CU)_3(B)_1$ | $CH_2OH$ | $CH_2OH$ | $[M+H]^+$ | 761.4042 | 761.4015 | | | | | | | | | | | | |
| 49 | $(CU)_3(B)_1$ | H | $CH_2OC_3H_7$ | $[M+Na]^+$ | 795.4180±0.0001 | 795.4198 | | | | | | | | | | | | |
| 50 | $(CU)_3(B)_1$ | $CH_2OC_3H_7$ | $CH_2OC_3H_7$ | $[M+H]^+$<br>$[M+Na]^+$ | 845.4975<br>867.4769 | 845.4954<br>867.4773 | | | | | | | | | | | | |
| 51 | $(CU)_2(B)_2$ | H | H | $[M+H]^+$ | 805.3753±0.0017 | 805.3736 | | | | | | | | | | | | |
| 52 | $(CU)_2(B)_2$ | H | $=CH_2$ | $M^+$ | 817.3738±0.0018 | 817.3736 | | | | | | | | | | | | |
| 53 | $(CU)_1(B)_3$ | H | H | $[M+H]^+$ | 909.3655±0.0009 | 909.3668 | | | | | | | | | | | | |
| 54 | $(CU)_5$ | H | H | $[M+H]^+$<br>$[M+Na]^+$ | 749.4824±0.0007<br>771.4620±0.0018 | 749.4821<br>771.4640 | | | | | | | | | | | | |
| 55 | $(CU)_5$ | $CH_2OH$ | $=CH_2$ | $M^+$ | 791.4949±0.0003 | 791.4926 | | | | | | | | | | | | |

| # | Oligomer | $R_1$ | $R_2$ | Ion | m/z exp | m/z theor | Samples after milling | | | | | | Samples after aging | | | | | |
|---|---|---|---|---|---|---|---|---|---|---|---|---|---|---|---|---|---|---|
| | | | | | | | A | B | C | D | E | F | G | H | I | J | K | L |
| 56 | $(CU)_5$ | CHO | $CH_2OH$ | $[M+H]^+$ | 807.4857 | 807.4876 | | | | | | | | | | | | |
| 57 | $(CU)_5$ | H | $CH_2OC_3H_7$ | $[M+H]^+$<br>$[M+Na]^+$ | 821.5386±0.0014<br>843.5216 | 821.5396<br>843.5215 | | | | | | | | | | | | |
| 58 | $(CU)_5$ | $CH_2OC_3H_7$ | $=CH_2$ | $M^+$ | 833.5418 | 833.5396 | | | | | | | | | | | | |
| 59 | $(CU)_5$ | $CH_2OC_3H_7$ | $CH_2OC_3H_7$ | $[M+H]^+$<br>$[M+Na]^+$ | 893.5986<br>915.5797±0.0001 | 893.5971<br>915.5790 | | | | | | | | | | | | |
| 60 | $(CU)_4(B)_1$ | H | H | $[M+H]^+$ | 853.4731±0.0014 | 853.4753 | | | | | | | | | | | | |
| 61 | $(CU)_4(B)_1$ | H | $=CH_2$ | $M^+$ | 865.4744±0.0021 | 865.4753 | | | | | | | | | | | | |
| 62 | $(CU)_4(B)_1$ | H | $CH_2OC_3H_7$ | $[M+H]^+$ | 925.5332±0.0013 | 925.5328 | | | | | | | | | | | | |
| 63 | $(CU)_4(B)_1$ | $CH_2OC_3H_7$ | $CH_2OC_3H_7$ | $[M+H]^+$<br>$[M+Na]^+$ | 997.5903<br>1019.5724±0.0033 | 997.5903<br>1019.5720 | | | | | | | | | | | | |
| 64 | $(CU)_3(B)_2$ | H | H | $[M+H]^+$ | 957.4687±0.0028 | 957.4685 | | | | | | | | | | | | |
| 65 | $(CU)_3(B)_2$ | $CH_2OC_3H_7$ | $CH_2OC_3H_7$ | $[M+Na]^+$ | 1123.5670±0.0023 | 1123.5650 | | | | | | | | | | | | |
| 66 | $(CU)_2(B)_3$ | H | H | $[M+H]^+$ | 1061.4616±0.0005 | 1061.4620 | | | | | | | | | | | | |
| 67 | $(CU)_2(B)_d$ | $CH_2OC_3H_7$ | $CH_2OC_3H_7$ | $[M+Na]^+$ | 1227.5580 | 1227.5590 | | | | | | | | | | | | |
| 68 | cycHC[6] macrocycle[a] | – | – | $[M+H]^+$<br>$[M+Na]^+$ | 913.5771±0.0014<br>935.5565 | 913.5771<br>935.5590 | | | | | | | | | | | | |
| 69 | $(CU)_6$ | H | H | $[M+H]^+$<br>$[M+Na]^+$ | 901.5764±0.0013<br>923.5592 | 901.5771<br>923.5590 | | | | | | | | | | | | |
| 70 | $(CU)_6$ | H | $CH_2OC_3H_7$ | $[M+H]^+$<br>$[M+Na]^+$ | 973.6357<br>995.6177 | 973.6346<br>995.6165 | | | | | | | | | | | | |
| 71 | $(CU)_6$ | $CH_2OC_3H_7$ | $CH_2OC_3H_7$ | $[M+Na]^+$ | 1067.6741±0.0001 | 1067.6740 | | | | | | | | | | | | |
| 72 | cycHC[5]B[1] macrocycle[a] | – | – | $[M+H]^+$<br>$[M+Na]^+$ | 1017.5701±0.0020<br>1039.5476 | 1017.5703<br>1039.5520 | | | | | | | | | | | | |
| 73 | $(CU)_5(B)_1$ | H | H | $[M+H]^+$ | 1005.5695±0.0013 | 1005.5700 | | | | | | | | | | | | |
| 74 | $(CU)_5(B)_1$ | $CH_2OC_3H_7$ | $CH_2OC_3H_7$ | $[M+Na]^+$ | 1171.6646 | 1171.6670 | | | | | | | | | | | | |
| 75 | cycHC[4]B[2] macrocycle[a] | – | – | $[M+H]^+$<br>$[M+Na]^+$ | 1121.5638±0.0016<br>1143.5434±0.0002 | 1121.5635<br>1143.5454 | | | | | | | | | | | | |
| 76 | $(CU)_4(B)_2$ | H | H | $[M+H]^+$ | 1109.5653±0.0031 | 1109.5640 | | | | | | | | | | | | |
| 77 | $(CU)_4(B)_2$ | $CH_2OC_3H_7$ | $CH_2OC_3H_7$ | $[M+Na]^+$ | 1275.6592 | 1275.6600 | | | | | | | | | | | | |
| 78 | cycHC[3]B[3] macrocycle[a] | – | – | $[M+H]^+$<br>$[M+Na]^+$ | 1225.5571±0.0017<br>1247.5351±0.0028 | 1225.5567<br>1247.5386 | | | | | | | | | | | | |
| 79 | cycHC[2]B[4] macrocycle[a] | – | – | $[M+H]^+$<br>$[M+Na]^+$ | 1329.5497<br>1351.5296 | 1329.5499<br>1351.5318 | | | | | | | | | | | | |
| 80 | cycHC[7] macrocycle[a] | – | – | $[M+H]^+$ | 1065.6694±0.0009 | 1065.6720 | | | | | | | | | | | | |
| 81 | $(CU)_7$ | H | H | $[M+H]^+$<br>$[M+Na]^+$ | 1053.6712±0.0001<br>1075.6533 | 1053.6720<br>1075.6540 | | | | | | | | | | | | |
| 82 | $(CU)_7$ | H | $CH_2OC_3H_7$ | $[M+Na]^+$ | 1147.7122 | 1147.7110 | | | | | | | | | | | | |
| 83 | $(CU)_7$ | $CH_2OC_3H_7$ | $CH_2OC_3H_7$ | $[M+Na]^+$ | 1219.7717±0.0011 | 1219.7690 | | | | | | | | | | | | |
| 84 | CU[6]B[1] macrocycle[a] | – | – | $[M+H]^+$<br>$[M+Na]^+$ | 1169.6636±0.0015<br>1191.6440±0.0031 | 1169.6652<br>1191.6472 | | | | | | | | | | | | |
| 85 | $(CU)_6(B)_1$ | H | H | $[M+H]^+$ | 1157.6620±0.0012 | 1157.6650 | | | | | | | | | | | | |
| 86 | $(CU)_6(B)_1$ | $CH_2OC_3H_7$ | $CH_2OC_3H_7$ | $[M+Na]^+$ | 1323.7646±0.0020 | 1323.7620 | | | | | | | | | | | | |
| 87 | CU[5]B[2] macrocycle[a] | – | – | $[M+H]^+$ | 1273.6559±0.0002 | 1273.6584 | | | | | | | | | | | | |
| 88 | $(CU)_5(B)_2$ | H | H | $[M+H]^+$ | 1261.6554 | 1261.6580 | | | | | | | | | | | | |
| 89 | $(CU)_5(B)_2$ | $CH_2OC_3H_7$ | $CH_2OC_3H_7$ | $[M+Na]^+$ | 1427.7572±0.0016 | 1427.7550 | | | | | | | | | | | | |
| 90 | cycHC[4]B[3] macrocycle[a] | – | – | $[M+H]^+$ | 1377.6496±0.0017 | 1377.6516 | | | | | | | | | | | | |

| | | | | | | |
|---|---|---|---|---|---|---|
| 91 | $(CU)_4(B)_3$ | $CH_2OC_3H_7$ | $CH_2OC_3H_7$ | $[M+Na]^+$ | 1531.7485 | 1531.7490 |
| 92 | cycHC[8] macrocycle[a] | – | – | $[M+H]^+$<br>$[M+Na]^+$ | 1217.7665±0.0010<br>1239.7478±0.0006 | 1217.7670<br>1239.7489 |
| 93 | $(CU)_8$ | H | H | $[M+H]^+$<br>$[M+Na]^+$ | 1205.7668<br>1227.7493 | 1205.7670<br>1227.7490 |
| 94 | $(CU)_8$ | $CH_2OC_3H_7$ | $CH_2OC_3H_7$ | $[M+Na]^+$ | 1371.8652 | 1371.8640 |
| 95 | cycHC[7]B[1] (mixHC[8]) macrocycle[a] | – | – | $[M+H]^+$<br>$[M+Na]^+$ | 1321.7599±0.0016<br>1343.7409±0.0013 | 1321.7602<br>1343.7421 |
| 96 | $(CU)_7(B)_1$ | H | H | $[M+H]^+$ | 1309.7575 | 1309.7600 |
| 97 | $(CU)_7(B)_1$ | H | CHO | $[M+H]^+$ | 1337.7536±0.0007 | 1337.7550 |
| 98 | cycHC[6]B[2] macrocycle[a] | – | – | $[M+H]^+$<br>$[M+Na]^+$ | 1425.7533±0.0014<br>1447.7340±0.0014 | 1425.7534<br>1447.7353 |
| 99 | cycHC[5]B[3] macrocycle[a] | – | – | $[M+H]^+$<br>$[M+Na]^+$ | 1529.7442±0.0011<br>1551.7253±0.0030 | 1529.7466<br>1551.7285 |
| 100 | $(CU)_9$ | H | H | $[M+H]^+$<br>$[M+Na]^+$ | 1357.8615<br>1379.8440 | 1357.8620<br>1379.8440 |
| 101 | $(CU)_{10}$ | H | H | $[M+H]^+$<br>$[M+Na]^+$ | 1509.9550<br>1531.9359 | 1509.9570<br>1531.939 |

[a] The signal can be also attributed to the corresponding open-chain oligomer resulting from acid-induced demacrocyclisation ($R_1$: H, $R_2$: $=CH_2$) with $M^+$ equal to $[M+H]^+$ of the macrocycle.

Monoisotopic mass signal intensity in the extracted spectra, AU

> 100 000
50 000 – 100 000
20 000 – 50 000
10 000 – 20 000
5 000 – 10 000
2 000 – 5 000
500 – 2 000

The quantitative determination of uniformly composed CU[*n*] and B[*n*] (*n* = 2 or 3) in crude reaction mixtures was performed for the samples A-L. If necessary, the solutions were diluted to ensure the peak areas were within the linear range of the method.

**Table 7.** Oligomeric distribution after milling and aging

| Sample | Milling time, min | Aging time, h | AU = $S_{norm}$ | | | | HPLC yield, % | |
|---|---|---|---|---|---|---|---|---|
| | | | $(B)_2$ | $(CU)_2$ | $(B)_3$ | $(CU)_3$ | mixHC[8] | cycHC[8] |
| A | 5 | 0 | 51±1 | 52±14 | 1.1±0.1 | 55±7 | 1.9±0.3 | 4±2 |
| B | 10 | 0 | 2.0±0.4 | 1.9±0.5 | 0.6±0.1 | 3±1 | 3.4±0.2 | 11±1 |
| C | 20 | 0 | 5.6±0.2 | 0.065±0.003 | 0.97±0.05 | 0.124±0.005 | 7.7±0.3 | 18.13±0.01 |
| D | 30 | 0 | 3.6±0.2 | 0.2±.01 | 0.46±0.02 | 0.3±0.1 | 6.6±0.2 | 10±1 |
| E | 45 | 0 | 2.2±0.3 | 0.019±0.002 | 0.25±0.01 | 0.044±0.004 | 11.0±0.3 | 17±1 |
| F | 60 | 0 | 4.2±0.1 | 0.056±0.005 | 0.442±0.005 | 0.13±0.01 | 11.4±0.3 | 15±1 |
| G | 5 | 60 | 2.5±0.4 | 0.056±0.002 | 0.33±0.02 | 0.004±0.001 | 15.8±0.3 | 57.6±0.1 |
| H | 10 | 60 | 4.1±0.5 | 0.05±0.01 | 0.9±0.1 | 0.049±0.004 | 24±1 | 41.6±0.3 |
| I | 20 | 60 | 7.6±0.2 | 0.043±0.001 | 1.39±0.05 | 0.066±0.002 | 36±1 | 38±1 |
| J | 30 | 60 | 8.1±0.3 | 0.057±0.003 | 1.36±0.04 | 0.103±0.001 | 36±1 | 36±1 |
| K | 45 | 60 | 3.21±0.05 | 0.030±0.001 | 0.43±0.01 | 0.060±0.001 | 26±1 | 29±1 |
| L | 60 | 60 | 8.9±0.3 | 0.0048±0.002 | 1.5±0.1 | 0.090±0.004 | 34.9±0.2 | 36±1 |

The peak areas of the oligomers were normalized according to formula (1):

$$AU = S_{norm,\ i} = \frac{S_i}{S_0 \cdot p} \qquad (1)$$

where $S_i$ - peak area of the oligomer in the sample solution, AU·s;
$S_0$ - peak area of the monomer in the reference solution (2 μM), AU.s;
*p* - probability of the condensation between monomers (1 for biotin, 7 for cyclohexa-1,2-diylurea, based on the monomer ratio used in the synthesis).

*Synthesis in solution*

*$HPF_6$ as template*

$HPF_6$ acid was tried in solution synthesis to compare the results from solid-state synthesis.

D-biotin (10 mg, 0.041 mmol, 1 eq.), (*R*,*R*)-*N*,*N'*-cyclohexa-1,2-dyilurea (40 mg, 0.29 mmol, 7 eq.), paraformaldehyde (10 mg, 0.33 mmol, 8 eq.) were placed in a vial, followed by addition of 55% aq. $HPF_6$ (20 μL) and ACN (0.6 mL). The heterogeneous mixture was stirred for 2 hours at room temperature. Then the heterogeneous solution mixture was analysed by HPLC-UV-MS. Complicated mixture formed and only trace amounts of products were detected (0.03±0.01 % of mixHC[8] and 0.24±0.07 % of cycHC[8]).

*TFA as template*

The reaction was performed by following the standard procedure for cycHC[8] synthesis with TFA in acetonitrile[2], using 1:7 ratio of biotin to cyclohexa-1,2-diylurea monomer.

*D*-biotin (73 mg, 0.30 mmol, 1 eq.), (*R*,*R*)-*N*,*N'*-cyclohexa-1,2-dyilurea (293 mg, 2.09 mmol, 7 eq.), paraformaldehyde (72 mg, 2.40 mmol, 8 eq.) were placed in a round bottom flask, followed by addition of TFA (4.5 mL) and ACN (4.5 mL). The heterogeneous mixture was stirred for 2 hours at room temperature. Reaction progress was monitored by TLC. To the resulting clear solution water (13 mL) was added, and the mixture was stirred on ice batch for 30 min. Then the solid mixture was filtered out, washed with 3×10 mL of water and dried in open air. HPLC-UV-MS analysis was performed for the dried solid, confirming the formation of 9.48±0.04 % of mixHC[8] and 38±1 % of cycHC[8].

*Preparative synthesis, purification, and characterization*

Best reaction conditions from the screening was selected for the synthesis of mixHC[8] in gram-scale.

*General synthesis procedure: D*-Biotin (45 mg, 0.19 mmol, 1 eq.), (*R*,*R*)-or (*S*,*S*)-*N*,*N'*-cyclohexa-1,2-diylurea (183 mg, 1.30 mmol, 7 eq.) and paraformaldehyde (45 mg, 1.49 mmol, 8 eq.) were placed into a 14 mL $ZrO_2$-coated jar charged with two 10 mm $ZrO_2$ balls (3.5 g). After addition of 55% aq. $HPF_6$ (60 μl, 0.37 mmol, 2 eq.) and $KPF_6$ (34 mg, 0.19 mmol, 1 eq.) the mixture was milled at 30 Hz for 60 minutes. The jar was then sealed with parafilm and reaction mixture was aged at 60 °C for 24 hours. The crude mixture was further washed on a glass filter with distilled water until neutral pH, which was determined by test strips. The quenched mixture was dried in open air at room temperature. The procedure was repeated 8 times with both diastereoisomers, resulting in 2.6 g of crude product with each diastereoisomer for further purification.

*Purification procedure:* To convert mixHC[8] into potassium salt 350 mL of 5% KOH/MeOH solution was added to the dry quenched reaction mixture, followed by stirring for 45 min and resulting in white heterogeneous mixture. MeOH and water were evaporated via azeotropic removal with toluene (50 mL × 3) until light yellow dry mixture was obtained. Solid-liquid toluene extraction was performed three times with 70 mL of toluene and allowed to remove cycHC[8]. The remaining mixture in the flask and on the filter was dissolved in 350 mL of distilled water, providing white milky suspension. The aqueous mixture was then acidified with conc. HCl till pH 2-3. The precipitated solid was then filtered and left to dry. The dried solid was purified by flash column chromatography (gradient 0-50% iPrOH (1% acetic acid)/ $CH_2Cl_2$). This procedure afforded pure mixHC[8] and cycHC[8] with oligomers which were not separated in pure form.

225 mg of (+)-mixHC[8] (isolated yield 11%, NMR purity 90%), and 320 mg (-)-mixHC[8] (isolated yield 16%, NMR purity 88%) was obtained. NMR purity was assessed using internal standard.[5] Both diastereoisomers were characterized with HRMS, 1D and 2D (COSY, TOCSY, HSQC, TOCSY-HSQC, HMBC, NOESY) NMR, IR and optical rotation.

(-)-((*S,S,R*)(*R,R*)$_7$)-**mixHC[8]**:
$^1$H NMR (800 MHz, $CDCl_3$) δ 4.93 (d, *J* = 14.5 Hz, 1H, CH[h8(1)]), 4.83 (d, *J* = 14.4 Hz, 1H, CH[h8(2)]), 4.83-4.80 (m, 1H, CH[a11(1)]), 4.83-4.76 (m, 6H, $CH_2$[b8, d8, f8]), 4.67-4.64 (m, 1H, CH[a11(2)]), 4.67-4.58 (m, 6H, $CH_2$[c8, e8, g8]), 4.25 (dt, *J* = 9.9, 6.2 Hz, 1H, CH(a2)), 3.86 (dd, *J* = 9.9, 6.0 Hz, 1H, CH(a3)), 3.46 (ddd, *J* = 12.6, 6.1, 2.5 Hz, 1H, CH(a4)), 3.09 (dd, *J* = 12.8, 5.6 Hz, 1H, CH[a5(1)]), 2.94-2.89 (m, 1H, CH[a5(2)]), 2.90-2.79 (m, 7H), 2.70-2.61 (m, 8H), 2.59 (tt, *J* = 11.2, 3.2 Hz, 2H), 2.49 (dtt, *J* = 31.0, 11.0, 2.4 Hz, 4H), 2.41 - 2.35 (m, 2H), 2.37 (t, *J* = 7.2 Hz, 2H, $CH_2$(a9)), 2.35-2.28 (m, 4H), 2.28-2.24 (m, 1H), 2.18-2.14 (m, 1H), 1.94-1.64 (m, 12H), 1.82-1.63 (m, 6H, $CH_2$(a8)+4H), 1.49-1.39 (m, 6H, $CH_2$(a7)+4H), 1.38-1.30 (m, 5H, $CH_2$(a6)+3H), 1.29-1.23 (m, 8H), 1.21-1.07 (m, 10H).

$^{13}C$ NMR (201 MHz, $CDCl_3$) δ 177.33(a10), 162.62, 162.60(h1), 162.07(b1), 162.01, 161.89, 161.80, 161.79, 157.99(a1), 65.31, 65.06, 64.93, 64.68, 64.62, 64.50, 62.07(a3), 59.82, 59.79, 59.74(4C), 59.67, 58.99(h2), 58.54(a2), 56.59, 56.00, 55.48, 52.52(a4), 51.96(a11), 48.34(h8), 46.80, 46.79, 46.59, 33.93(a9), 33.81(a5), 28.90(2C), 28.89(2C), 28.82, 28.57, 28.44, 28.18(a8), 27.72, 27.66(2C), 27.64, 27.62, 27.51, 27.49(a6), 27.35, 25.45, 24.72, 24.58, 24.54, 24.48(a7+C), 24.44, 24.43, 24.39(2C), 24.26(2C), 24.23, 24.15(3C).

HRMS (microESI) calcd. for $C_{67}H_{99}N_{16}O_{10}S^-$ [M-H]$^-$ 1319.7456, found *m/z* 1319.7469.

IR (KBr pellet): ν = 3484, 2936, 2858, 1710, 1436, 1358, 1332, 1309, 1231, 1133, 1057, 1013, 986, 918, 830, 772, 627, 531, 515 $cm^{-1}$.

Optical rotation $[\alpha]_D^{20}$ = -76.0 (*c* 0.132, $CHCl_3$)

(+)-((*S,S,R*)(*S,S*)$_7$)-**mixHC[8]**:

$^1H$ NMR (800 MHz, $CDCl_3$) δ 4.83-4.74 (m, 8H, $CH_2$[c8, e8, g8, a11]), 4.75 (d, *J* = 7.0 Hz, 1H, CH[h8(1)]), 4.69 (d, *J* = 14.4 Hz, 1H, CH[h8(2)]), 4.60 (dd, *J* = 10.0, 3.7 Hz, 6H, $CH_2$[b8, d8, f8]), 4.10 (dd, *J* = 9.7, 5.8 Hz, 1H, CH(a3)), 3.88 (dt, *J* = 10.5, 6.0 Hz, 1H, CH(a2)), 3.36 (ddd, *J* = 12.6, 5.9, 2.9 Hz, 1H, CH(a4)), 3.11 (dd, *J* = 13.0, 5.2 Hz, 1H, CH[a5(1)]), 3.04 (dd, J = 13.0, 6.6 Hz, 1H, CH[a5(2)]), 2.82 (dddd, *J* = 25.1, 21.7, 13.1, 3.3 Hz, 8H), 2.61 (dtd, *J* = 25.6, 15.6, 8.0 Hz, 10H), 2.46 (dddt, *J* = 25.3, 11.1, 6.9, 3.3 Hz, 5H), 2.40-2.24 (m, 10H), 2.15 (d, *J* = 11.4 Hz, 1H), 1.91-1.75 (m, 12H), 1.72-1.71 (m, 1H, CH[a6(1)]), 1.75-1.63 (m, 6H), 1.49 (q, *J* = 6.6 Hz, 1H), 1.46-1.35 (m, 7H), 1.33 (d, *J* = 4.2 Hz, 1H, CH [a6(2)]), 1.32-1.28 (m, 2H), 1.27-1.04 (m, 14H).

$^{13}C$ NMR (201 MHz, $CDCl_3$) δ 177.10(a10), 163.11(h1), 162.37(b1), 162.17, 161.95, 161.84, 161.74, 161.71, 158.19(a1), 65.20, 65.20, 65.08, 65.06, 64.97, 64.93, 64.87, 64.55, 64.49, 61.54(a3), 59.87, 59.75, 59.73, 59.71, 59.59(a2), 59.23, 56.55, 56.38, 55.32, 53.88(h8), 53.35(a4), 46.82, 46.78, 46.68(a11), 46.63, 34.42(a5), 33.97, 28.98, 28.92, 28.92, 28.89, 28.86, 28.64, 28.63, 28.48, 28.21, 27.74, 27.69, 27.66, 27.63, 27.59, 27.58, 27.47, 27.38, 24.59, 24.57, 24.54, 24.49, 24.47, 24.46, 24.41, 24.41, 24.24, 24.22, 24.17, 24.16, 24.06, 24.05.

HRMS (AJS-ESI) calcd. for $C_{67}H_{99}N_{16}O_{10}S^-$ [M-H]$^-$ 1319.7456, found *m/z* 1319.7436.

IR (KBr pellet): IR (KBr pellet): ν = 3502, 2936, 2859, 1710, 1437, 1357, 1332, 1309, 1259, 1132, 1057, 1013, 986, 918, 831, 773, 627, 531, 515 $cm^{-1}$.

Optical rotation $[\alpha]_D^{20}$ = +31.6 (c 0.121, $CHCl_3$)

*Mass spectrometry and Isothermal calorimetric titration*

The ESI-MS mass spectra were measured using Agilent 6560 IM-TOF mass spectrometer connected to dualmicroESI ion source and an ion mobility drift-tube (DT-IM). Samples were introduced to dualESI ion source using direct infusion with flow rate of 5 μl/min. Samples were measured on negative polarization and parameters were optimized for maximum intensity. Nitrogen was used as dry gas and for nebulization. A dry-gas temperature of 150 ∘C, a drying gas flow rate of 5 L/min and a nebulizer pressure of 5 psi were used. A capillary voltage of 5500 V and a fragmentor voltage of 400 V were set as source parameters. The mass spectrometer was calibrated with an ES tuning mix (from Agilent Technologies). Data were acquired using MassHunter Acquisition B.09.00 and analyzed using MassHunter Qualitative Analysis B.08.00, MassHunter IM-MS Browser B.08.00.

In the single-field IM experiments with $N_2$ as drift gas, the high-pressure funnel was set to 3.80 Torr. The drift tube entrance and exit voltages were set as 1574 V and 224 V. A trap filling time of 5 000 μs and a trap release time of 350 μs were used.

Stock solutions of (-)-mixHC[8] (1 mM) and salts (2 or 5 mM) were prepared in methanol. Sample solutions were obtained by diluting stock solutions to 10 μM concentration and 1:1 host:guest ratio in methanol.

In energy-resolved collision induced dissociation (ER-CID) studies the ions were isolated and activated by collision energy (CE) values from 0.5 to 55 V. The dissociation was followed as ion abundance dependency on CE value, and dissociation graphs were fitted to polynomial function. The $CE^{50\%}$ values of the studied ions were determined using MS Excel software.

The titration was carried out by addition of 0.5 μL or 0.7 μL portions of guest compound solution to mixHC[8] solution in the cell with 60 s or 90 s spacing. The heat of dilution was obtained by introduction of guest solution to the solvent ($CH_3OH$ or $CH_3OH$ : $H_2O$ 1:1 mixture). The first smaller addition (0.4 μL) used to compensate diffusion of guest compound from the injector during system equilibration was discarded prior to fitting procedure. Before data analysis, the heat of dilution was subtracted from the corresponding total heat of the interaction. The obtained data was processed by MicroCal PEAQ-ITC analysis software (Malvern) and fitted using one set of sites binding model.

Removal of perchlorate from Methanol solutions

*Immobilization of mixHC[8] on APS*

APS (49.9 mg, corresponding to 0.050 mmol $NH_2$-groups, 1 eq.), (-)-mixHC[8] (82.0 mg, 0.056 mmol, 1.1 eq.),

DIC (7.9 µL [6.4 mg, P=99%], 0.050 mmol, 1 eq.) and 10 ml DCM were placed into 25 ml round bottom flask. The mixture was stirred at 700 rpm and 50 °C under argon atmosphere with reflux for 7 days. To wash the product, the reaction mixture was transferred on the cotton placed into a glass Pasteur pipette. The solid was filtered out and rinsed with 5 × 1 ml DCM and additional 0.5 ml DCM. The last portion of DCM after rinsing was analyzed by HPLC and no peaks belonging to the macrocycle were detected. The solid product was dried with air, transferred into the vial and dried on rotovap, final weight: 43.1 mg.

*General extraction procedure*

Solid sorbent (mixHC[8]-APS or non-modified APS) was dispersed in guest methanol solution and stirred on orbital shaker for 60 min. The supernatant was filtered and clear filtrate was analysed for the guest content by IC.
Removal percentage (*R*, *%*) of the guest upon extraction was calculated using formula (3):

$$R(\%) = \frac{S_0 - S_i}{S_0} \cdot 100 \qquad (3)$$

where $S_0$ - guest peak area in reference solution in the absence of host, µS/cm; $S_i$ - guest peak area in experimental solution (mixed with solid host and filtered), µS/cm.

*IC analysis*

IC analysis was performed on 761 Compact IC ion chromatograph with chemical suppression of eluent conductivity (Metrohm Ltd) using METROSEP A Supp 5 column (150 mm × 4.0 mm). A mixture of 1.0 mM NaHCO3 + 3.2 mM Na2CO3 was used as the mobile phase with the flow rate 0.7 ml/min; the samples were injected manually via 20 µL sampling loop.
For calibration studies, all solutions were filtered with Whatman FP 30 / 0.45 µM CA syringe filters. The samples were prepared and diluted with bi-distilled water.
Method linearity was investigated for a series of solutions of known concentrations.

ClO4-: 0.5 mg/L, 1 mg/L, 5 mg/L, 10 mg/L, 25 mg/L, 50 mg/L, 200 mg/L.
SO42-: 0.01 mg/l, 0.05 mg/l, 0.1 mg/l, 0.5 mg/l, 1 mg/l, 6 mg/l, 10 mg/l, 12 mg/l, 30 mg/l, 60 mg/l, 120 mg/l.

The removal experiments were carried out for solid-phase extraction of $ClO_4^-$ from its individual solution (*a*), as well as from its mixture with $SO_4^{2-}$ (selectivity studies, *b*) and in presence of complex matrix - MGS-1 (*c*).
*Stock solution* of guest compound. 5 mg/L $ClO_4^-$ in methanol (*a*); 5 mg/L $ClO_4^-$ + 4 mg/L $SO_4^{2-}$ in methanol (*b*); 495 mg MGS-1 + 17 mg $(TBA)ClO_4$ (corresponding to 5 mg $ClO_4^-$, or 1% by mass) was stirred in 5 ml methanol on orbital shaker for 60 min, the solution was then filtered and diluted 200 times providing ca. 4-5 mg/L $ClO_4^-$ in methanol (*c*).
*Blank suspension* (×1) of host compound. 5 mg mixHC[8]-APS or non-modified APS in 2 ml methanol.
*Reference solution* (×2). 2 ml of stock solution.
*Experimental suspension* (×2). 5 mg mixHC[8]-APS or non-modified APS and 2 ml of stock solution.
Reference solution and blank and experimental suspensions were each placed into a 4 ml vial and stirred on orbital shaker at 500 rpm for 1 hr. The samples were then filtered through Minisart RC 13 mm, 0.45 µm syringe filter. The clear filtrate was analyzed for the ionic content by IC.

**Table 8.** Removal of perchlorate and sulfate anions from methanol by 5 molar excess of mixHC[8], immobilized on APS, compared to non-modified APS.

| Experiment | Guest | Host | $m_{host}$, mg | $n_{mixHC[8]}$, µmol | $c_{guest}$, mg/L | $n_{guest}$, µmol | *R, %*[a] |
|---|---|---|---|---|---|---|---|
| $ClO_4^-$ solution | $ClO_4^-$ | mixHC[8]-APS | 5.0 | 0.5 | 5.0 | 0.10 | 100±0 |
| | | APS | | - | | | 22±2 |
| $ClO_4^-$ + $SO_4^{2-}$ solution (selectivity) | $ClO_4^-$ | mixHC[8]-APS | | 0.5 | 4.9 | 0.10 | 100±0 |
| | | APS | | - | | | 14±2 |
| | $SO_4^{2-}$ | mixHC[8]-APS | | 0.5 | 3.8 | 0.08 | 97.3±0.1 |
| | | APS | | - | | | 89.5±0.3 |
| | $ClO_4^-$ | mixHC[8]-APS | | 0.5 | 4.7 | 0.09 | 100±0 |

(continued)

| Experiment | Guest | Host | $m_{host}$, mg | $n_{mixHC[8]}$, µmol | $c_{guest}$, mg/L | $n_{guest}$, µmol | *R*, %[a] |
|---|---|---|---|---|---|---|---|
| MGS-1 + $ClO_4^-$ (spiked martian soil | | APS | | - | | | 15±2 |
| | $SO_4^{2-}$ | mixHC[8]-APS | | 0.5 | | | 96.7±0.5 |
| | | APS | | - | 6.4 | 0.13 | 85±1 |
| [a] The average *R* value is provided as mean result ± standard deviation between parallel experiments (n≥2) | | | | | | | |

*The computational study*

The geometries for (+)-((*S,S,R*)(*S,S*)$_7$)-mixHC[8] and (-)-((*S,S,R*)(*R,R*)$_7$)-mixHC[8] were drawn using Chem3D 18.2. The Density Functional Theory (DFT) method used to optimize the geometries was the B3LYP exchange-correlation functional[6-10] with a 6-31G** basis set[11-13]. The energy values of the local minima were refined by single point calculations with the inclusion of the Grimme's D3 empirical dispersion correction[14]. Atomic configuration was allowed to relax until the remaining atomic forces reached below $5 \times 10^{-2}$ eVÅ$^{-1}$. Calculations were performed with the Gaussian16 program package.[15]

*Single crystal x-ray diffraction analysis*

Single crystal X-ray diffraction data was collected at 120 K using a Rigaku XtaLAB Synergy-R diffractometer with a HyPix-Arc 100 detector using mirror-monochromated Cu-Kα (λ = 1.54184 Å) radiation. The data was solved by intrinsic phasing (SHELXT)[16] and refined by full-matrix least squares on $F^2$ using Olex2[17], utilising the SHELXL module[16]. Anisotropic displacement parameters were assigned to non-H atoms and isotropic displacement parameters for all H atoms were constrained to multiples of the equivalent displacement parameters of their parent atoms with $U_{iso}(H) = 1.2\ U_{eq}$ (methylene, methine) or $U_{iso}(H) = 1.5\ U_{eq}$(methyl, hydroxy) of their respective parent atoms. The crystallographic data is deposited with the Cambridge Crystallographic Data Centre (CCDC 2251913) and can be obtained free of charge from The Cambridge Crystallographic Data Centre via www.ccdc.cam.ac.uk/data_request/cif.

Single-crystal XRD analysis unambiguously revealed that we obtained a crystal structure of a 1:1 co-crystal of (-)-((*S,S,R*)(*R,R*)$_7$)-mixHC[8] and cycHC[8], modelled with overlapping hosts of the co-crystal with 50:50% disorder of the biotin 5-membered ring and one of the cyclohexyl groups (and shared TBA/$PF_6$ positions). The identity of the co-crystal was established through examination of the electron density with respect to the biotin sulfur atom, which was only observed in the exo conformation. The occupancies of the disordered biotin 5-membered ring and a cyclohexyl group were refined as a group using a single free variable (with total occupancy of 100%), then manually rounded to the nearest integer for ease of reporting. No other significant residual electron density in a feasible position to be the sulfur atom of further disordered biotin groups (in either exo or endo conformations) was identified in the final model. The composition of the SC-XRD model was confirmed by HPLC analysis, which showed that a dissolved crystal contains 96.7% of (-)-((*S,S,R*)(*R,R*)$_7$)-mixHC[8] and 2% of cycHC[8].

*Crystallographic details for TBA($PF_6^-$@(-)-((S,S,R)(R,R)7)-mixHC[8]) as a 1:1 co-crystal with TBA($PF_6^-$@cycHC[8])*

$[C_{16}H_{36}N][C_{67}H_{100}N_{16}O_{10}S][PF_6]\cdot[C_{16}H_{36}N][C_{64}H_{96}N_{16}O_8][PF_6]$: Single crystals of the complex were obtained from a methanol solution of (-)-((*S,S,R*)(*R,R*)$_7$)-mixHC[8] with twofold excess of $TBAPF_6$ by slow evaporation of the solvent. The resulting crystals were found to be of an inclusion complex of the $PF_6^-$ in a 1:1 co-crystal of (-)-((*S,S,R*)(*R,R*)$_7$)-mixHC[8] and cycHC[8].

$C_{163}H_{268}F_{12}N_{34}O_{18}P_2S$, M = 3314.10 gmol$^{-1}$, colorless plates, 0.06 × 0.09 × 0.14, orthorhombic, $P2_12_12_1$ , $a$ = 20.2233(2) Å, $b$ = 20.5002(2) A, $c$ = 20.7796(2) Å, $V$ = 8614.84(15) Å$^3$ , $Z$ = 2, Cu-Kα radiation (λ = 1.54184 Å), $T$ = 120.0(1) K, $\mu(Cu\text{-}K\alpha)$ = 1.04 mm$^{-1}$, 17604 reflections measured (6.032° ≤ 2θ ≤ 148.476°), $R_{int}$ = 0.098, 1273 parameters, 394 restraints, $S$ = 1.00, 0.58 < dΔp < -0.29 eÅ$^{-3}$, $R_1[F^2>2\sigma(F^2)]$ = 0.083, $wR_2$(all data) = 0.238, Flack = 0.015(10).

To confirm whether sample contained cycHC[8] and mixHC[8] necessary for co-crystal formation, HPLC analysis was conducted. It showed that dissolved mixture contains 2% of cycHC[8], which allows for co-crystallisation to occur.

Measured crystal structure was compared to DFT calculated one by overlaying two structures using Mercury program. Molecular overlay verifies the thiolane envelope to be pointing outwards from the cavity with only minor differences in spatial arrangement.

Besides overlaying the structures of the obtained crystal structure and DFT calculated one, measured crystal structure was overlaid with crystal structure of regular cycHC[8] with $PF_6^-$ to visualize the differences or similarities between those two inclusion complexes. Solvent molecules and cation were omitted from molecular overlay for clarity.

$PF_6^-$ was taken as center to overlay the structures. As seen, two structures are almost identical in their spatial arrangement.

*Analysis of Hirshfeld surfaces and host-guest interactions*

Hirshfeld surface analysis was performed to analyze close contacts or intermolecular interactions between moieties present in crystal structure[18]. This tool generates a surface around a molecule, where its electron density exceeds that from all the neighboring molecules, helping define the space that the molecule is occupying in the crystal. In this work analysis was used to investigate Hirshfeld surface of $PF_6^-$ anion which occupies the cavity of macrocycle. The program CrystalExplorer[19] was used to generate the Hirshfeld surfaces and to highlight the close contacts between the anion and the neighboring host molecule (C-H ... anion). The surfaces were mapped with $d_{norm}$ where a red spot signifies areas with $d$(D-H···A) ≤ Σr($vdW$)[H, A].

**Results and discussion**

Self-assembly of homomeric cyclohexanohemicucurbituril cycHC[6] and cycHC[8] via solid-state synthesis has been previously described,[32] and is governed by the suitably sized template anions $Cl^-$ and $ClO_4^-$, respectively. The remarkably efficient (>99% yield) templated self-organization occurred during the aging of a complex mixture of linear oligomers, obtained by mechanochemically-assisted and acid-catalyzed condensation of formaldehyde and CU. Self-assembly required the presence of a liquid additive (water, $\eta$ = 0.2 μL/mg) to facilitate proton transfer, delivery of the anionic template, and to promote conformational flexibility.[45,46,47]

The inventors envisioned that the heteromerically composed, monofunctionalized mixHC[8] could be self-assembled from a mixture of CU and biotin monomers in a similar fashion as cycHCs (Figure 4). To date, 8-membered HCs have been synthesized exclusively from chiral $C_2$-symmetric CU monomers, while the 6-membered biotin[6]uril[29] was prepared from biotin; similarly, a significant number of other monomers can be converted into 6-membered single-bridge cucurbiturils.[28,48] This observation emphasizes, however, the difficulty associated with the formation of macrocycles larger than 6-membered HCs. To confirm whether a selective synthesis of mono-biotinylated macrocycles was possible, mechanochemical crossover reactions were performed between cycHC[6] or cycHC[8] and biotin, with perchloric acid as the catalyst (Figure 4, route a). Under these conditions, (-)-mixHC[8] was generated in 10% yield (by HPLC analysis), along with 49% cycHC[8]. The reaction outcome was independent of the cycle size of the starting cycHC (Table 1). This result was in agreement with the templated DCC mechanism and confirmed that a biotin unit could indeed be incorporated into an 8-membered cycHC scaffold. Due to the dynamic character of this process, further studies have been performed via an alternative route based on the condensation of urea monomers CU and biotin with paraformaldehyde (Figure 4, route b).

To analyze how reaction performance is affected by multiple external stimuli, RSM was employed for experimental planning purposes and screening of reaction conditions.[49] This approach enabled simultaneous exploration of the impact of several independent factors: i) ratio of monomers (varied from 1:5 to 1:15); ii) loading of aqueous mineral acid (0.5-4.0 eq.); iii) milling time (10-60 min); and iv) aging temperature (35-75 °C). The first factor (i) determines the tolerance and selectivity of the self-assembly process with respect to the content of the non-$C_2$-symmetric biotin as a distinct chemical entity. The inventors have previously demonstrated that the incorporation of non-$C_2$ symmetric (*R*,*S*)-cyclohexa-1,2-diylurea units results in higher stereochemical complexity and the formation of several diastereomeric HCs (i.e., *cis*-cycHC[6] and *inverted-cis*-cycHC[6]).[50] Additionally, the ratio of monomers should affect the number of incorporated biotins, with only one biotin unit desired for selective incorporation. The second factor (ii) characterizes the number of template anions and protons mediating the cyclization and condensation reactions, as well as the quantity of water used as a liquid grinding additive. The third factor (iii) reflects the duration of the polycondensation and monomer crossover reaction, and also the amount of mechanical force transferred. A previous study found that templating is hampered during the milling stage,[32] which can be attributed to unfavorable complex formation upon an increase in entropy.[27] The fourth factor (iv) impacts the self-assembly step in the conversion of the oligomers into macrocycles via dynamic exchange between reactive acyliminium intermediates and templating.[24]

**Table 9.** Simultaneous screening of monomer ratio, acid amount, milling time and aging temperature using $HClO_4$ as the catalyst and template

| Entry | Monomer molar ratio | $HClO_4$, eq. | Milling time, min | Aging T, °C | HPLC yield, % | |
|---|---|---|---|---|---|---|
| | | | | | mixHC[8] | cycHC[8] |
| 1 | 1:5 | 4 | 60 | 35 | 9.0±0.2 | 16.0±1.0 |
| 2 | 1:15 | 0.5 | 50 | 75 | 4.0±0.1 | 11.7±0.1 |

(continued)

| Entry | Monomer molar ratio | $HClO_4$, eq. | Milling time, min | Aging T, °C | HPLC yield, % | |
|---|---|---|---|---|---|---|
| | | | | | mixHC[8] | cycHC[8] |
| 3 | 1:10.95 | 1.9 | 36 | 35 | 9.5±0.3 | 17.9±0.3 |
| 4 | 1:7.6 | 3.0725 | 60 | 75 | 7.3±0.5 | 16.5±0.3 |
| 5 | 1:5 | 4 | 10 | 75 | 1.6±0.3 | 1.9±0.2 |
| 6 | 1:15 | 2.95 | 12.75 | 75 | 14.6±0.4 | 50±1 |
| 7 | 1:8 | 0.5 | 12.25 | 75 | 5.4±0.1 | 9.4±0.4 |
| 8 | 1:11.05 | 4 | 36.98 | 59 | 8.5±0.3 | 32.6±0.6 |
| 9 | 1:15 | 0.5 | 12.5 | 46.9 | 2.1±0.2 | 5.8±0.5 |
| 10 | 1:5 | 1.8475 | 36.75 | 59 | 16.4±0.3 | 23.7±0.5 |
| 11 | 1:6.6 | 0.5 | 60 | 75 | 4.6±0.1 | 5.5±0.2 |
| 12 | 1:11.35 | 1.76 | 48.31 | 59 | 19.9±0.2 | 44.3±0.4 |
| 13 | 1:5 | 0.5 | 10 | 35 | 2.0±0.1 | 3.6±0.4 |
| 14 | 1:15 | 3.0375 | 60 | 45 | 20.2±0.5 | 7211 |
| 15 | 1:5 | 1.8475 | 36.75 | 59 | 17.0±0.5 | 27.7±0.9 |
| 16 | 1:7.85 | 0.5 | 60 | 45.2 | 2.0±0.2 | 3.0±0.2 |
| 17 | 1:10.95 | 1.9 | 36 | 35 | 7.9±0.1 | 16.5±0.1 |
| 18 | 1:8.5 | 2.74 | 10 | 49.4 | 16.5±0.3 | 43.5±0.2 |
| 19 | 1:15 | 4 | 10 | 35 | 9.3±0.8 | 50.6±0.3 |
| 20 | 1:5 | 3.9634 | 25.71 | 35 | 11.2±0.5 | 16.9±0.2 |
| 21 | 1:11.05 | 4 | 36.98 | 59 | 6.9±0.3 | 35.5±0.9 |
| 22 | 1:8.5 | 2.74 | 10 | 49.4 | 14.2±0.5 | 3912 |
| 23 | 1:7 | 3 | 60 | 60 | 1911 | 40±2 |

*Each reaction was performed once, the HPLC yields are provided as the mean value ± standard deviation between triplicate measurements.*

The HPLC yields of mixHC[8] and cycHC[8] obtained after the aging step were chosen as the response to factors (i)-(iv). The results were visualized as 3D response surfaces highlighting the most favorable conditions for the assembly of mixHC[8] or cycHC[8] (Figure 5, Table 9).

Yields of mixHC[8] and cycHC[8] showed a notably distinct response to the monomer ratio and acid loading (Figure 5). Variations of the monomer ratios within the range 1:5-1:15 did not significantly influence the yield of mixHC[8] (Figure 5a). In contrast, increasing the fraction of CU in the starting mixture of monomers resulted in an increased yield of cycHC[8] (Figure 5b). The quantity of $HClO_4$ appeared to be the most crucial parameter affecting formation of mixHC[8]. The highest yields of mixHC[8] were attained within the specific range of $HClO_4$ loadings (1.2-3.0 eq.), with a drastic decrease outside this range (Figure 5a). On the other hand, the yield of cycHC[8] was less affected by the quantity of acid catalyst used. Overall, the set of 3D response surfaces (Figure 5) suggested that the quantity of acid and the aging temperature are the most significant parameters influencing the formation of mixHC[8], whereas the generation of cycHC[8] is primarily controlled by the initial ratio of monomers, and less dependent on milling time and aging temperature. During the aging process, the optimal temperature for ripening mixHC[8] was found to be within 40-65°C. By contrast, cycHC[8] did not show an explicit favorable range (Figure 5). With this understanding, the inventors looked for further options to improve the formation of mixHC[8].

Hexafluorophosphate ($PF_6^-$) also serves as a suitable template for the synthesis of homomeric cycHC[8] in solution.[51] Moreover, this anion has a greater affinity for cycHC[8] compared to that of perchlorate.[27] Due to its advantageous templating effect, hexafluorophosphoric acid ($HPF_6$) was chosen as an alternative reagent to mediate the solid-state synthesis of mixHC[8]. This choice was also preferred due to the undesirable oxidative properties of perchlorates.[52,53] To our delight, $HPF_6$ showed promise in the initial experiment and resulted in an improved yield (28%) of

mixHC[8] (Figure 6a, Table 2). Equipped with the knowledge that the yield of mixHC[8] is highly sensitive to the quantity of templating acid (Figure 5a), the inventors investigated three different hexafluorophosphate salts ($AgPF_6$, $[Cu(CH_3CN)_4]PF_6$, $KPF_6$) as additives to increase the loading of the templating anion without a concurrent increase in acidity (Figure 6b, Table 2). Moreover, the inventors anticipated that the $Ag^+$ and $Cu^+$ cations could serve as potential promoters for the generation of mixHC[8] due to their affinity for biotin.[54-56] As depicted in Figure 6b, the $Ag^+$ and $Cu^+$ salts significantly increased the yield of homomeric cycHC[8]. This result could point to the effective downregulation of antagonistic[57] biotinylated mixed-oligomeric species, as the latter could disassemble and provide CU-oligomers for self-organization into cycHC[8]. Notably, $Ag^+$ and $Cu^+$ had no effect on the formation of the biotinylated macrocycle. The best result was achieved with $KPF_6$, which afforded the highest yield (37%) of mixHC[8], while having a minor effect on the accompanying formation of cycHC[8] (38% yield). Maintaining the same anion in the salt/acid mixture was crucial since the aqueous $H_2SO_4/KPF_6$ mixture was less efficient and resulted in significantly lower yields of both macrocycles (Table 3). These results confirm that the high affinity of the templating anion (Table 3), alkali metal salt, and the presence of a strong acid governed the adaptive self-assembly of oligomers into 8-membered HCs in the solid state. Variation of the urea monomer ratios (1:5, 1:7, and 1:15) without changing other parameters in the hexafluorophosphate-templated reaction confirmed that a 1:7 stoichiometric ratio afforded the highest yield of mixHC[8] (Figure 6c, Table 3). After identifying the key chemical factors and their optimal values, the duration of mechanical agitation and aging at moderately elevated temperatures were explored.

The changes in the chemical space of oligomeric intermediates and the distribution of biotin monomers among them, were tracked by a comprehensive study with the aid of HPLC-HRMS analysis. The oligomeric and macrocyclic compositions of the crude reaction mixtures produced at different milling times were investigated prior to and after the aging stage. Altogether, over 100 reaction intermediates were identified, and their concentrations were mapped based on MS signal intensities (Table 7). The results are summarized in a flow chart (Figure 7) that highlights the dynamic changes in the composition of the reaction mixture during milling and aging. It was found that biotin is incorporated into different linear oligomers $(CU)_x(B)_y$ ($x$ = 1...7, $y$ = 1...4), as well as into a number of mixHC[n] macrocycles ($n$ = 6...8), with up to 4 biotin units. Homomeric cyclohexadiylurea oligomers dominate at the initial phase of the polycondensation reaction (milling time: 5 min) and are kinetically favored products. Consequently, the milling time must be sufficient to enable the accumulation of the more slowly generated mixed biotin-containing oligomers (mixOLIGO). The content of the mixOLIGO increased in the sample milled for 60 min, during which a substantial change in the reaction mixture composition was observed. This difference in the contents of the short- and long-milled samples highlights the dynamic shuffling of the monomeric units, which resulted in an increase in the random distribution of biotin upon prolonged milling. Remarkably, generation of the macrocycles was already observed at the milling stage in the present study, although in small quantities. This observation is in line with the previously observed unfavorable complexation,[27] most likely due to an increase in entropy in the analogous syntheses of cycHC[*n*]s,[32] which prevented the templated self-organization process during milling. The macrocyclic products predominantly ripened at the aging stage, at which point the hetero- and homomeric oligomers underwent additional (though less intensive) crossover unit exchange.

Further understanding of the dynamic processes and interconversion of intermediates occurring at the milling stage was obtained by tracking the fate of selected intermediate species, namely, homodimers $(B)_2$ and $(CU)_2$, and homotrimers of CU and biotin (Figure 7 and Table 8). In this more detailed study, the milling time range was expanded to 5-90 min, along with more frequent data sampling within the time interval (Table 5). In addition, the reaction mixtures produced were subjected to 24 h of aging. HPLC-MS analysis was performed to determine the relative content of the characteristic short oligomers and yields of mixHC[8] and cycHC[8] prior to and after the aging stage.

The more detailed study confirmed that coupling between the CU monomers is kinetically preferred and occurs at the initial phase of the polycondensation reaction. Thus, the quantity of homodimer $(CU)_2$ drastically increased after 5 min of milling and subsequently underwent rapid decay (Figure 7a). The inclusion of the biotin units occurred at a slower rate, and the concentration of the respective homodimer $(B)_2$ remained nearly steady. Similar behavior was observed with the respective trimers (Table 8). The yields of the macrocycles generated at the milling stage did not exceed 20%, but greatly increased at the aging stage. Notably, the macrocyclic composition of the aged mixtures was strongly affected by the milling time, which reflects the composition of the initial oligomeric mixture. This dependence confirms that the shuffling of the monomeric units is inconsequential at the aging step. Thus, aging of the short-milled reaction mixture (5 min), which is composed mainly of homomeric cyclohexadiylurea oligomers, resulted in the ripening of cycHC[8] as the dominant product (55% yield), along with only a minor quantity of mixHC[8] (16% yield). However, fast C-N bond formation and cleavage occurring during the milling resulted in rapid dynamic changes in the composition of the oligomeric mixture, which is expected to vary until equilibrium is reached, with a random distribution of the biotin units. The reaction mixtures agitated for more than 20 min resulted in, after aging, drastic differences in the macrocyclic product distribution, in contrast to the shorter milling periods (Figure 7d). The yield of mixHC[8] notably increased from 16% to 37%, concurrently with a decreased yield of cycHC[8]. The yields of both macrocycles were expected to reach a plateau at longer milling times; however, they approached the equilibrium point in a peculiar oscillating manner.

The oscillating behavior was not an artefact since it was readily reproducible in a series of replicated experiments,

and the observed deviations exceeded the calculated confidence intervals. The inventors believe that this bizarre oscillating behavior is a consequence of complex dynamic processes occurring in the self-assembling oligomeric mixtures, in which a subtle change can result in the amplification of different intermediate products. Finally, close examination of the aging time (Figure 7e, Table 5) revealed that the maximal content of mixHC[8] was already achieved after 3 h, and the self-assembly process was essentially complete.

To further elucidate the advantages of the developed solid-state synthesis compared to the conventional solution-based approach, the inventors attempted a preparation of mixHC[8] in solution. The use of aqueous $HPF_6$ in the mixture of acetonitrile proved to be unfeasible as it polymerizes the co-solvent at higher concentrations, and at lower equivalents resulted in a complicated mixture of products with only traces of mixHC[8]. Therefore, a milder synthetic protocol, which was used for the preparation of cycHC[8] was employed.[51] The condensation of biotin and CU monomers (1:7) with paraformaldehyde and mediated by trifluoroacetic acid in acetonitrile produced 10% mixHC[8] and 38% cycHC [8] (Table 10). Moreover, the formation of cycHC[8] was favored, as shown in the selectivity values (Table 10). The outcome of this protocol confirms the superiority of the solid-state mechanochemical approach. In addition, the latter showed remarkably low process mass intensity (PMI = 4) compared to the solution synthesis (PMI = 306). The essentially solvent-free conditions enhance the templating effect via the high concentration of reactants and the desolvation effect.[22] Solution state processes are strongly dependent on diffusion, and diffusion constants vary widely for monomers, aggregates, and oligomeric intermediates, thereby affecting their reactivity during self-assembly. In the mechanochemically-assisted solid-state reaction, the molecular size of the intermediates does not strongly influence their mixing; thus, different cross-over reactions between variably sized intermediates proceed more effectively. This could be one of the factors that explains the observed highly efficient covalent self-assembly. In addition to the chemical advantages, solvent-free synthesis is also greener, produces less waste, and is less dangerous, as shown by a comparison of the respective green metrics (Table 10).[58]

**Table 10.** Comparison of mechanochemical and solution-based synthesis of mixHC[8].

| Metrics[a] | Mechanochemistry | Solution |
|---|---|---|
| HPLC yield | 37% | 10% |
| PMI | 4 | 306 |
| Conversion | 76% | 53% |
| Selectivity | 50% | 18% |

[a] Throughout the metric calculations, the HPLC yield was used to characterize the synthetic method. Isolation was considered a separate procedure. PMI, process mass intensity

After the mechanochemical procedure, which afforded the highest rate of conversion, mixHC[8], (-)-mixHC[8] and its pseudo-enantiomer (+)-mixHC[8], were isolated after purification in 16% and 11% yields and in highly pure form (88% and 90% by NMR, respectively) in ca. 300 mg quantities after a single preparative run. The structure of the diastereomeric mixHC[8]s was characterized by 1D and 2D NMR and provided clearly distinguishable spectra. The density functional theory (DFT) calculations revealed two distinct conformations for both diastereomers: one in which the sulfur atom of the thiolane envelope points toward the macrocycle and another where the same sulfur atom points away from the macrocycle (Figure 9).

According to the DFT calculations, neither of the diastereomers had a favorable conformer, as the energy difference between the conformers was less than 1 kJ $mol^{-1}$. Thus, the calculations suggest that the thiolane envelope is adaptive and lacks a preference for either of the conformers. However, when comparing the two diastereoisomers with $PF_6^-$ as a guest, the conformers with the sulfur pointing outside were favored.

The insertion of a biotin unit into the structure of the macrocycle creates new potential binding sites. Thus, its behavior as anion receptor was investigated and compared to the parent cycHC[8].

*Anion binding properties*

The electron-deficient cavity of HCs enables encapsulation of suitably sized anionic guests.[28] Efficient anion recognition has been reported for bambusurils,[43,59-62] heterobambusurils,[63] biotinurils,[64,65] and cycHCs,[27] The anion binding of the new (-)-mixHC[8] was screened by ESI-MS. Singly charged 1:1 complexes were detected with $PF_6^-$, $SbF_6^-$, $ClO_4^-$, $I^-$, $NO_3^-$, $Cl^-$, $ReO_4^-$, $SCN^-$, $H_2PO_4^-$, $Br^-$, $HSO_4^-$, $CF_3SO_3^-$, $NO_2^-$, and $BF_4^-$. The ion mobility MS analysis showed that the drift times of all complexes did not significantly differ, confirming their similar size. The most abundant complexes were observed for large chaotropic[66] $PF_6^-$, $SbF_6^-$, $ClO_4^-$, and $I^-$ anions, which included the templates used in the synthesis. Unlike cycHC[8], which showed only weak complexation with the halides, mixHC[8] interacted more readily with iodide. No binding was observed for $F^-$, $CO_3^{2-}$, and $CH_3CO_2^-$. Complex kinetic stability in the gas phase was studied for the selected

anionic guests via MS/MS energy-resolved collision-induced dissociation (ER-CID) experiments. Analogous to the trends observed for cycHC[8], the most efficient dissociation of inclusion complexes with mixHC[8] was observed for the larger anions. Solution phase binding of $PF_6^-$ and $ClO_4^-$ with mixHC[8] was investigated by ITC (Table 11) in methanol and a methanol:water mixture (1:1), with the collected data showing an exothermic enthalpy-driven complexation process. The association constants for $PF_6^-$ are 23 and 33 times greater than that of $ClO_4^-$ in methanol and the 1:1 methanol:water mixture, respectively, which explains the better templating property of $PF_6^-$. Slightly lower binding of $PF_6^-$ by (-)-mixHC[8] 10500 $M^{-1}$ compared to cycHC[8] 12900 $M^{-1}$ in methanol reflects changes in the specific interactions caused by the biotin insertion.

**Table 11.** Isothermal calorimetric titration outcomes for the complexation of mixHC[8] with (TBA)$ClO_4$ and (TBA)$PF_6$ (TBA = tetrabutylammonium) in methanol and a 1:1 methanol/water mixture; association constant *K* was determined using a 1:1 stoichiometry model.

| Guest | Solvent | (-)-mixHC[8] | | | (+)-mixHC[8] | | |
|---|---|---|---|---|---|---|---|
| | | *K* ($M^{-1}$) | *ΔH* (kJ/mol) | -*TΔS* (kJ/mol) | *K* ($M^{-1}$) | *ΔH* (kJ/mol) | -*TΔS* (kJ/mol) |
| (TBA) $ClO_4$ | $CH_3OH$ | 460±5 | -30.5±0.4 | 15.3±0.4 | 970±20 | -33.8±0.7 | 16.8±0.6 |
| | $CH_3OH:H_2O$ 1:1 | 19013 | -30.8±0.3 | 17.8±0.3 | 45612 | -40.7±0.8 | 25.5±0.8 |
| (TBA) $PF_6$ | $CH_3OH$ | 1050017 00 | -38.5±0.6 | 15.6±0.4 | 15900±4 00 | -41.7±0.5 | 17.6±0.6 |
| | $CH_3OH:H_2O$ 1:1 | 6300±20 0 | -4412 | 2212 | 14000±3 000 | -60.3±0.5 | 3711 |

It is noteworthy that the association constants of (+)-mixHC[8] diastereomer are 1.5-2.5 times higher than the those obtained for (-)-mixHC[8], reflecting slight differences in the steric accessibility of the biotin unit for anion binding.

The inventors were fortunate to obtain a co-crystal, containing the $PF_6^-$@(-)-((*S,S,R*)(*R,R*)$_7$)-mixHC[8] inclusion complex (Figure 10). Superimposing the measured crystal structure and the DFT-calculated structure of (-)-((*S,S,R*)(*R,R*)$_7$)-mixHC[8] revealed a very similar arrangement of the atoms in space, which confirms an outward pointing envelope confirmation of the biotin thiolane unit.

Single crystal X-ray diffraction (SC-XRD) afforded the crystal structure of the host-guest complex, which unequivocally confirms the 1:1 stoichiometry of the anion inclusion complex in the solid state. Single crystals of the $PF_6^-$@(-)-((*S,S,R*)(*R,R*)$_7$-mixHC[8] inclusion complex were obtained from a solution of (-)-((*S,S,R*)(*R,R*)$_7$)-mixHC[8] in methanol with $PF_6^-$ added as a tetrabutylammonium (TBA) salt. Additionally, the TBA salt of $ClO_4^-$, as well as $Ag^+$, $K^+$, and $Na^+$ salts of $PF_6^-$ were screened; however, only the TBA salt of $PF_6^-$ yielded single crystals. Crystallizations were also set up with (+)-((*S,S,R*)(*S,S*)$_7$)-mixHC[8], however, crystals did not form under any of the conditions used.

The guest anion $PF_6^-$ is locked in the fixed position at the center of the (-)-((*S,S,R*)(*R,R*)$_7$)-mixHC[8], and displays no disorder. Similar to the inclusion complex of cycHC[8] with $PF_6^-$, two fluorine atoms point toward the portals of the macrocycle,[27] while the other four fluorine atoms of $PF_6^-$ lie on the equatorial plane of the macrocycle and point toward the four corners of its square-shaped belt. Thorough SC-XRD analysis unambiguously revealed that the obtained crystal structure is a co-crystal with the cycHC[8] and (-)-((*S,S,R*)(*R,R*)$_7$)-mixHC[8] with a 50:50 disordered state of the cyclohexyl and biotin 5-membered ring. Hirshfeld surface[67] analysis of the complex revealed that the C-H···F interactions are responsible for the fixed orientation of the guest. Analysis of the distances between guest and host revealed the shortest distance between $PF_6^-$ and biotin (2.448(1) Å), indicating a strong contribution from the biotin unit to the complex's stability. Calculations predicted the adaptability of the thiolane conformation. In the crystal structure, however, the preferred sulfur atom position is toward the outside of the macrocycle. This preference is most likely due to the electron-rich guest, which minimizes the complex's energy via repulsion of the electron-dense sulfur atom. The crystal structure of (-)-mixHC[8] also shows that the tethered carboxylic group is not participating in the formation of the host-guest complex and can be employed for chemical modification without disruption of the macrocycle's binding properties.

*Removal of perchlorate from a Martian soil simulant*

Here the inventors demonstrate how the selective anion-capturing properties of mixHC[8] can be utilized for the removal of perchlorates from contaminated soil samples. Perchlorate is a persistent pollutant that adversely affects human health by interfering with thyroid hormone production, and occurs in soil, ground water, and food.[52,53] The accumulation of perchlorate in fertilizers, soil and irrigation water leads to increased plant uptake and subsequent food-chain transfer.[68,69] This pollutant has been found in various environmental matrices on Earth and typically originates from human activities. However, a surprisingly high content of naturally occurring perchlorates has been discovered in Martian soil, which could contain up to 0.5-1% of distributed $ClO_4^-$ on that planet.[70] The perchlorate on Mars could become a critical chemical hazard for astronauts during a manned Mars exploratory mission, or it could be converted into a valuable resource as a globally-abundant source of oxygen.[70,71] Hence, Martian soil was an aptly novel substrate to test the ability of mixHC[8] to capture

perchlorate via a solid-phase extraction (SPE) process. The carboxylate side-chain of mixHC[8] enabled its facile covalent immobilization on the surface of 3-aminopropyl silica gel (APS).[72] The solid perchlorate-extracting material obtained (mixHC[8]-APS) contained ca. 12% (w/w) of mixHC[8], according to infrared spectroscopic analysis (Figure 11a). Exolith MGS-1 Martian Global Simulant[73] was employed as the matrix and spiked with (TBA)$ClO_4$, imitating contamination with perchlorate (1% w/w). According to ion chromatography analysis, the methanolic extract of the contaminated MGS-1 contained primarily perchlorate and sulfate, the latter arising from the $MgSO_4$ component of MGS-1. Treatment of the methanolic extract with solid mixHC[8]-APS resulted in complete removal of $ClO_4^-$ (Figure 11b, Table 8). In contrast, non-modified APS resulted in poor extraction of $ClO_4^-$ (ca. 15%) but removed a significant fraction (85%) of $SO_4^{2-}$. Sulfate removal occurred with similar efficiency (ca. 85-97%) using both APS and mixHC[8]-APS, demonstrating that mixHC[8] is not the main contributor responsible for the capture of $SO_4^{2-}$. Latter was also proven by an ITC experiment.

The captured perchlorate was easily removed by washing with water, since the binding constant is lower in the aqueous phase and demonstrates the potential for the reusability of the material.

Conclusions

An efficient mechanochemical protocol for the synthesis of the first chimeric enantiopure mono-biotinylated hemicucurbit[8]urils was developed. The process involves two stages: (i) the mechanochemically-assisted and acid-catalyzed polycondensation of $_D$-biotin, (*R*,*R*)- or (*S*,*S*)-cyclohexa-1,2-diylurea and formaldehyde; and (ii) the aging step, in which a template-driven self-assembly of linear-chain oligomers into macrocycles takes place. The process of optimization uncovered a set of crucial parameters affecting the yields of the macrocyclic products. Among them, the ratio of the monomers, loading of acid catalysts, and the nature of the templating anion were the most significant factors. Different responses to the parameters have been observed for mixHC[8] and cycHC[8], which enabled the development of an optimal synthetic methodology featuring the highest yield (37%) of mixHC[8] from a 1:7 mixture of the urea monomers in the presence of $HPF_6$ and $KPF_6$, as an acid catalyst and a donor of the templating anion, respectively. The developed solid-state approach greatly outperformed the synthetic method in solution, in terms of yield, product selectivity, process mass intensity, and other relevant green chemistry metrics. The present study also offers a rare example of a comprehensive insight into the complex mixture of oligomeric intermediates, including their interconversion and self-organization processes while on route to the macrocyclic products. The fate of selected oligomeric intermediates was tracked with HPLC-MS analysis and revealed the importance of the agitation time in the ball mill prior to the aging step. Yields of mixHC [8] after the aging step were found to vary from 16% to 37% and generally increased with an increase in milling time, although this association showed a peculiar oscillating character. The low yield of mixHC[8] obtained after short milling periods was attributed to the formation of homomeric cyclohexadiylurea oligomers as kinetic products at the initial phase of the polycondensation reaction. The relatively slower inclusion of biotin, compared to the cyclohexane-derived urea, heavily influenced the dynamics of the process. Therefore, the milling time must be sufficient (> 20 min) to ensure uniform distribution of the biotin units in the dynamically interconverted mixture of oligomers and, consequently, high-yielding ripening of mixHC[8] at the aging stage.

Anion binding studies of new mixHC[8] in the gas phase and in solution demonstrated its higher affinity for $PF_6^-$, $SbF_6^-$ , $ClO_4^-$, and $I^-$ anions. The carboxylate group of mixHC[8] enabled its facile covalent immobilization on aminated silica. The functional material obtained was employed for the selective capture of anions, as demonstrated by the complete removal of perchlorate from an extract of a Martian soil simulant.

**References**

(1) Aav, R.; Shmatova, E.; Reile, I.; Borissova, M.; Topić, F.; Rissanen, K. New Chiral Cyclohexylhemicucurbit[6]Uril. *Org. Lett.* **2013,** *15* (14), 3786-3789. https://doi.org/10.1021/ol401766a.

(2) Prigorchenko, E.; Öeren, M.; Kaabel, S.; Fomitšenko, M.; Reile, I.; Järving, I.; Tamm, T.; Topić, F.; Rissanen, K.; Aav, R. Template-Controlled Synthesis of Chiral Cyclohexylhemicucurbit[8]Uril. *Chem. Commun.* **2015,** *51* (54), 10921-10924. https://doi.org/10.1039/C5CC04101E.

(3) Fuentes de Arriba, Á. L.; Seisdedos, D. G.; Simón, L.; Alcázar, V.; Raposo, C.; Morán, J. R. Synthesis of Monoacylated Derivatives of 1,2- Cyclohexanediamine. Evaluation of Their Catalytic Activity in the Preparation of Wieland-Miescher Ketone. J. Org. Chem. 2010, 75 (23), 8303-8306. https://doi.org/10.1021/jo101723v.

(4) Kaabel, S.; Stein, R. S.; Fomitšenko, M.; Järving, I.; Friščić, T.; Aav, R. Size-Control by Anion Templating in Mechanochemical Synthesis of Hemicucurbiturils in the Solid State. *Angew. Chem. Int. Ed.* **2019,** *58* (19), 6230-6234. https://doi.org/10.1002/anie.201813431.

(5) Fomitšenko, M.; Peterson, A.; Reile, I.; Cong, H.; Kaabel, S.; Prigorchenko, E.; Järving, I.; Aav, R. A Quantitative Method for Analysis of Mixtures of Homologues and Stereoisomers of Hemicucurbiturils That Allows Us to Follow

Their Formation and Stability. *New J. Chem.* **2017,** *41* (6), 2490-2497. https://doi.org/10.1039/C6NJ03050E.
(6) Becke, A. D. Density-Functional Thermochemistry. III. The Role of Exact Exchange. J. Chem. Phys. 1993, 98 (7), 5648-5652. https://doi.org/10.1063/1.464913.
(7) Lee, C.; Yang, W.; Parr, R. G. Development of the Colle-Salvetti Correlation-Energy Formula into a Functional of the Electron Density. Phys. Rev. B 1988, 37 (2), 785-789. https://doi.org/10.1103/PhysRevB.37.785.
(8) Vosko, S. H.; Wilk, L.; Nusair, M. Accurate Spin-Dependent Electron Liquid Correlation Energies for Local Spin Density Calculations: A Critical Analysis. Con. J. Phys. 1980, 58 (8), 1200-1211. https://doi.org/10.1139/p80-159.
(9) Stephens, P. J.; Devlin, F. J.; Chabalowski, C. F.; Frisch, M. J. Ab Initio Calculation of Vibrational Absorption and Circular Dichroism Spectra Using Density Functional Force Fields. J. Phys. Chem. 1994, 98 (45), 11623-11627. https://doi.org/10.1021/j100096a001.
(10) Dewar, M. J. S.; Zoebisch, E. G.; Healy, E. F.; Stewart, J. J. P. Development and Use of Quantum Mechanical Molecular Models. 76. AM1: A New General Purpose Quantum Mechanical Molecular Model. J. Am. Chem. Soc. 1985, 107 (13), 3902-3909. https://doi.org/10.1021/ja00299a024.
(11) Hariharan, P. C.; Pople, J. A. The Influence of Polarization Functions on Molecular Orbital Hydrogenation Energies. Theor. Chim. Acta 1973, 28 (3), 213-222. https://doi.org/10.1007/BF00533485.
(12) Ditchfield, R.; Hehre, W. J.; Pople, J. A. Self-Consistent Molecular-Orbital Methods. IX. An Extended Gaussian-Type Basis for Molecular-Orbital Studies of Organic Molecules. J. Chem. Phys. 1971, 54 (2), 724-728. https://doi.org/10.1063/1.1674902.
(13) Hehre, W. J.; Ditchfield, K.; Pople, J. A. Self-Consistent Molecular Orbital Methods. XII. Further Extensions of Gaussian-Type Basis Sets for Use in Molecular Orbital Studies of Organic Molecules. J. Chem. Phys. 1972, 56 (5), 2257-2261. https://doi.org/10.1063/1.1677527.
(14) Grimme, S.; Ehrlich, S.; Goerigk, L. Effect of the Damping Function in Dispersion Corrected Density Functional Theory. J. Comput. Chem. 2011, 32 (7), 1456-1465. https://doi.org/10.1002/jcc.21759.
(15) Frisch, M. J.; Trucks, G. W.; Schlegel, H. B.; Scuseria, G. E.; Robb, M. A.; Cheeseman, J. R.; Scalmani, G.; Barone, V.; Petersson, G. A.; Nakatsuji, H.; Li, X.; Caricato, M.; Marenich, A. V.; Bloino, J.; Janesko, B. G.; Gomperts, R.; Mennucci, B.; Hratchian, H. P.; Ortiz, J. V.; Izmaylov, A. F.; Sonnenberg, J. L.; Williams; Ding, F.; Lipparini, F.; Egidi, F.; Goings, J.; Peng, B.; Petrone, A.; Henderson, T.; Ranasinghe, D.; Zakrzewski, V. G.; Gao, J.; Rega, N.; Zheng, G.; Liang, W.; Hada, M.; Ehara, M.; Toyota, K.; Fukuda, R.; Hasegawa, J.; Ishida, M.; Nakajima, T.; Honda, Y.; Kitao, O.; Nakai, H.; Vreven, T.; Throssell, K.; Montgomery Jr., J. A.; Peralta, J. E.; Ogliaro, F.; Bearpark, M. J.; Heyd, J. J.; Brothers, E. N.; Kudin, K. N.; Staroverov, V. N.; Keith, T. A.; Kobayashi, R.; Normand, J.; Raghavachari, K.; Rendell, A. P.; Burant, J. C.; Iyengar, S. S.; Tomasi, J.; Cossi, M.; Millam, J. M.; Klene, M.; Adamo, C.; Cammi, R.; Ochterski, J. W.; Martin, R. L.; Morokuma, K.; Farkas, O.; Foresman, J. B.; Fox, D. J. Gaussian 16 Rev. C.01, 2016.
(16) Sheldrick, G. M. SHELXT - Integrated Space-Group and Crystal-Structure Determination. Acta Crystallogr. Sect. Found. Adv. 2015, 71 (1), 3-8. https://doi.org/10.1107/S2053273314026370.
(17) Dolomanov, O. V.; Bourhis, L. J.; Gildea, R. J.; Howard, J. a. K.; Puschmann, H. OLEX2: A Complete Structure Solution, Refinement and Analysis Program.J. Appl. Crystallogr. 2009, 42 (2), 339-341. https://doi.org/10.1107/S0021889808042726.
(18) Spackman, M. A.; Jayatilaka, D. Hirshfeld Surface Analysis. CrystEngComm 2009, 11 (1), 19-32. https://doi.org/10.1039/B818330A.
(19) Spackman, P. R.; Turner, M. J.; McKinnon, J. J.; Wolff, S. K.; Grimwood, D. J.; Jayatilaka, D.; Spackman, M. A. CrystalExplorer: A Program for Hirshfeld Surface Analysis, Visualization and Quantitative Analysis of Molecular Crystals. J. Appl. Crystallogr. 2021, 54 (3), 1006-1011. https://doi.org/10.1107/S1600576721002910.
(20) Schnitzer, T.; Preuss, M. D.; van Basten, J.; Schoenmakers, S. M. C.; Spiering, A. J. H.; Vantomme, G.; Meijer, E. W. How Subtle Changes Can Make a Difference: Reproducibility in Complex Supramolecular Systems. Angew. Chem. 2022, 134 (41), e202206738. https://doi.org/10.1002/ange.202206738.
(21) Yamaoki, Y.; Nagata, T.; Kondo, K.; Sakamoto, T.; Takami, S.; Katahira, M. Shedding Light on the Base-Pair Opening Dynamics of Nucleic Acids in Living Human Cells. Nat. Commun. 2022, 13 (1), 7143. https://doi.org/10.1038/s41467-022-34822-4.
(22) Kwon, T.; Song, B.; Nam, K. W.; Stoddart, J. F. Mechanochemical Enhancement of the Structural Stability of Pseudorotaxane Intermediates in the Synthesis of Rotaxanes. J Am Chem Soc 2022, 144 (28), 12595-12601. https://doi.org/10.1021/jacs.2c00515.
(23) Drašínský, M.; Hurtado, C. S.; Masson, E.; Kaleta, J. Stuffed Pumpkins: Mechanochemical Synthesis of Host-Guest Complexes with Cucurbit[7]Uril. *Chem. Commun.* **2021,** *57* (17), 2132-2135. https://doi.org/10.1039/D1CC00240F.
(24) Kaabel, S.; Aav, R. Templating Effects in the Dynamic Chemistry of Cucurbiturils and Hemicucurbiturils. Isr. J. Chem. 2018, 58 (3-4), 296-313. https://doi.org/10.1002/ijch.201700106.
(25) Lehn, J.-M. Dynamic Combinatorial Chemistry and Virtual Combinatorial Libraries. Chem. - Eur. J. 1999, 5 (9), 2455-2463. https://doi.org/10.1002/(SICI)1521-3765(19990903)5:9<2455::AID-CHEM2455>3.0.CO;2-H.

(26) Corbett, P. T.; Leclaire, J.; Vial, L.; West, K. R.; Wietor, J.-L.; Sanders, J. K. M.; Otto, S. Dynamic Combinatorial Chemistry. Chem. Rev. 2006, 106 (9), 3652-3711. https://doi.org/10.1021/cr020452p.
(27) Kaabel, S.; Adamson, J.; Topić, F.; Kiesilä, A.; Kalenius, E.; Öeren, M.; Reimund, M.; Prigorchenko, E.; Lõokene, A.; Reich, H. J.; Rissanen, K.; Aav, R. Chiral Hemicucurbit[8]Uril as an Anion Receptor: Selectivity to Size, Shape and Charge Distribution. *Chem. Sci.* **2017,** *8* (3), 2184-2190. https://doi.org/10.1039/C6SC05058A.
(28) Andersen, N. N.; Lisbjerg, M.; Eriksen, K.; Pittelkow, M. Hemicucurbit[n]Urils and Their Derivatives - Synthesis and Applications. Isr. J. Chem. 2018, 58 (3-4), 435-448. https://doi.org/10.1002/ijch.201700129.
(29) Lisbjerg, M.; Jessen, B. M.; Rasmussen, B.; Nielsen, B. E.; Madsen, A. Ø.; Pittelkow, M. Discovery of a Cyclic 6 + 6 Hexamer of D-Biotin and Formaldehyde. Chem. Sci. 2014, 5 (7), 2647-2650. https://doi.org/10.1039/C4SC00990H.
(30) Havel, V.; Yawer, M. A.; Sindelar, V. Real-Time Analysis of Multiple Anion Mixtures in Aqueous Media Using a Single Receptor. Chem. Commun. 2015, 51 (22), 4666-4669. https://doi.org/10.1039/C4CC10108A.
(31) Yawer, M. A.; Havel, V.; Sindelar, V. A Bambusuril Macrocycle That Binds Anions in Water with High Affinity and Selectivity. Angew. Chem. Int. Ed. 2015, 54 (1), 276-279. https://doi.org/10.1002/anie.201409895.
(32) Kaabel, S.; Stein, R. S.; Fomitšenko, M.; Järving, I.; Friščić, T.; Aav, R. Size-Control by Anion Templating in Mechanochemical Synthesis of Hemicucurbiturils in the Solid State. *Angew. Chem. Int. Ed.* **2019,** *58* (19), 6230-6234. https://doi.org/10.1002/anie.201813431.
(33) Langerreiter, D.; Kostiainen, M. A.; Kaabel, S.; Anaya-Plaza, E. A Greener Route to Blue: Solid-State Synthesis of Phthalocyanines. Angew. Chem. Int. Ed. 2022, 61 (42), e202209033. https://doi.org/10.1002/anie.202209033.
(34) Pascu, M.; Ruggi, A.; Scopelliti, R.; Severin, K. Synthesis of Borasiloxane-Based Macrocycles by Multicomponent Condensation Reactions in Solution or in a Ball Mill. Chem. Commun. 2012, 49 (1), 45-47. https://doi.org/10.1039/C2CC37538A.
(35) Sim Y.;, Shi, Y. X.; Ganguly, R.; Li Y.;, Garcia, F. Mechanochemical Synthesis of Phosphazane-Based Frameworks. Chem. - Eur. J. 2017, 23 (47), 11279-11285. https://doi.org/10.1002/chem.201701619.
(36) Xi, H.-T.; Zhao, T.; Sun, X.-Q.; Miao, C.-B.; Zong, T.; Meng, Q. Rapid and Efficient Solvent-Free Synthesis of Cyclophanes Based on Bipyridinium under Mechanical Ball Milling. RSC Adv. 2012, 3 (3), 691-694. https://doi.org/10.1039/C2RA22802E.
(37) Kunde, T.; Pausch, T.; Guńka, P. A.; Krzyżanowski, M.; Kasprzak, A.; Schmidt, B. M. Fast, Solvent-Free Synthesis of Ferrocene-Containing Organic Cages via Dynamic Covalent Chemistry in the Solid State. *Chem. Sci.* **2022,** *13* (10), 2877-2883. https://doi.org/10.1039/D1SC06372C.
(38) Shy, H.; Mackin, P.; Orvieto, A. S.; Gharbharan, D.; Peterson, G. R.; Bampos, N.; Hamilton, T. D. The Two-Step Mechanochemical Synthesis of Porphyrins. Faraday Discuss. 2014, 170 (0), 59-69. https://doi.org/10.1039/C3FD00140G.
(39) Su, Q.; Hamilton, T. D. Extending Mechanochemical Porphyrin Synthesis to Bulkier Aromatics: Tetramesitylporphyrin. Beilstein J. Org. Chem. 2019, 15 (1), 1149-1153. https://doi.org/10.3762/bjoc.15.111.
(40) Zeng, Q.; Long, Q.; Lu, J.; Wang, L.; You, Yuan, X.; Zhang, Q.; Ge, Q.; Cong, H.; Liu, M. Synthesis of a Novel Aminobenzene-Containing Hemicucurbituril and Its Fluorescence Spectral Properties with Ions. Beilstein J. Org. Chem. 2021, 17 (1), 2840-2847. https://doi.org/10.3762/bjoc.17.195.
(41) Wang, L.; Han, J.; Pan, R.; Yuan, X.; You, Cen, X.; Zhang, Q.; Ge, Q.; Cong, H.; Liu, M. Synthesis of Hybrid Thiohemicucurbiturils. Tetrahedron Lett. 2022, 101, 153918. https://doi.org/10.1016/j.tetlet.2022.153918.
(42) Maršálek, K.; Šindelář, V. Monofunctionalized Bambus[6]Urils and Their Conjugates with Crown Ethers for Liquid-Liquid Extraction of Inorganic Salts. *Org Lett* **2020,** *22* (4), 1633-1637. https://doi.org/10.1021/acs.orglett.0c00216.
(43) De Simone, N. A.; Chvojka, M.; Lapešová, J.; Martínez-Crespo, L.; Slávik, P.; Sokolov, J.; Butler, S. J.; Valkenier, H.; Šindelář, V. Monofunctionalized Fluorinated Bambusurils and Their Conjugates for Anion Transport and Extraction. *J Org Chem* **2022,** *87* (15), 9829-9838. https://doi.org/10.1021/acs.joc.2c00870.
(44) Marti-Centelles, V.; Pandey, M. D.; Burguete, M. I.; Luis, S. V. Macrocyclization Reactions: The Importance of Conformational, Configurational, and Template-Induced Preorganization. Chem. Rev. 2015, 115 (16), 8736-8834. https://doi.org/10.1021/acs.chemrev.5b00056.
(45) Friščić, T.; Childs, S. L.; Rizvi, S. A. A.; Jones, W. The Role of Solvent in Mechanochemical and Sonochemical Cocrystal Formation: A Solubility-Based Approach for Predicting Cocrystallisation Outcome. *CrystEngComm* **2009,** *11* (3), 418-426. https://doi.org/10.1039/B815174A.
(46) Do, J.-L.; Friščić, T. Chemistry 2.0: Developing a New, Solvent-Free System of Chemical Synthesis Based on Mechanochemistry. *Synlett* **2017,** *28* (16), 2066-2092. https://doi.org/10.1055/s-0036-1590854.
(47) Belenguer, A. M.; Lampronti, G. I.; De Mitri, N.; Driver, M.; Hunter, C. A.; Sanders, J. K. M. Understanding the Influence of Surface Solvation and Structure on Polymorph Stability: A Combined Mechanochemical and Theoretical Approach. J. Am. Chem. Soc. 2018, 140 (49), 17051-17059. https://doi.org/10.1021/jacs.8b08549.
(48) Lizal, T.; Sindelar, V. Bambusuril Anion Receptors. Isr. J. Chem. 2018, 58 (3-4), 326-333. https://doi.org/10.1002/ijch.201700111.

(49) Myers, R. H.; Montgomery, D. C.; Anderson-Cook, C. M. Response Surface Methodology: Process and Product Optimization Using Designed Experiments; John Wiley & Sons, 2016.
(50) Prigorchenko, E.; Kaabel, S.; Narva, T.; Baškir, A.; Fomitšenko, M.; Adamson, J.; Järving, I.; Rissanen, K.; Tamm, T.; Aav, R. Formation and Trapping of the Thermodynamically Unfavoured Inverted-Hemicucurbit[6]Uril. *Chem. Commun.* **2019**, *55* (63), 9307-9310. https://doi.org/10.1039/C9CC04990H.
(51) Prigorchenko, E.; Öeren, M.; Kaabel, S.; Fomitšenko, M.; Reile, I.; Järving, I.; Tamm, T.; Topić, F.; Rissanen, K.; Aav, R. Template-Controlled Synthesis of Chiral Cyclohexylhemicucurbit[8]Uril. *Chem. Commun.* **2015,** *51* (54), 10921-10924. https://doi.org/10.1039/C5CC04101E.
(52) Urbansky, E. T. Perchlorate as an Environmental Contaminant. Environ. Sci. Pollut. Res. 2002, 9 (3), 187-192. https://doi.org/10.1007/BF02987487.
(53) Kumarathilaka, P.; Oze, C.; Indraratne, S. P.; Vithanage, M. Perchlorate as an Emerging Contaminant in Soil, Water and Food. Chemosphere 2016, 150, 667-677. https://doi.org/10.1016/j.chemosphere.2016.01.109.
(54) Sigel, H.; Scheller, K. H. Metal Ion Complexes of D-Biotin in Solution. Stability of the Stereoselective Thioether Coordination. J. Inorg. Biochem. 1982, 16 (4), 297-310. https://doi.org/10.1016/S0162-0134(00)80266-4.
(55) Aoki, K.; Saenger, W. Interactions of Biotin with Metal Ions. X-Ray Crystal Structure of the Polymeric Biotin-Silver(i) Nitrate Complex: Metal Bonding to Thioether and Ureido Carbonyl Groups. J. Inorg. Biochem. 1983, 19 (3), 269-273. https://doi.org/10.1016/0162-0134(83)85031-4.
(56) Altaf, M.; Stoeckli-Evans, H. Chiral One- and Two-Dimensional Silver(I)-Biotin Coordination Polymers. Acta Crystallogr. C 2013, 69 (2), 127-137. https://doi.org/10.1107/S0108270113000322.
(57) Lehn, J.-M. Perspectives in Chemistry-Steps towards Complex Matter. Angew. Chem. Int. Ed. 2013, 52 (10), 2836-2850. https://doi.org/10.1002/anie.201208397.
(58) McElroy, C. R.; Constantinou, A.; Jones, L. C.; Summerton, L.; Clark, J. H. Towards a Holistic Approach to Metrics for the 21st Century Pharmaceutical Industry. Green Chem. 2015, 17 (5), 3111-3121. https://doi.org/10.1039/C5GC00340G.
(59) Jašíková, L.; Rodrigues, M.; Lapešová, J.; Lizal, T.; Šindelář, V.; Roithová, J. Bambusurils as a Mechanistic Tool for Probing Anion Effects. *Faraday Discuss.* **2019,** *220* (0), 58-70. https://doi.org/10.1039/C9FD00038K.
(60) Sokolov, J.; Štefek, A.; Šindelář, V. Functionalized Chiral Bambusurils: Synthesis and Host-Guest Interactions with Chiral Carboxylates. *ChemPlusChem* **2020,** *85* (6), 1307-1314. https://doi.org/10.1002/cplu.202000261.
(61) Itterheimová, P.; Bobacka, J.; Šindelář, V.; Lubal, P. Perchlorate Solid-Contact Ion-Selective Electrode Based on Dodecabenzylbambus[6]Uril. *Chemosensors* **2022,** *10* (3), 115. https://doi.org/10.3390/chemosensors10030115.
(62) Rando, C.; Vázquez, J.; Sokolov, J.; Kokan, Z.; Nečas, M.; Šindelář, V. Highly Efficient and Selective Recognition of Dicyanoaurate(I) by a Bambusuril Macrocycle in Water. *Angew. Chem. Int. Ed.* **2022,** 61 (43), e202210184. https://doi.org/10.1002/anie.202210184.
(63) Reany, O.; Mohite, A.; Keinan, E. Hetero-Bambusurils. Isr. J. Chem. 2018, 58 (3-4), 449-460. https://doi.org/10.1002/ijch.201700138.
(64) Lisbjerg, M.; Nielsen, B. E.; Milhøj, B. O.; Sauer, S. P. A.; Pittelkow, M. Anion Binding by Biotin[6]Uril in Water. Org. Biomol. Chem. 2014, 13 (2), 369-373. https://doi.org/10.1039/C4OB02211D.
(65) Andersen, N. N.; Eriksen, K.; Lisbjerg, M.; Ottesen, M. E.; Milhøj, B. O.; Sauer, S. P. A.; Pittelkow, M. Entropy/Enthalpy Compensation in Anion Binding: Biotin[6]Uril and Biotin-I-Sulfoxide[6]Uril Reveal Strong Solvent Dependency. J. Org. Chem. 2019, 84 (5), 2577-2584. https://doi.org/10.1021/acs.joc.8b02797.
(66) Assaf, K. I.; Nau, W. M. The Chaotropic Effect as an Assembly Motif in Chemistry. Angew. Chem. Int. Ed. 2018, 57 (43), 13968-13981. https://doi.org/10.1002/anie.201804597.
(67) Spackman, M. A.; Jayatilaka, D. Hirshfeld Surface Analysis. CrystEngComm 2009, 11 (1), 19-32. https://doi.org/10.1039/B818330A.
(68) Calderón, R.; Palma, P.; Arancibia-Miranda, N.; Kim, U.-J.; Silva-Moreno, E.; Kannan, K. Occurrence, Distribution and Dynamics of Perchlorate in Soil, Water, Fertilizers, Vegetables and Fruits and Associated Human Exposure in Chile. Environ. Geochem. Health 2022, 44 (2), 527-535. https://doi.org/10.1007/s10653-020-00680-6.
(69) Chen, Zhu, Z.; Wu, X.; Zhang, D.; Tong, J.; Lin, Yin, L.; Li, X.; Zheng, Q.; Lu, S. A Nationwide Investigation of Perchlorate Levels in Staple Foods from China: Implications for Human Exposure and Risk Assessment. J. Hazard. Mater. 2022, 439, 129629. https://doi.org/10.1016/j.jhazmat.2022.129629.
(70) Davila, A. F.; Willson, D.; Coates, J. D.; McKay, C. P. Perchlorate on Mars: A Chemical Hazard and a Resource for Humans. Int. J. Astrobiol. 2013, 12 (4), 321-325. https://doi.org/10.1017/S1473550413000189.
(71) Gayen, P.; Sankarasubramanian, S.; Ramani, V. K. Fuel and Oxygen Harvesting from Martian Regolithic Brine. Proc. Natl. Acad. Sci. 2020, 117 (50), 31685-31689. https://doi.org/10.1073/pnas.2008613117.
(72) Tabakci, M. Immobilization of Calix[6]Arene Bearing Carboxylic Acid and Amide Groups on Aminopropyl Silica Gel and Its Sorption Properties for Cr(VI). J. Incl. Phenom. Macrocycl. Chem. 2008, 61 (1), 53-60. https://doi.org/10.1007/s10847-007-9392-2.
(73) Cannon, K. M.; Britt, D. T.; Smith, T. M.; Fritsche, R. F.; Batcheldor, D. Mars Global Simulant MGS-1: A Rocknest-Based Open Standard for Basaltic Martian Regolith Simulants. Icarus 2019, 317, 470-478. https://doi.org/10.1016/j.icarus.2018.08.019.

**Example 2** - **Synthesis of mixHC[8] derivatives: mixHC[8]-$H_2$TPP, mixHC[8]-ZnTPP, mixHC[8]-BODIPY and mixHC[8]-Amine derivatives.**

**Material & Method**

*Procedure for the synthesis of mixHC[8]-$H_2$TPP derivative.*

To the solution of MixHC[8] (0.0454 mmol, 1 eq., 60 mg) and 5-(4-aminophenyl)-10,15,20-(triphenyl)porphyrin ($H_2$TPP-$NH_2$)(0.0545 mmol, 1.2 eq., 34.3 mg) in anhydrous dimethylformamide (DMF) (2 mL) diisopropylamine (DIPEA) (0.0908 mmol, 2 eq., 15.8 μl) was added, followed by the addition of hexafluorophosphate azabenzotriazole tetramethyl uronium (HATU) (0.0545 mmol, 1.2 eq., 20.7 mg) and further overnight stirring at r.t. The obtained reaction mixture was then concentrated under reduced pressure, and the residue was purified by silica gel chromatography with dicholoromethane (DCM)/Methanol (97:3) as eluent to give MixHC[8]-$H_2$TPP derivative (32 mg, 36% yield). HRMS (AJS-ESI) calc. for $C_{111}H_{130}N_{21}O_9S$ $[M+H]^+$ 1933.0076, found m/z 1933.0125; calc. for $C_{111}H_{131}N_{21}O_9S$ $[M+2H]^{2+}$ 967.0074, found *m*/*z* 967.0095.

*HPLC analysis- Column*: Waters Symmetry 300TM C18, 150x4.6mm, 5um, no guard column. Eluents: A - $H_2O$, C - $CH_3OH$, D - DCM, A:C:D 18:55:27. Flow rate -1 mL/min, 40°C.

HPLC purity - 68%.

*Procedure for the synthesis of mixHC[8]-ZnTPP derivative*

To the solution of Mix[8]-$H_2$TPP conjugate (0.00362 mmol, 1 eq., 7.0 mg in the mixture of $CHCl_3$:$CH_3OH$=5:3 (1.6 mL) $Zn(OAc)_2$ (0.0362 mmol, 10 equiv., 7.0 mg) was added, the resulting reaction mixture was refluxed for 2 hours, then cooled to room temperature and concentrated under reduced pressure. The residue was then transferred to the filter with water (5 mL), again washed with water (3×5 mL), methanol (3×5 mL) and dried in air. The crude product was then purified by silica gel chromatography with chloroform / methanol (99:1 to to 97:3) as eluent to give MixHC[8]-ZnTPP derivative (1.4 mg). HRMS (AJS-ESI) calc. for $C_{111}H_{127}N_{21}O_9SZnNa$ $[M+Na]^+$ 2016.9030, found *m*/*z* 2016.9012; calc. for $C_{111}H_{127}N_{21}O_9SZnNa_2$ $[M+2Na]^{2+}$ 1019.9461, found *m*/*z* 1019.9468.

*HPLC analysis* - Column: Hypersil ODS (C18), 150x4.6mm, 5um, no guard column. Eluents: A - $H_2O$, C - $CH_3OH$, D - DCM, A:C:D 14:56:30. Flow rate -1.0 mL/min, T: 30°C.

HPLC purity -70%.

*Procedure for the synthesis of mixHC[8]-BODIPY derivative*

To the solution of MixHC[8] (0.0454 mmol, 1 eq., 60 mg) and 1,3,5,7-tetramethyl-2,6-diethyl-8-(4-aminophenyl) BODIPY (0.0545 mmol, 1.2 eq., 21.5 mg) in anhydrous DMF (2 mL) DIPEA (0.0908 mmol, 2 eq., 15.8 μL) was added, followed by the addition of HATU (0.0545 mmol, 1.2 eq., 20.7 mg) and further stirring at r.t for 2 days. The obtained reaction mixture was then concentrated under reduced pressure, the residue was then purified by silica gel chromatography with dichloromethane/methanol (98:2 to 97:3) as eluent to give of MixHC[8]-BODIPY derivative (42 mg, 54 % yield). MS (ESI) calc. for $C_{90}H_{127}BF_2N_{19}O_9S^+$ $[M+H]^+$ 1699.0, found *m*/*z* 1699.0.

*HPLC analysis* - Column: KinetexC18, 100x2.1mm, 2.6 um + guard column. Eluents: C - $H_2O$+0.1% FA, D - ACN+0.1% FA. Flow rate - 0.25 ml/min, T:30°C.

Gradient:

time (min) -C (%) - D (%).

0-50-50

10- 10-90

15 - 10 - 90

15.01 - 50 - 50

22-50-50

HPLC purity - 90%.

*Procedure for the synthesis of mixHC[8]-Benzyl amine derivative*

To the solution of MixHC[8] (0.0303 mmol, 1 eq., 40 mg) and N-Benzylethane-1,2-diamine (0.0606 mmol, 2 eq., 9.1 μL) in anhydrous DMF (0.7 mL) DIPEA (0.0606 mmol, 2 eq., 10.5 μL) was added, followed by the addition of HATU (0.0606 mmol, 2 eq., 23 mg) and further stirring at r.t for 24 hrs. The obtained reaction mixture was then concentrated under reduced pressure, the residue was then purified by silica gel chromatography with dichloromethane/methanol (8:1) as eluent to give MixHC[8]-Benzyl amine derivative (40 mg, 91%yield).

*Procedure for the synthesis of mixHC[8]-Amine derivative*

To the solution of MixHC[8]-Benzyl amine derivative (0.0296 mmol, 1 eq., 40 mg) in methanol (3.0 mL) 10% Pd/C (~20 mg) was added, and the mixture was stirred under $H_2$ gas at r.t. for 24 hrs. The obtained reaction mixture was filtered through Celite® and concentrated under reduced pressure, the residue was then purified by silica gel chromatography with dichloromethane/methanol (1:1) as eluent to give MixHC[8]-Amine derivative (3 mg). MS (ESI) calc. for $C_{69}H_{107}N_{18}O_9S^+$ $[M+H]^+$ 1363.8, found *m/z* 1363.8.

*HPLC analysis* - Column: Kinetex XB-C18, 150x4.6 mm, 2.6um, with guard column. Eluents: C - $H_2O$+0.1% FA, D - ACN+0.1% FA. Flow rate - 0.75 ml/min, T:30°C.

Gradient:

time (min) -C (%) - D (%).

0-90-10

15 - 10 - 90.

HPLC purity - 79%.

## Results

Synthesis methods for five derivatives of monobiotinylated hemicucurbit[8]uril is achieved in good yields, these compounds can be further used for sensing and catalysis applications. For all these compounds versatile amidation reaction was used to attach fluorescent dye to the hemicucurbituril. Porphyrin free base conjugate of hemicucurbituril is on the figure 12, zinc tetraphenylprophyrin conjugate with hemicucurbituril on figure 14, BODIPY is covalently attached for the hemicucurbituril (cf.Figure 16) and aminofunctionalised mixed macorcycles is shown on the figure 18 and 19.

## Claims

**1.** Method of preparation of at least one chimeric hemicucurbit[n]uril, comprising the following steps:

- Contacting, under mechanical agitation, at least two different cyclic urea derivatives and a compound that can form methylene bridges in the presence of at least one acid, and at least one solvent;
- Aging the products obtained at the previous step for at least about 30 minutes;

wherein n is an even positive integer comprised between 4 and 14;
wherein the amount of liquid per amount of solid in the reaction media is initially < 1 μL/mg.

**2.** Method according to claim 1, wherein the at least two different cyclic urea derivatives are *N,N'*-(1,2-ethanediyl)-urea derivatives according to formula (I):

(I) 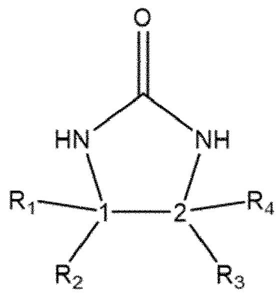

wherein 1 and 2 are carbon atoms and at least one of them is a stereogenic center, preferably both of them are stereogenic centers; wherein at least one of the substituents R1 to R4 bears a stereogenic center; and wherein $R_1$, $R_2$, $R_3$, and $R_4$ can each independently be selected from the group consisting in a hydrogen atom, -OH, -COOH, $-NH_2$, $-NO_2$, - $NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl.

**3.** Method according to claim 1 or 2, wherein the at least two different cyclic urea derivatives are selected from the group consisting in 4,5-Dimethyl-2-imidazolidinone, 4-alkyl-2-imidazolidinone, 2-imidazolidinone, rel-(3aR,6aS)-Hexahydropyrrolo[3,4-d]imidazol-2(1H)-one, 2-Oxo-4-imidazolidinecarboxylic acid, 2-Oxo-4-imidazolidinecarbonitrile, 4-(Aminomethyl)-2-imidazolidinone, 4-(Hydroxymethyl)-2-imidazolidinone, hexahydro-2(1H)-Cyclopentimidazolone, 4-(2-Hydroxyethyl)-2-imidazolidinone, rel-(3aR,6aS)-Tetrahydro-1H-furo[3,4-d]imidazol-2(3H)-one, Octahydroimidazo[4,5-d]azepin-2(1H)-one, 4-(Ethoxymethyl)-2-imidazolidinone, 4,5-Dipropyl-2-imidazolidinone, (4R or 4S)-4-Phenyl-2-imidazolidinone, (4S or 4R)-2-Oxo-4-imidazolidineacetic acid, 8-Methyl-1,3,7-triazaspiro[4.4]nonan-2-one, (4S or 4R)-4-(4-Pyridinyl)-2-imidazolidinone, 4,5-Dialkyl-2-imidazolidinone, 2-Oxo-4-imidazolidinepropanoic acid, 4-(4-Hydroxyphenyl)-2-imidazolidinone, 1H-Thieno[3,4-d]imidazol-2(3H)-one, tetrahydro-, 5,5-dioxide, (3aR,6aR)-Spiro[1-azabicyclo[2.2.2]octane-3,4'-imidazolidin]-2'-one, 2-[(4R)-2-Oxo-4-imidazolidinyl]benzonitrile, ethylene urea, *N,N'*-cyclohexa-1,2-diylurea, D-biotin, their stereoisomers thereof, and their derivatives thereof, preferably ethylene urea, *N,N'*-cyclohexa-1,2-diylurea, D-biotin, their stereoisomers thereof, and their derivatives thereof.

**4.** Method according to any of the preceding claims, wherein one of the cyclic urea derivative is (*S,S*)-*N,N'*-cyclohexa-1,2-diylurea or (*R,R*)-*N,N'*-cyclohexa-1,2-dylurea and the other cyclic urea derivative is D-biotin.

**5.** Method according to any of the preceding claims wherein n is comprised between 6 and 14 and the step of contacting further takes place in the presence of at least one templating anion.

**6.** Method according to claim 5, wherein the at least one templating anion is selected from the group consisting in $Cl^-$, $Br^-$, $I^-$, $BF_4^-$, $IO_4^-$, $ClO_4^-$, $ReO_4^-$ , $PF_6^-$, $SbF_6^-$, $SO_4^{2-}$, $BO_3^{2-}$, $NO^{3-}$, $PO_3^{3-}$, $PO4^{2-}$, and $RCO_2^-$, R being selected from the group consisting in hydrogen atom, -OH, -COOH, $-NH_2$, $-NO_2$, $-NHNH_2$, nitrile, alkyl, fluoro-alkyl, bromo-alkyl, chloro-alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl, or a combination thereof, preferably $PF_6^-$.

**7.** Method according to any of the preceding claims wherein the at least one acid has a pK < 1, and is preferably selected from the group consisting in HCl, HBr, HI, $H_2SO_4$, $H_3BO_3$, $HNO_3$, $H_3PO_3$, $H_3PO_4$, $HReO_4$, $HClO_4$, $HPF_6$, $HSbF_6$, $HBF_4$, $HIO_4$, RCOOH, R being selected from or RCOOH, R being selected from the group consisting in hydrogen atom, - OH, -COOH, $-NH_2$, $-NO_2$, $-NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl, or a combination thereof, preferably $HPF_6$.

**8.** Method according to any of the preceding claims, wherein the step of contacting further takes place in the presence of a salt and said salt is preferably selected from the list consisting in the combination of any templating anion according to claim 6 with a cation, and a combination thereof, more preferably selected from the list consisting in $AgPF_6$, $[Cu(CH_3CN)4]PF_6$, $KPF_6$, and a combination thereof, even more preferably said salt is $KPF_6$.

**9.** Method according to any of the preceding claims, wherein the aging step last at least about 1 h, more preferably at least about 1 h 30, still preferably between about 2 h and about 24 h, yet preferably between about 2 h 30 and about 16 h, even more preferably between about 3 h and about 12 h.

**10.** Chimeric hemicucurbit[n]uril obtained by the method according to anyone of claims 1to 9.

**11.** Chimeric hemicucurbit[n]uril comprising at least two different cyclic urea monomers, wherein n is an even positive integer comprised between 4 and 14 and wherein the at least two different cyclic urea monomers are *N,N'*-(1,2-ethanediyl)-urea derivatives according to formula (I):

(I)

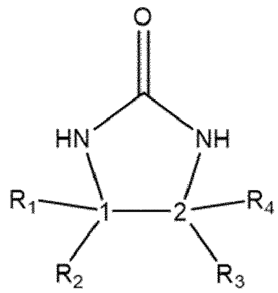

wherein 1 and 2 are carbon atoms and at least one of them is a stereogenic center, preferably both of them are stereogenic centers; wherein at least one of the substituents R1 to R4 bears a stereogenic center; and wherein $R_1$, $R_2$, $R_3$, and $R_4$ can each independently be selected from the group consisting in a hydrogen atom, -OH, -COOH, $-NH_2$, $-NO_2$, - $NHNH_2$, nitrile, alkyl, alkenyl, alkynyl, cycloalkyl, cycloalkenyl, aryl, alkoxy, hydrocarbylthio, hydrocarbylamino, dihydrocarbylamino, carboxyl, aryl and heteroaryl.

**12.** Chimeric hemicucurbit[n]uril according to claim 11, wherein said chimeric hemicucurbit[n]uril is according to one of the following formula, the different monomers present in the cycle of said formula being in any order:

(II) -[-$CH_2$-(Cyclic urea derivative $1)_a$-$CH_2$-(Cyclic urea derivative $2)_b$-$CH_2$-]-

wherein a + b = n;

(III) -[-$CH_2$-( Cyclic urea derivative $1)_a$-$CH_2$-( Cyclic urea derivative $2)_b$-$CH_2$-]-

wherein a + b = 6;

(IV) -[-$CH_2$-( Cyclic urea derivative $1)_a$-$CH_2$-( Cyclic urea derivative $2)_b$-$CH_2$-]-

wherein a + b = 8;

(VIII)

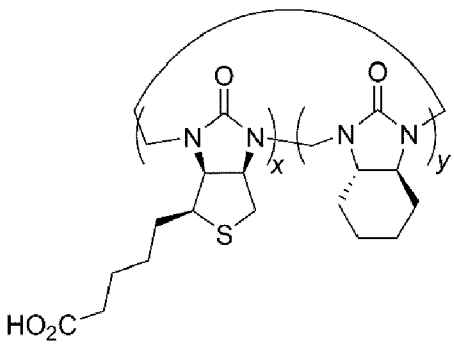

wherein x + y = 6;

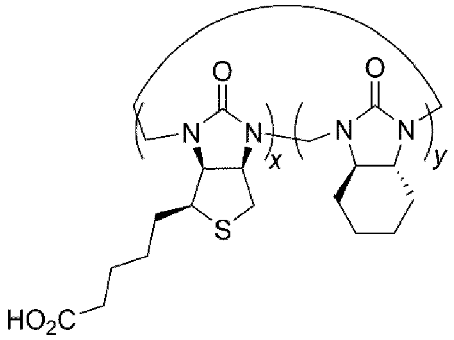

(IX)

wherein x + y = 6;

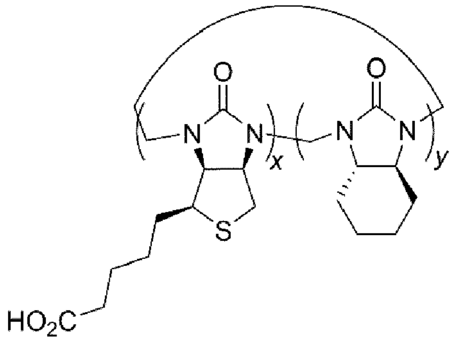

(X)

wherein x + y = 8;

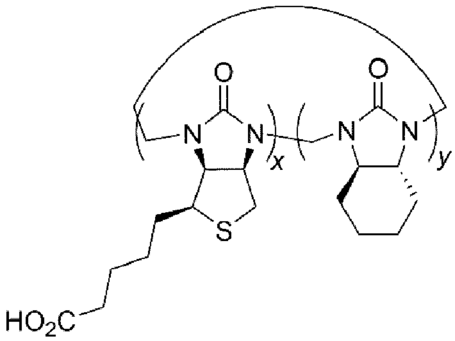

(XI)

wherein x + y = 8;

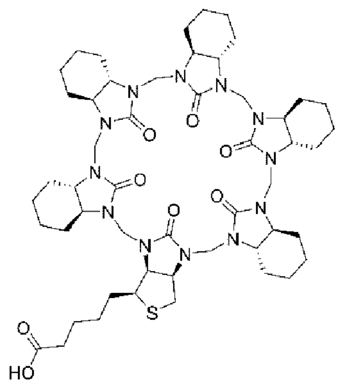

(XII) ;

(XIII) 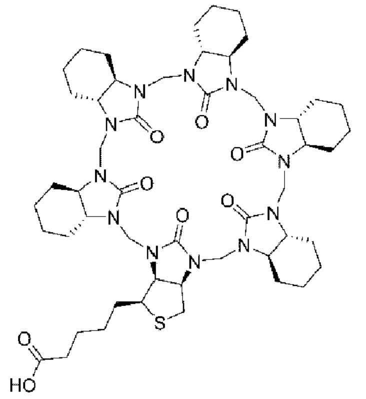 ;

(XIV) 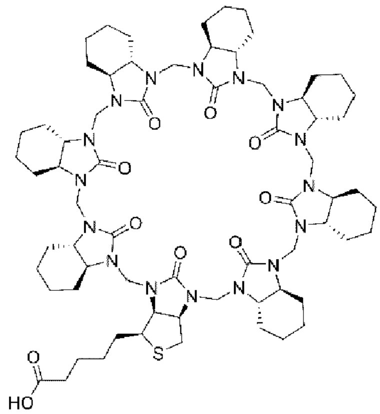 ;

(XV) 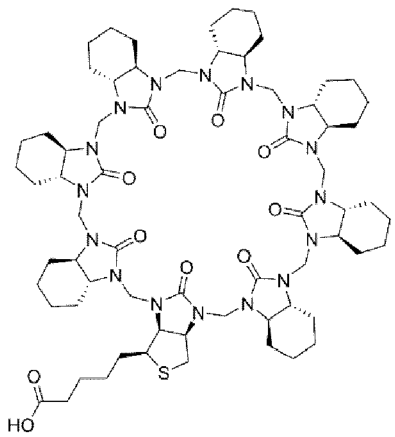 ;

(XVI) 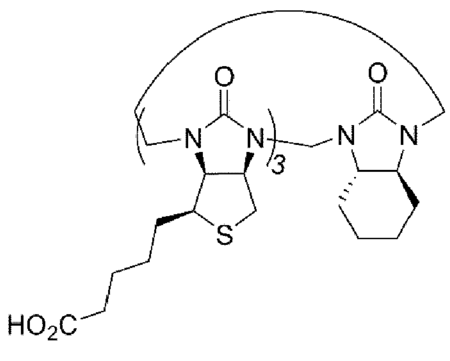 ;

(XVII) 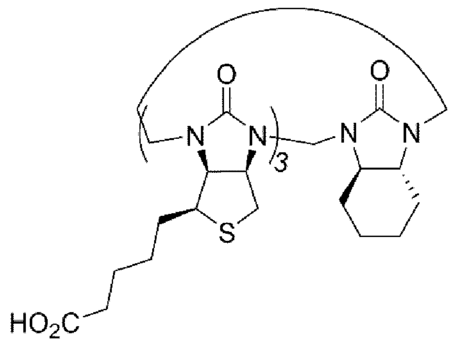 ;

(XVIII) 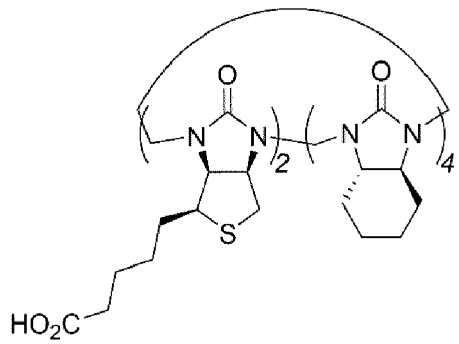 ;

(XIX) 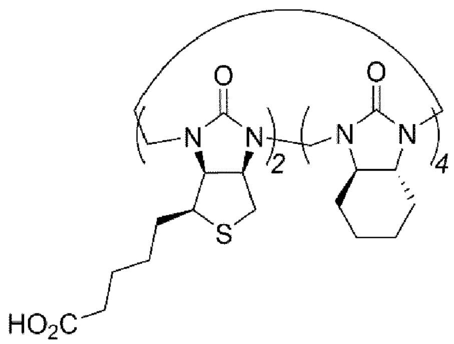 ;

(XX) 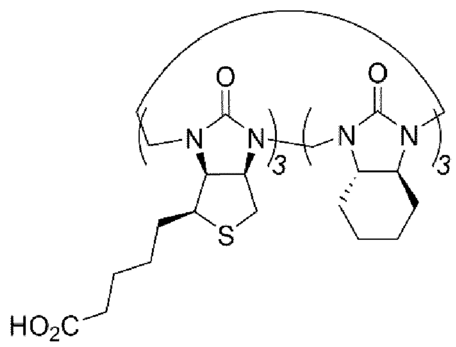 ;

(XXI) 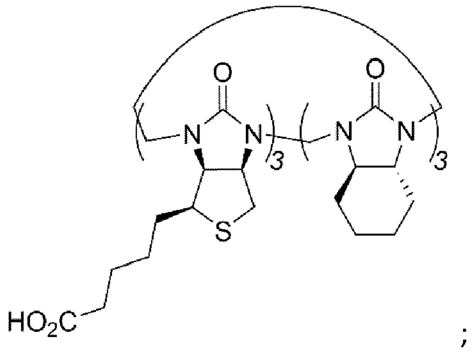 ;

(XXII) 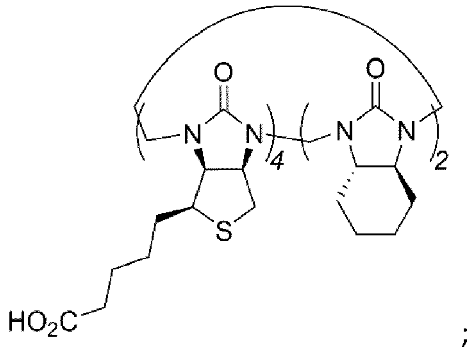 ;

(XXIII) 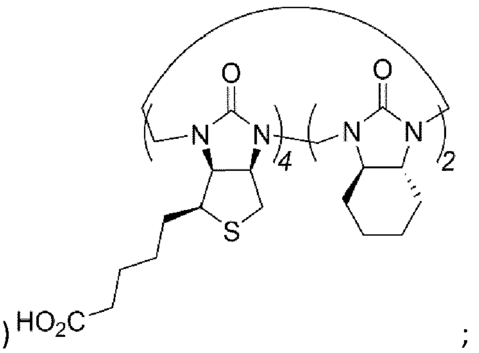 ;

(XXIV) 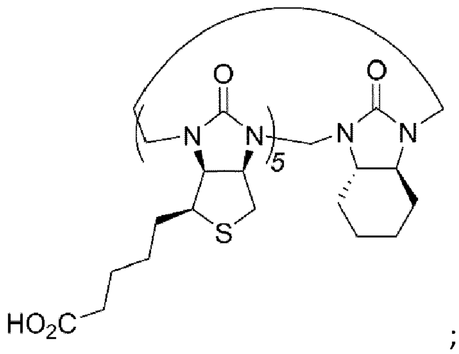 ;

(XXV) 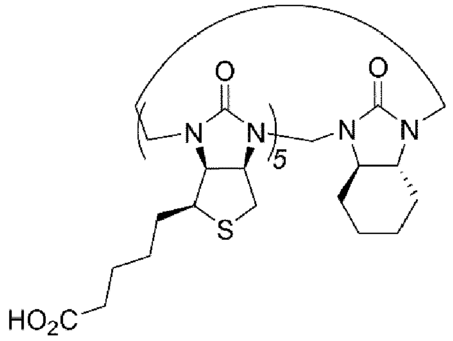 ;

(XXVI) 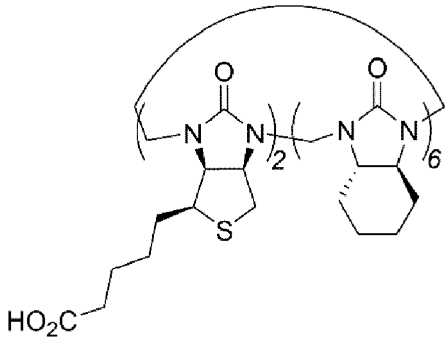 ;

(XXVII) 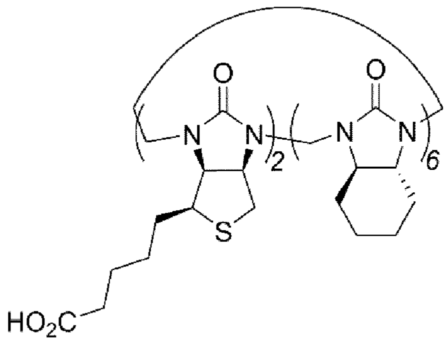 ;

(XXVIII) 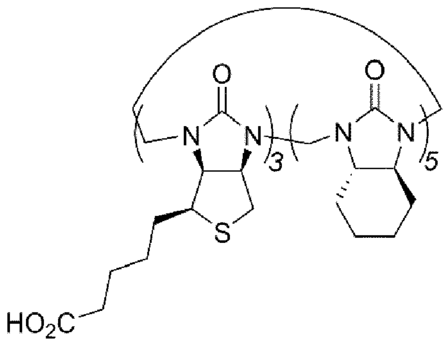 ;

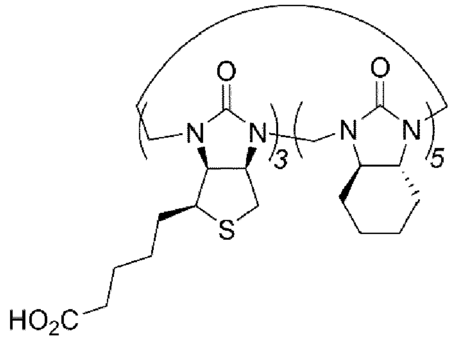

(XXIX) ;

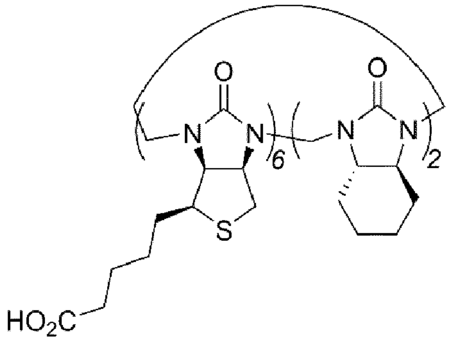

(XXX) ;

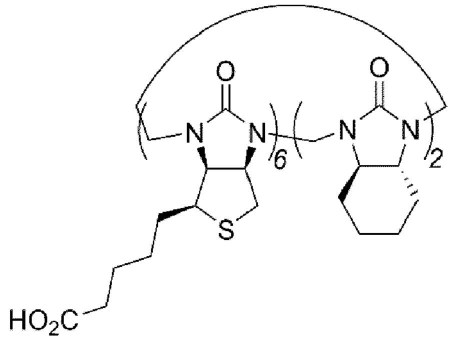

(XXXI) ;

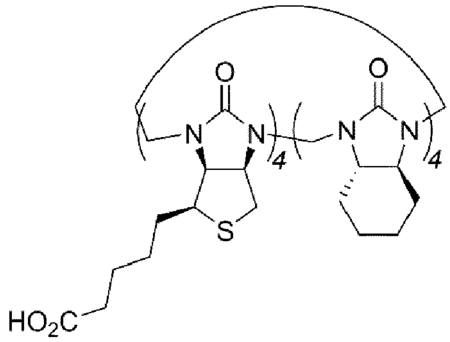

(XXXII) ;

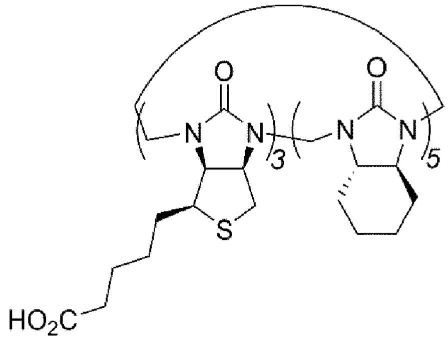

(XXXIV) ;

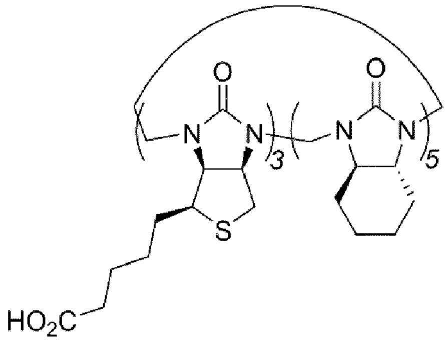

(XXXV) ;

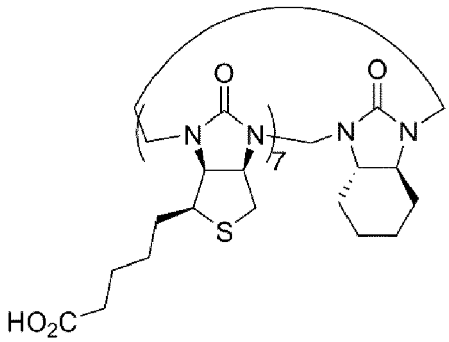

(XXXVI) ;

or

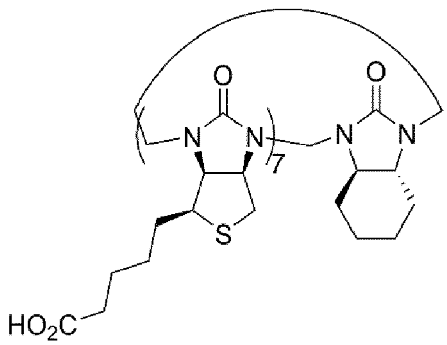

(XXXVII) .

**13.** Chimeric hemicucurbit[n]uril according to claim 12, wherein the chimeric hemicucurbit[n]uril is the (-)-((*S,S,R*)(*R,R*)$_7$)-mono-biotinylated cyclohexanohemicucurbit[8]uril (XIV) or the(+)-((*S,S,R*)(*S,S*)$_7$)-mono-biotinylated cyclohexanohemicucurbit[8]uril (XV) according to the following formula:

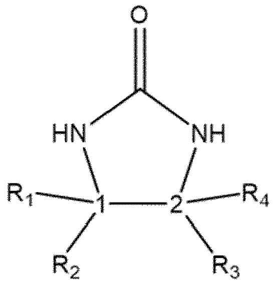

(XIV)

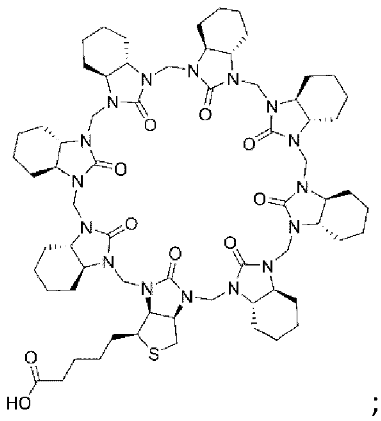

;

and

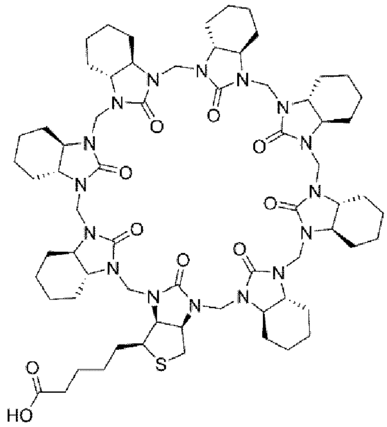

(XV)

.

**14.** Chimeric hemicucurbit[n]uril-modified material wherein a chimeric hemicucurbit[n]uril according to anyone of claims 10 to 13 is attached to a material preferably selected from the group consisting in a solid surface or an insoluble compound, preferably from a metal surface, a silica support, an alumina support and a polymer support, more preferably a silica support, even more preferably a 3-aminopropyl silico gel.

**15.** Use of the chimeric hemicucurbit[n]uril(s) according to anyone of claims 10 to 13 or the chimeric hemicucurbit[n]uril-modified material according to claim 14 for binding anions, cations or organic molecules.

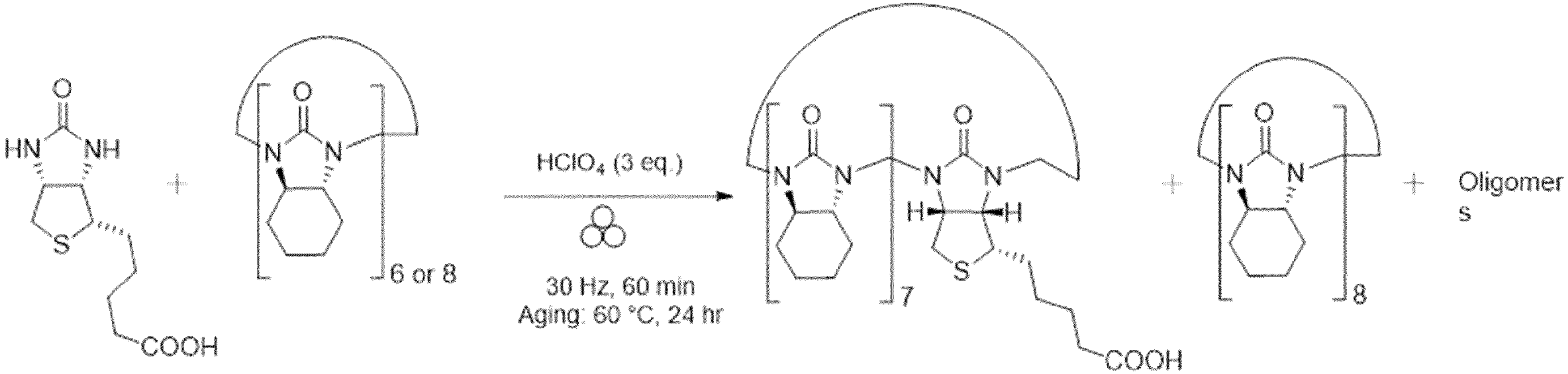


Biotin

cycHC[6]
or cycHC[8]

mixHC[8]

cycHC[8]

**Figure 1**

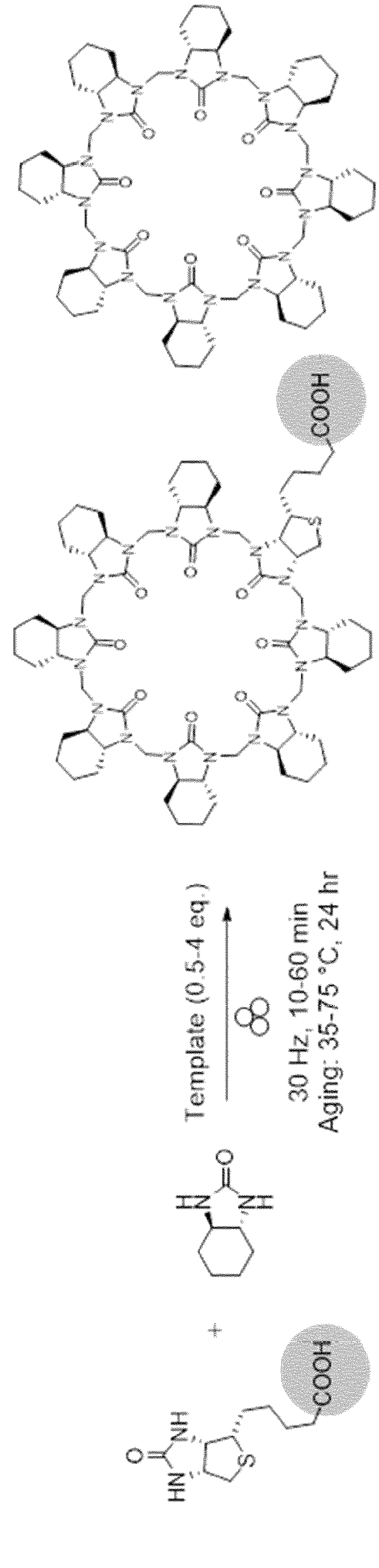


Biotin (1 eq.)

+

(*R,R*)- or (*S,S*)-cyclohexa-1,2-diylurea (5-15 eq.)

(−)- or (+)-mixHC[8]

(*R,R*)- or (*S,S*)-cycHC[8]

**Figure 2**

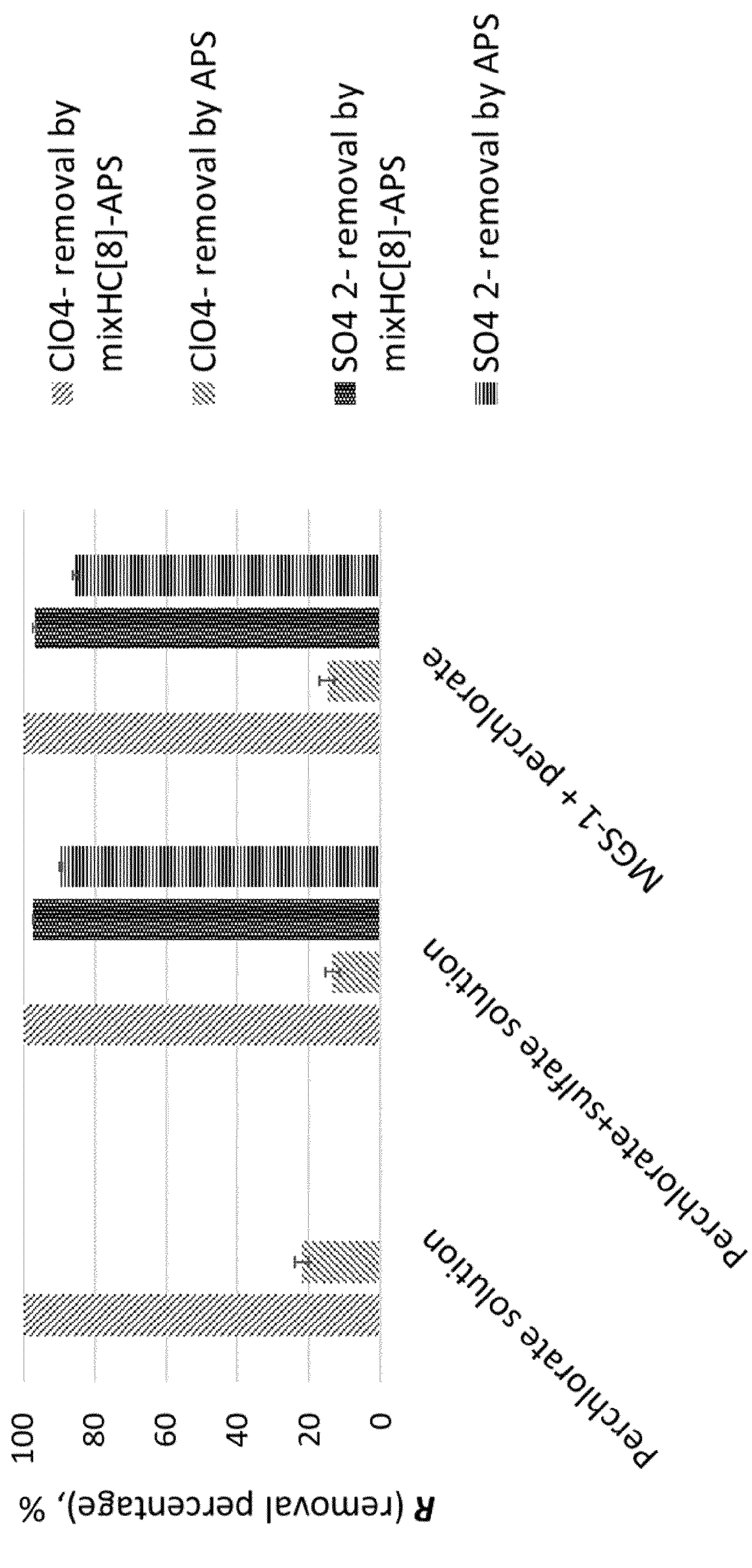
ClO4- removal by mixHC[8]-APS
ClO4- removal by APS
SO4 2- removal by mixHC[8]-APS
SO4 2- removal by APS
R (removal percentage), %
100
80
60
40
20
0
Perchlorate solution
Perchlorate+sulfate solution
MGS-1 + perchlorate

**Figure 3**

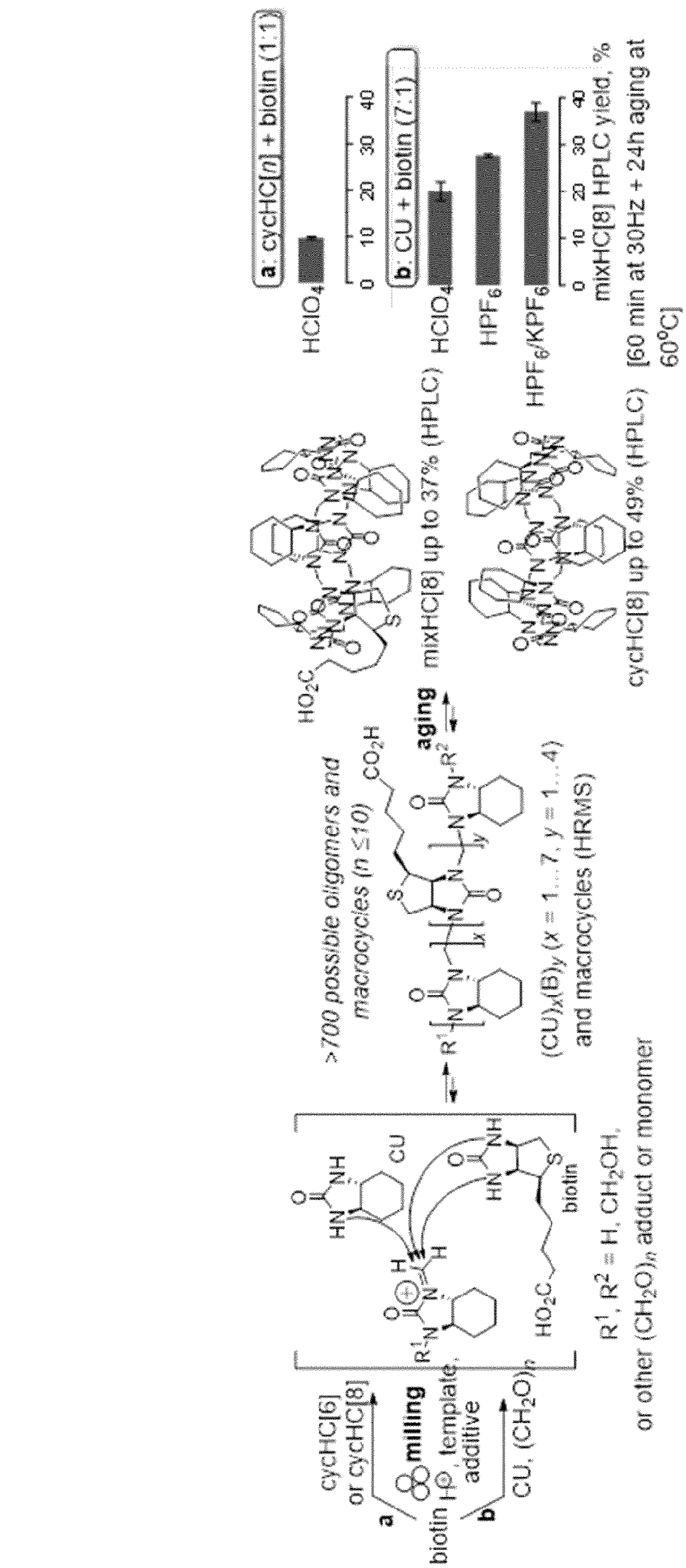
a
cycHC[6]
or cycHC[8]
biotin
milling
H⊕, template,
additive
b
CU, $(CH_2O)_n$
CU
biotin
$R^1$, $R^2$ = H, $CH_2OH$,
or other $(CH_2O)_n$ adduct or monomer
>700 possible oligomers and
macrocycles (n ≤10)
$(CU)_x(B)_y$ (x = 1…7, y = 1…4)
and macrocycles (HRMS)
aging
mixHC[8] up to 37% (HPLC)
cycHC[8] up to 49% (HPLC)
a: cycHC[n] + biotin (1:1)
$HClO_4$
b: CU + biotin (7:1)
$HClO_4$
$HPF_6$
$HPF_6/KPF_6$
0 10 20 30 40
mixHC[8] HPLC yield, %
[60 min at 30Hz + 24h aging at
60°C]

Figure 4

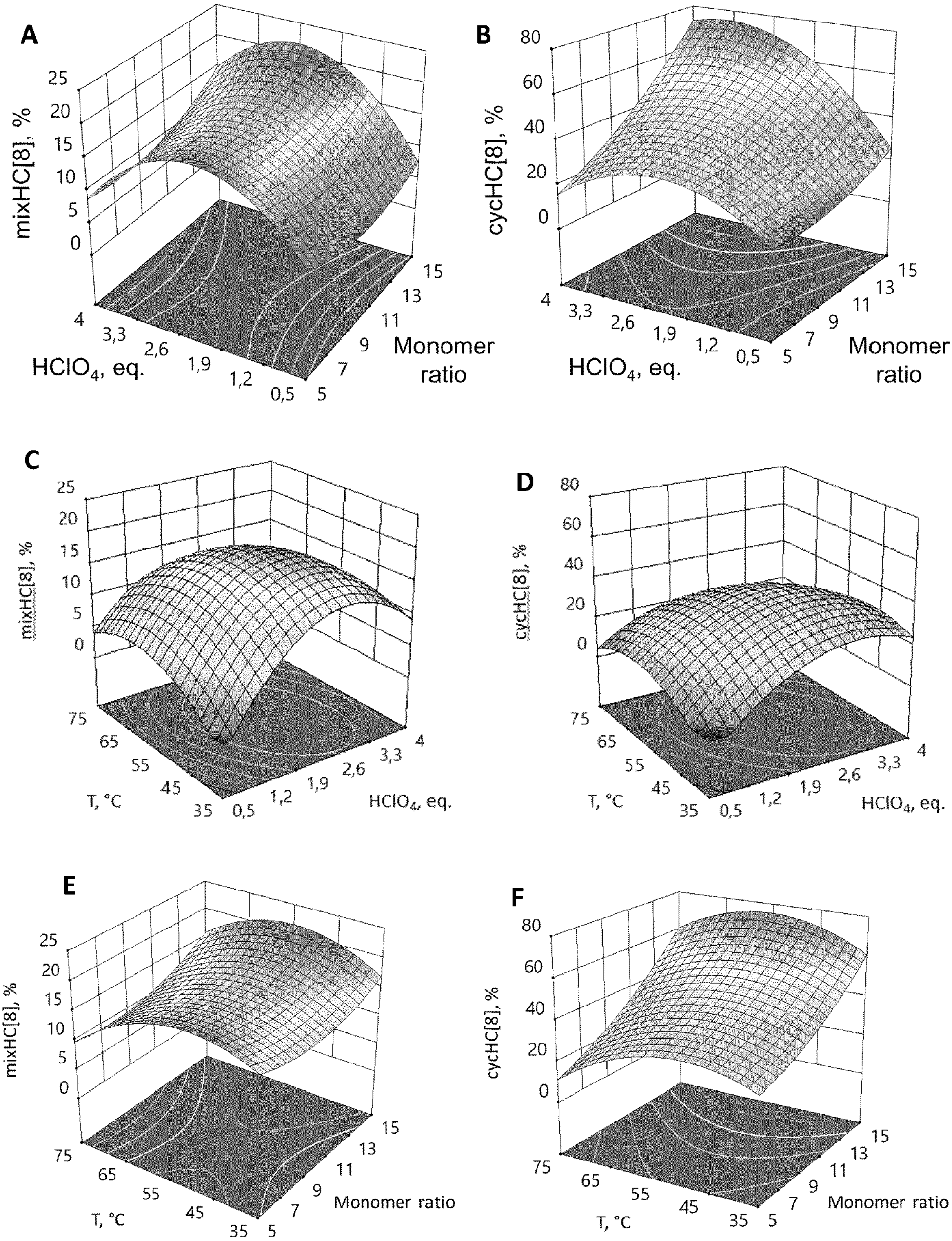
A
mixHC[8], %
25
20
15
10
5
0
4
3,3
2,6
1,9
1,2
0,5
HClO4, eq.
5
7
9
11
13
15
Monomer ratio
B
cycHC[8], %
80
60
40
20
0
HClO4, eq.
Monomer ratio
C
mixHC[8], %
T, °C
75
65
55
45
35
HClO4, eq.
D
cycHC[8], %
T, °C
HClO4, eq.
E
mixHC[8], %
T, °C
Monomer ratio
F
cycHC[8], %
T, °C
Monomer ratio

**Figure 5**

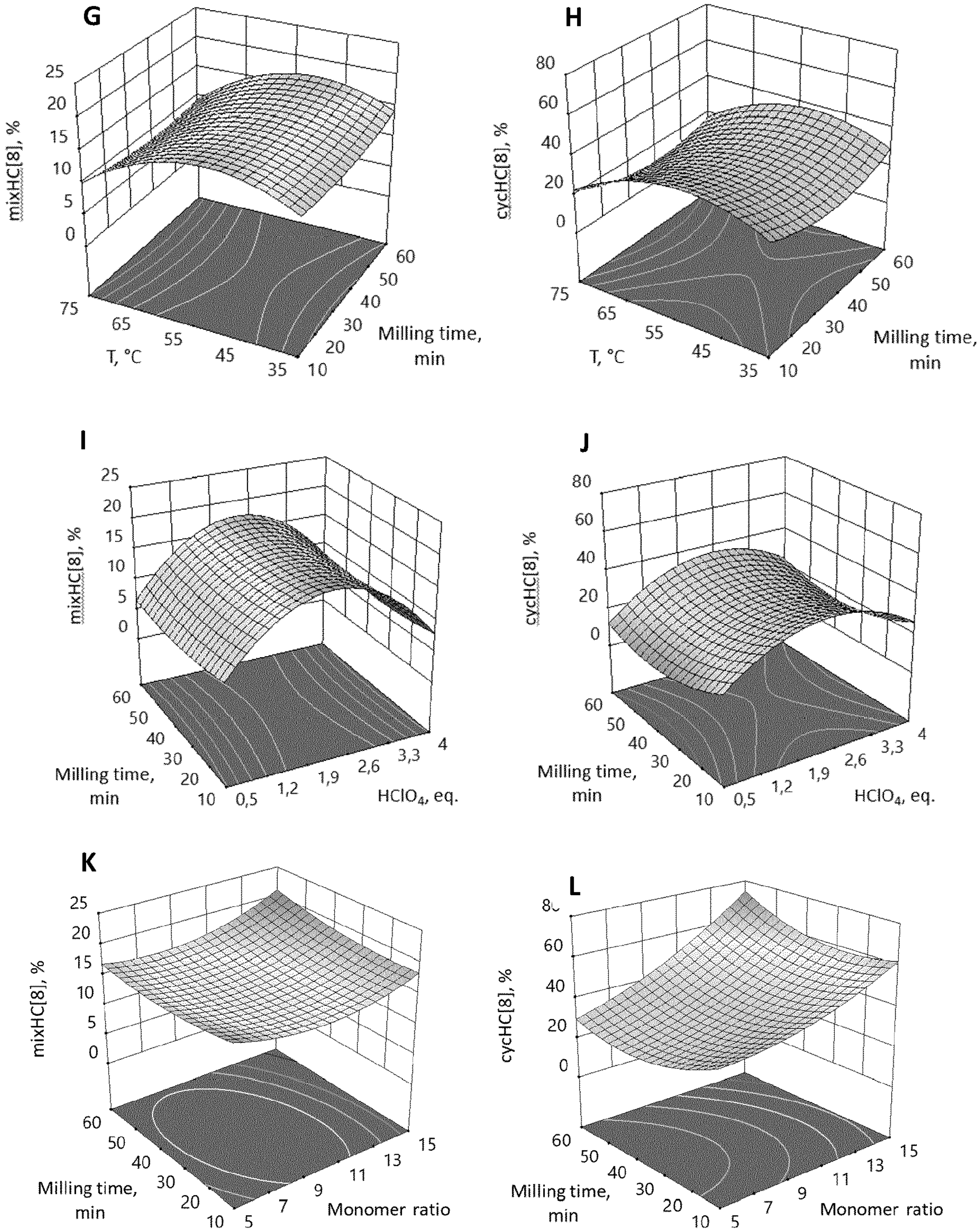


**Figure 5 (following)**

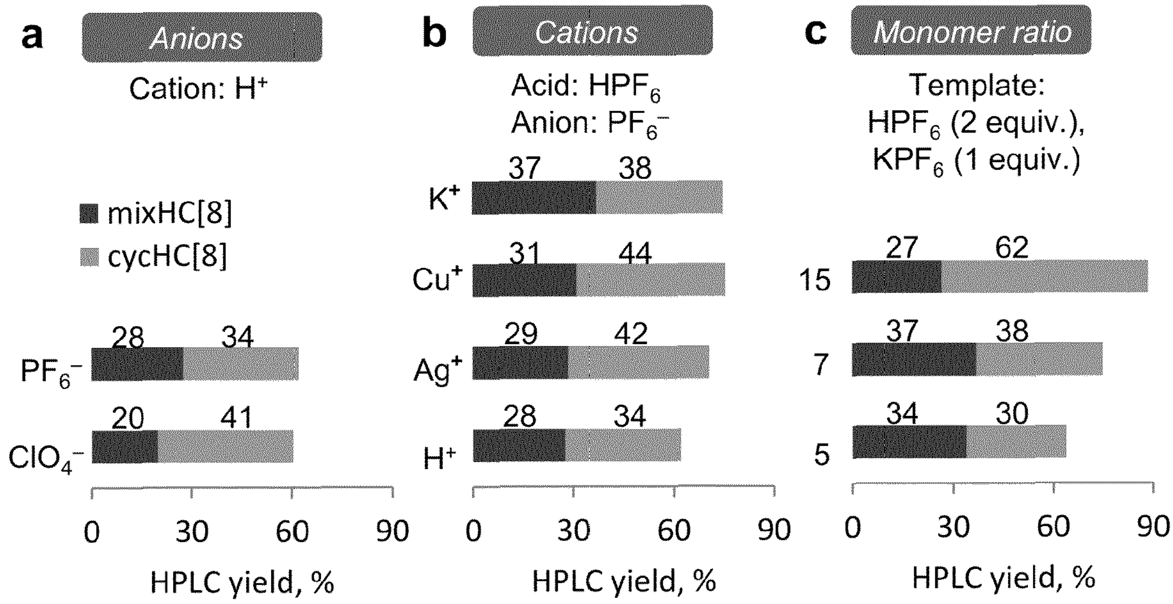


**Figure 6**

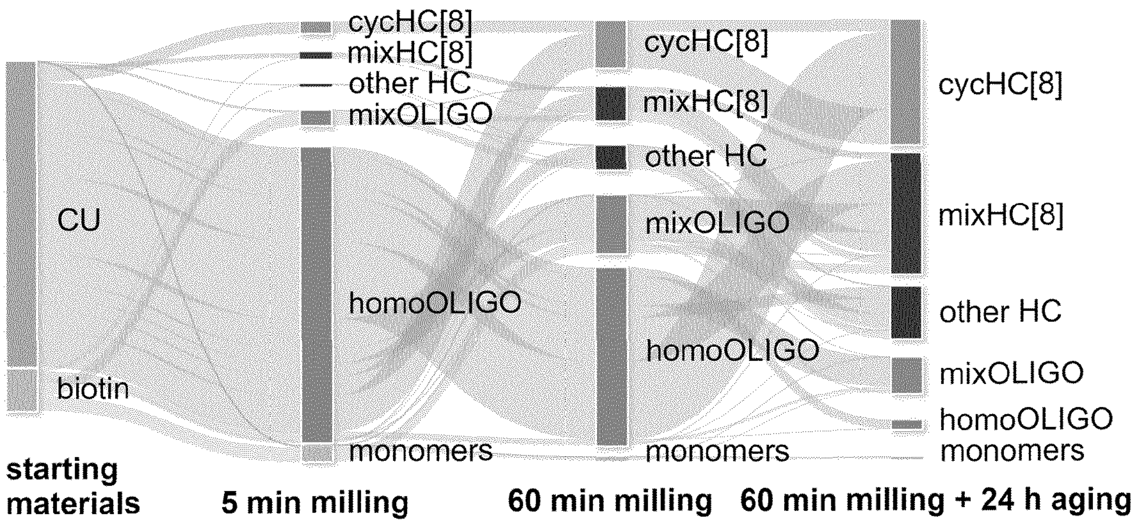


**Figure 7**

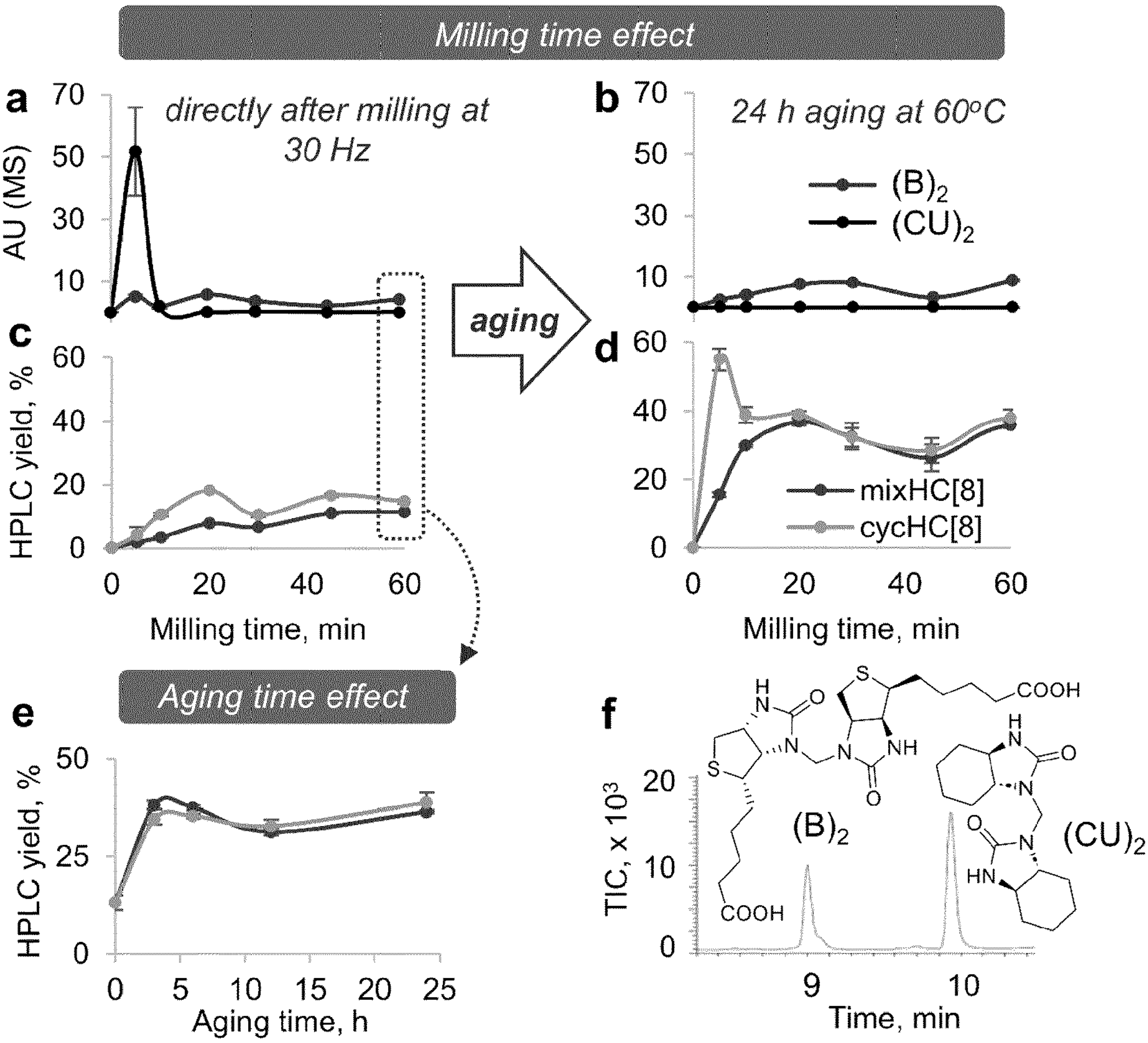
Milling time effect
a
directly after milling at 30 Hz
AU (MS)
70
50
30
10
b
24 h aging at 60°C
(B)2
(CU)2
aging
c
HPLC yield, %
60
40
20
0
Milling time, min
d
mixHC[8]
cycHC[8]
Aging time effect
e
HPLC yield, %
50
25
0
0
5
10
15
20
25
Aging time, h
f
TIC, x 10^3
(B)2
(CU)2
9
10
Time, min

**Figure 8**

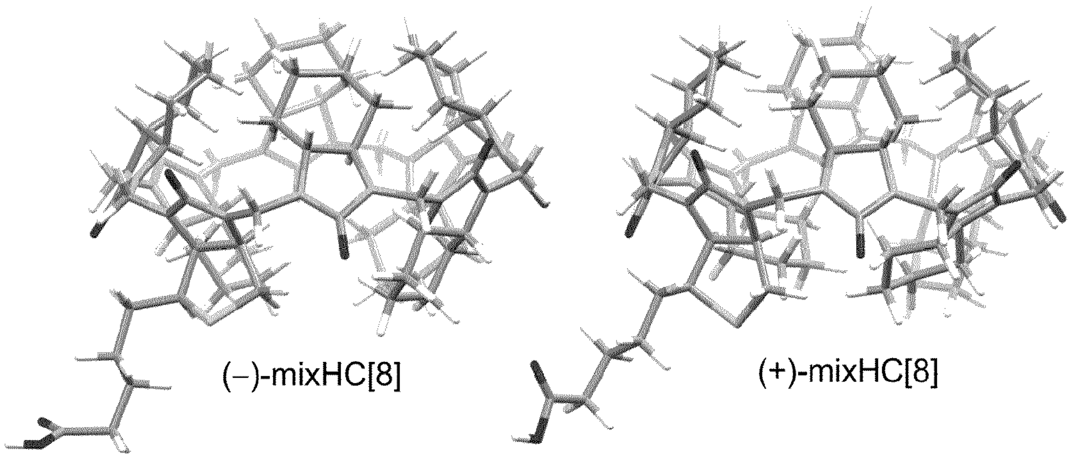
(−)-mixHC[8]
(+)-mixHC[8]

**Figure 9**

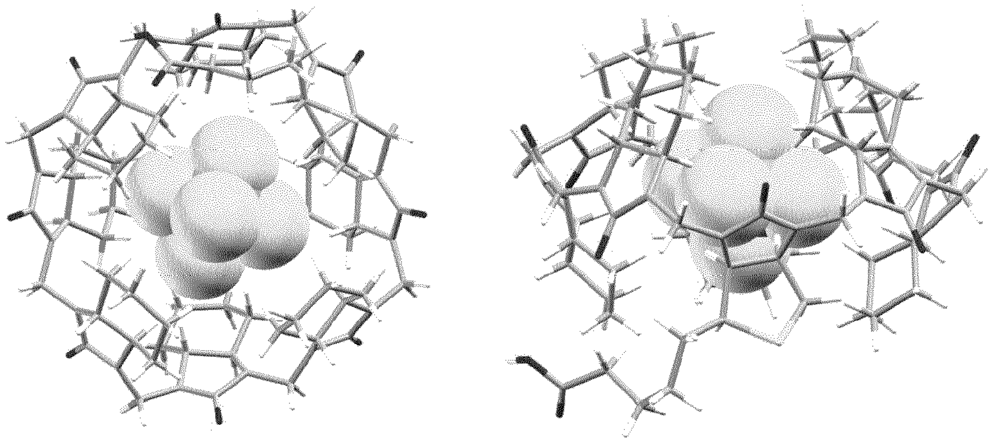

**Figure 10**

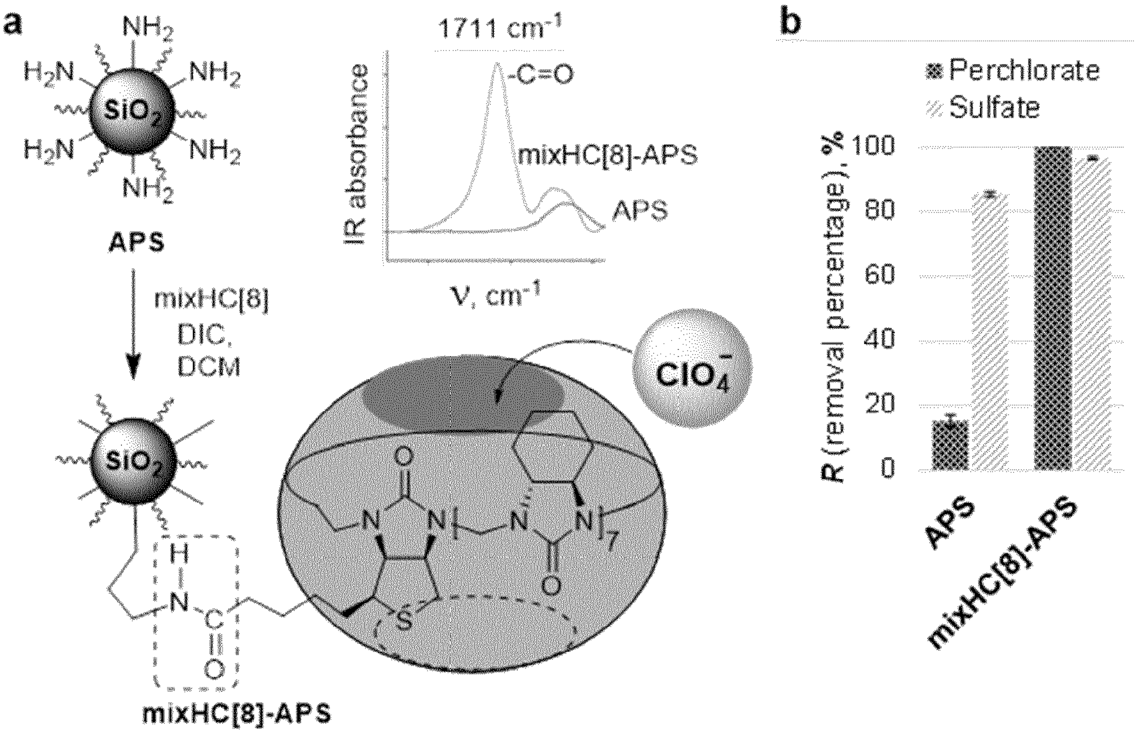
a
NH2
H2N
NH2
SiO2
H2N
NH2
NH2
APS
mixHC[8]
DIC,
DCM
1711 cm-1
-C=O
IR absorbance
mixHC[8]-APS
APS
ν, cm-1
ClO4-
SiO2
H
N
C
O
S
N
N
N
N
O
O
7
mixHC[8]-APS
b
Perchlorate
Sulfate
R (removal percentage), %
100
80
60
40
20
0
APS
mixHC[8]-APS

Figure 11

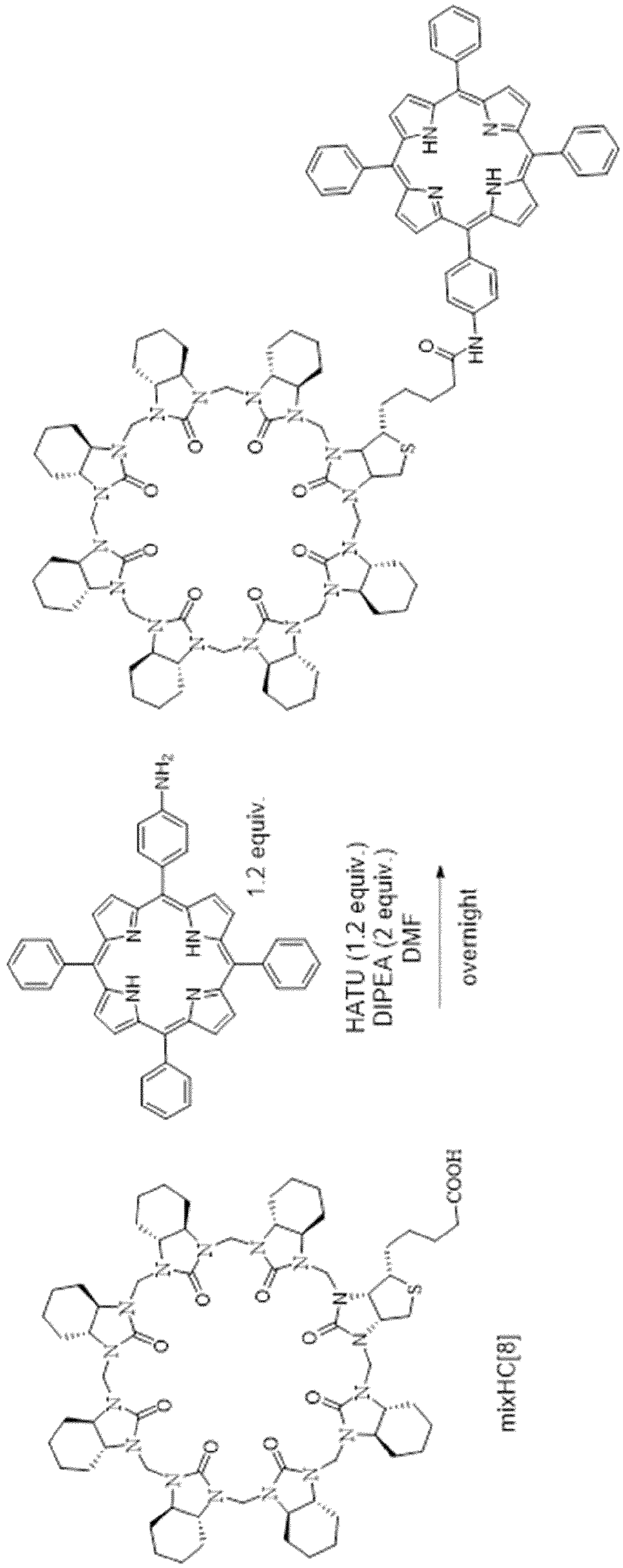


mixHC[8]

Figure 12

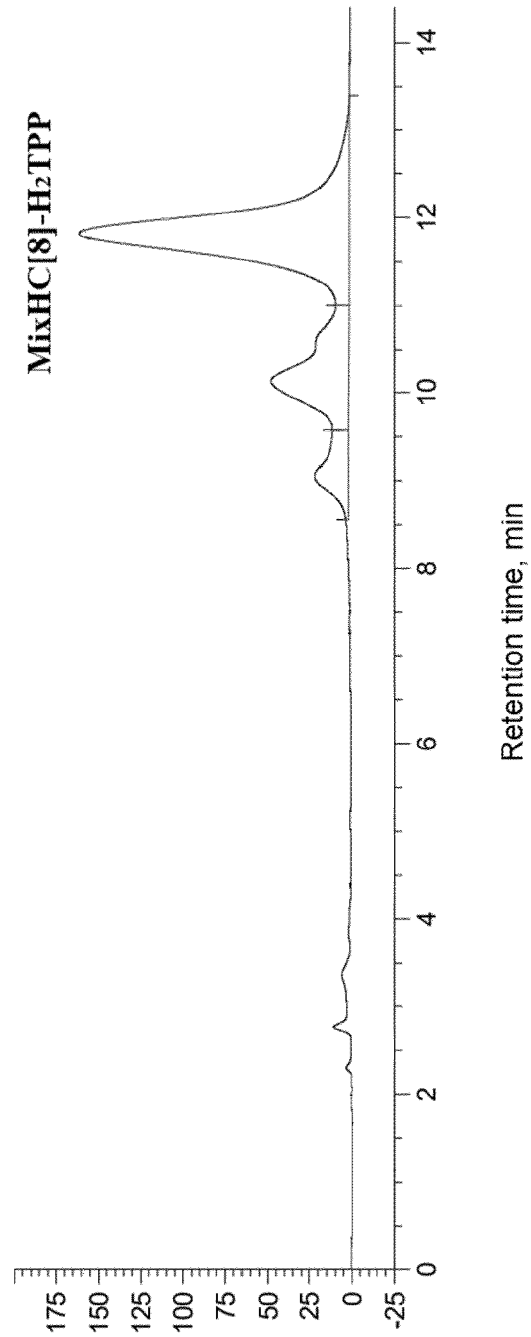
MixHC[8]-H$_2$TPP
Absorbance at 420 nm, mAU
175
150
125
100
75
50
25
0
-25
0
2
4
6
8
10
12
14
Retention time, min

Figure 13

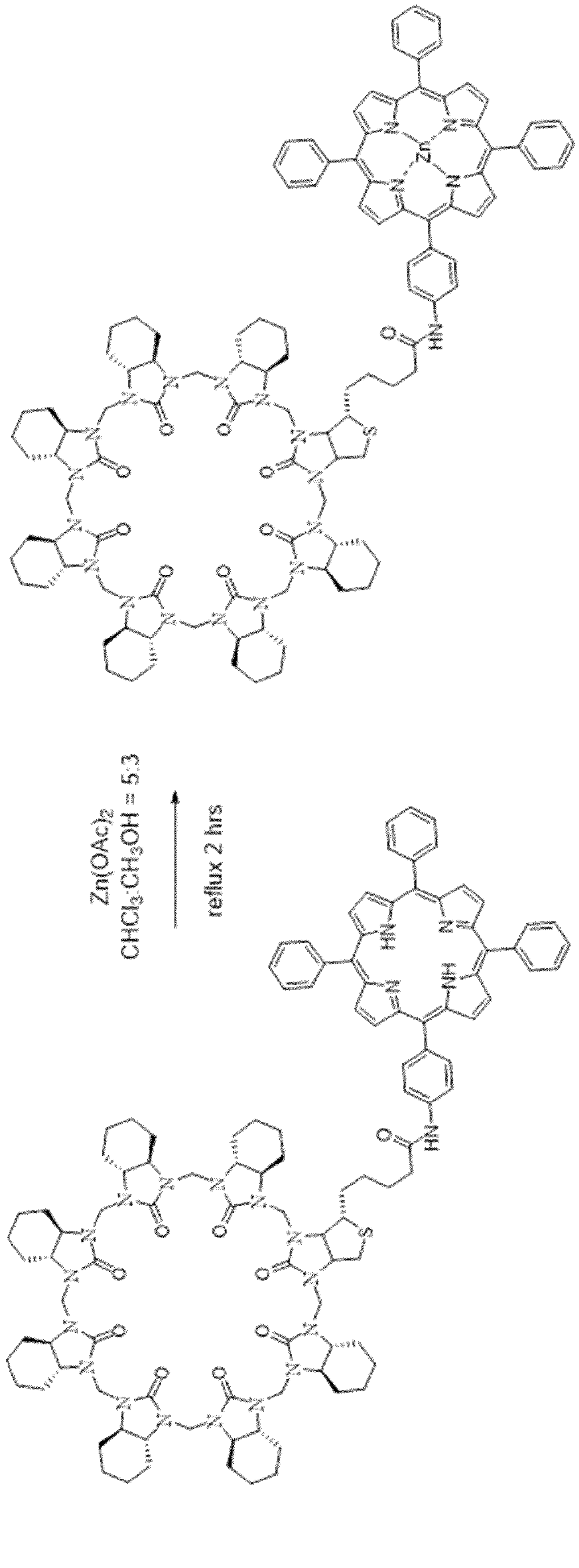


**Figure 14**

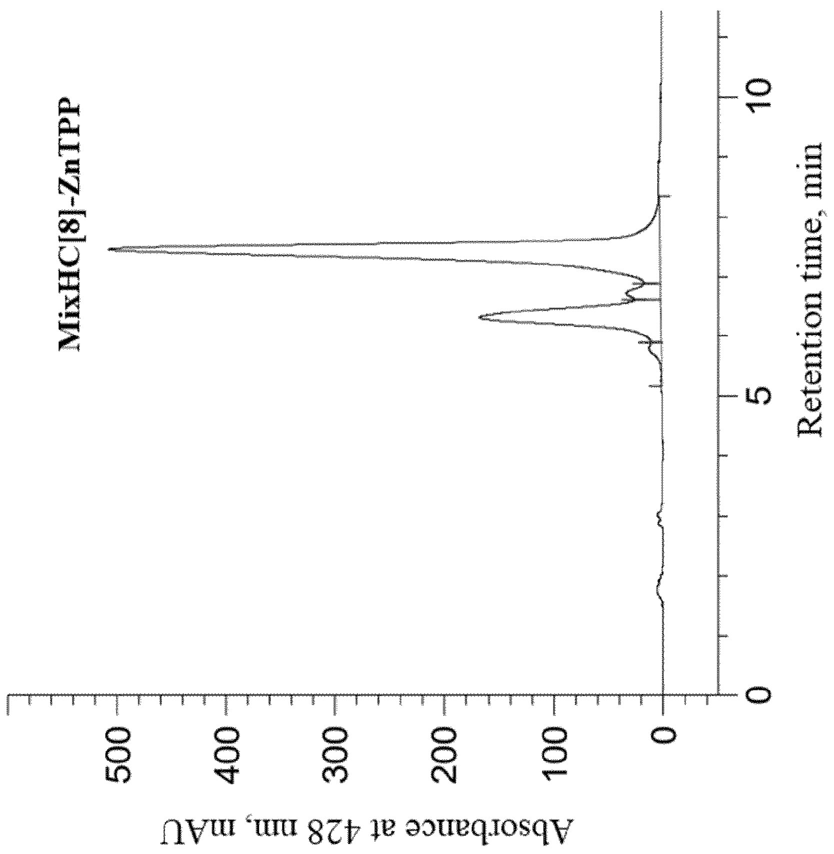
MixHC[8]-ZnTPP
Absorbance at 428 nm, mAU
500
400
300
200
100
0
0
5
10
Retention time, min

Figure 15

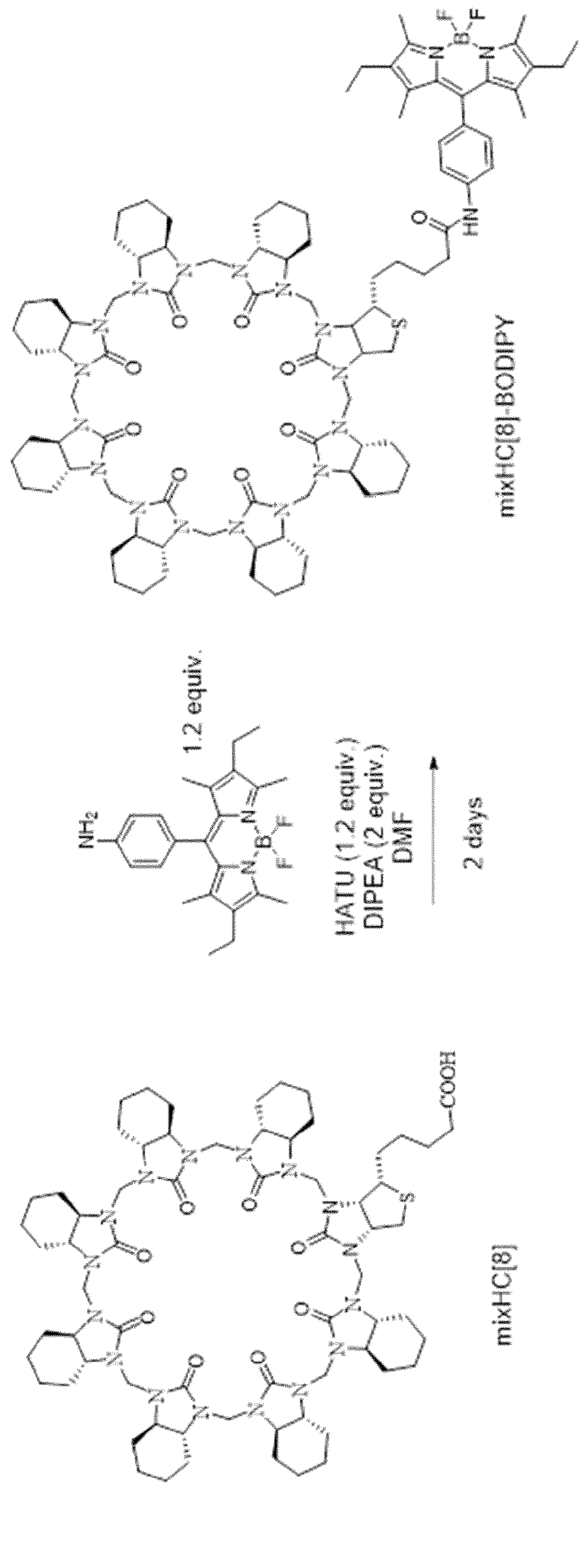


mixHC[8]

mixHC[8]-BODIPY

**Figure 16**

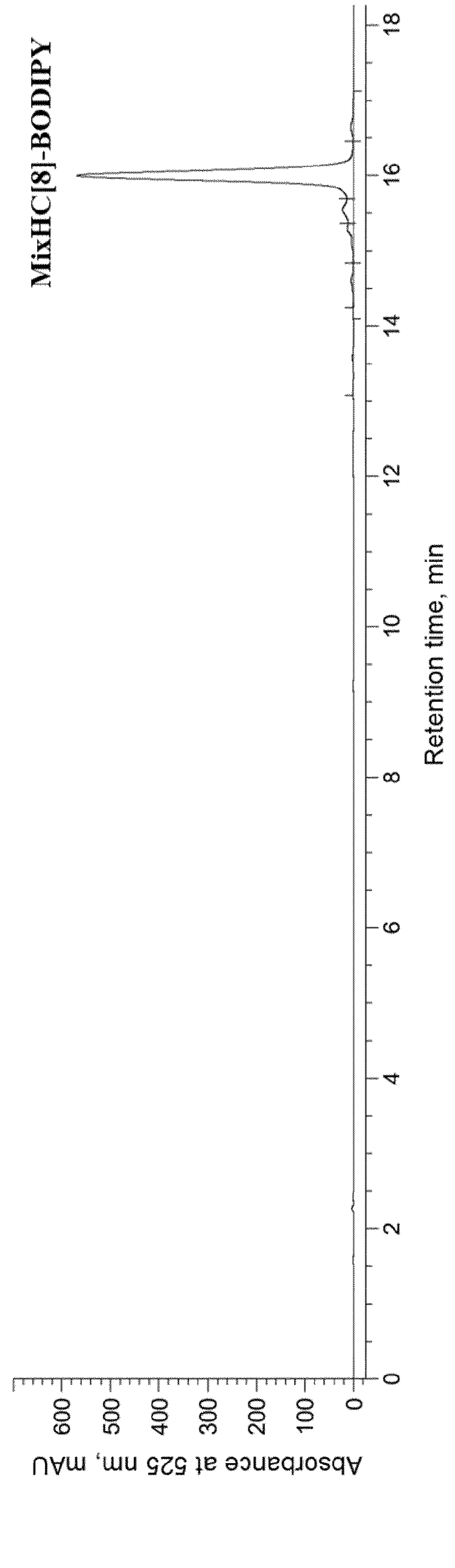
MixHC[8]-BODIPY
Absorbance at 525 nm, mAU
0
100
200
300
400
500
600
Retention time, min
0
2
4
6
8
10
12
14
16
18

Figure 17

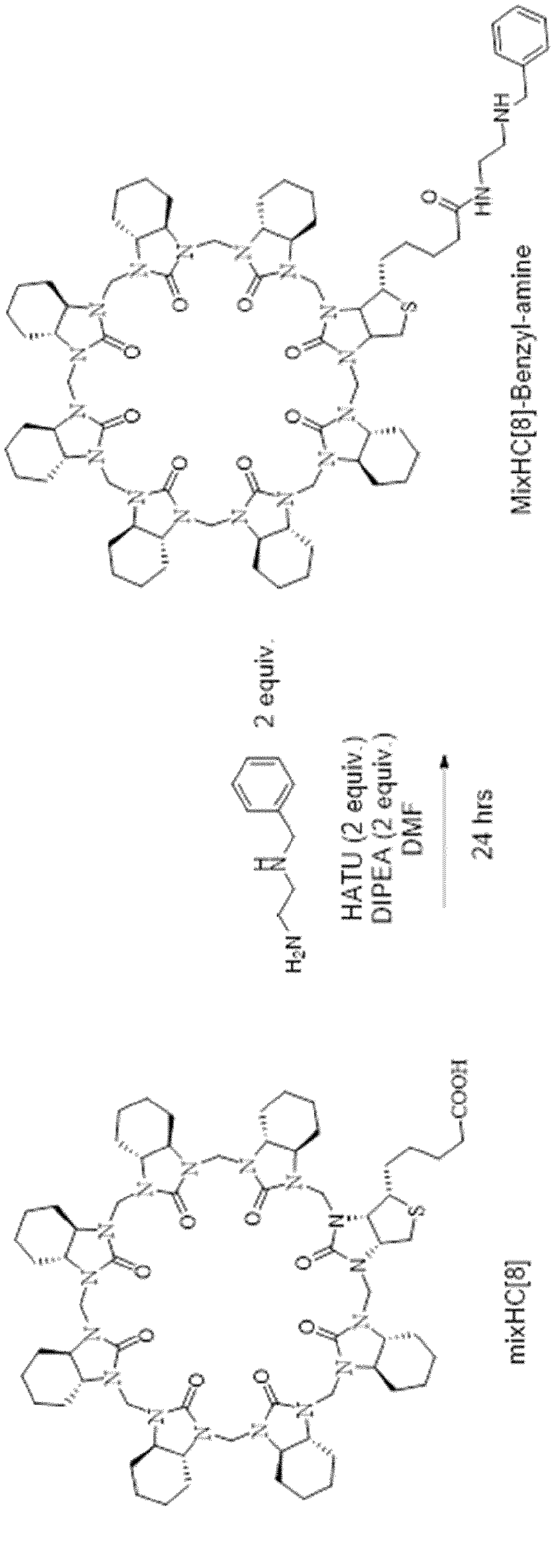


mixHC[8]

MixHC[8]-Benzyl-amine

**Figure 18**

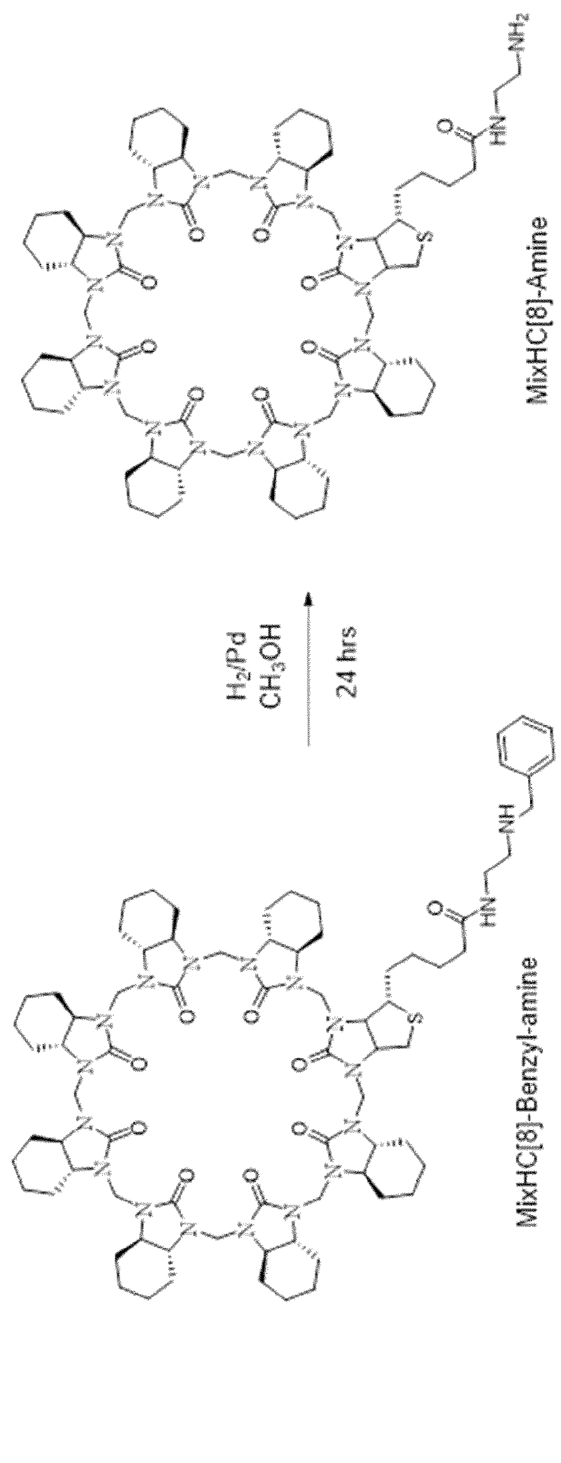


MixHC[8]-Benzyl-amine

MixHC[8]-Amine

**Figure 19**

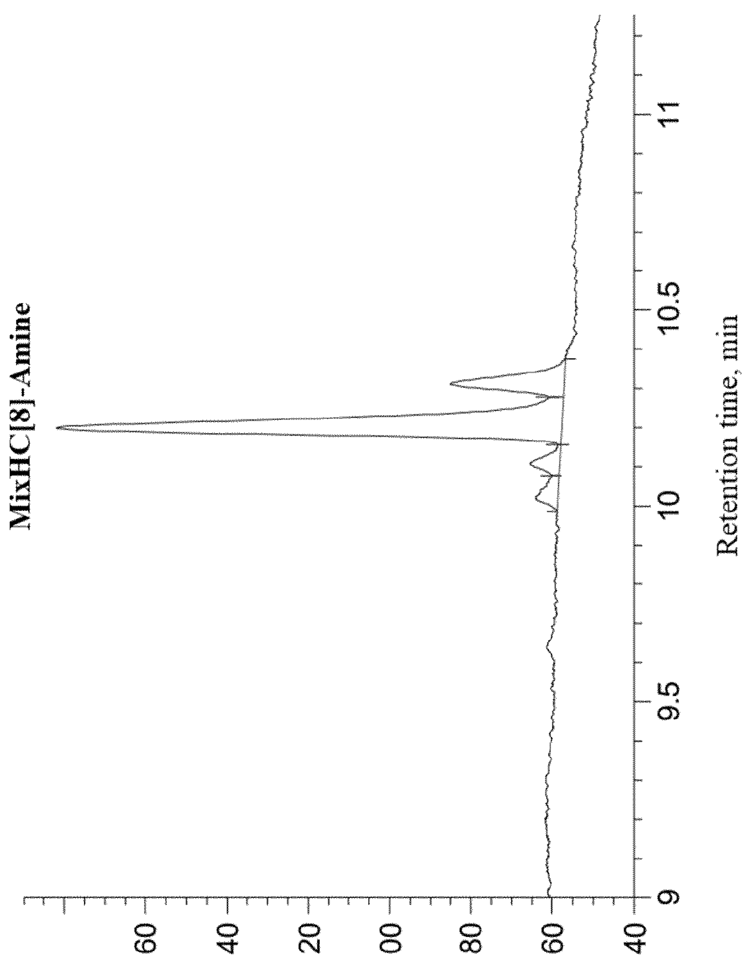
MixHC[8]-Amine
Absorbance at 210 nm, mAU
60
40
20
00
80
60
40
Retention time, min
9
9.5
10
10.5
11

Figure 20

Europäisches Patentamt
European Patent Office
Office européen des brevets

# EUROPEAN SEARCH REPORT

Application Number
EP 23 18 1344

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MARSÁLEK KAMIL ET AL: "Monofunctionalized Bambus[6]urils and Their Conjugates with Crown Ethers for Liquid-Liquid Extraction of Inorganic Salts", ORGANIC LETTERS, vol. 22, no. 4, 5 February 2020 (2020-02-05), pages 1633-1637, XP093108702, US ISSN: 1523-7060, DOI: 10.1021/acs.orglett.0c00216 | 10,14,15 | INV. C07D409/04 |
| A | * scheme 1 * | 1-9, 11-13 | |
| | ----- | | |
| X | WANG LI ET AL: "Synthesis of hybrid thiohemicucurbiturils", TETRAHEDRON LETTERS, vol. 101, 1 July 2022 (2022-07-01), page 153918, XP093108703, Amsterdam , NL ISSN: 0040-4039, DOI: 10.1016/j.tetlet.2022.153918 Retrieved from the Internet: URL:https://www.sciencedirect.com/science/article/pii/S0040403922003501/pdfft?md5=a89b5218d7936cd9409341ae714b667c&pid=1-s2.0-S0040403922003501-main.pdf> | 10 | TECHNICAL FIELDS SEARCHED (IPC) C07D |
| A | * Scheme 1 * | 1-9, 11-15 | |
| | ----- -/-- | | |

The present search report has been drawn up for all claims

1

| Place of search | Date of completion of the search | Examiner |
|---|---|---|
| Munich | 12 December 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 18 1344

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE SIMONE NICOLA ALESSANDRO ET AL: "Monofunctionalized Fluorinated Bambusurils and Their Conjugates for Anion Transport and Extraction", THE JOURNAL OF ORGANIC CHEMISTRY, vol. 87, no. 15, 21 July 2022 (2022-07-21), pages 9829-9838, XP093108696, ISSN: 0022-3263, DOI: 10.1021/acs.joc.2c00870 Retrieved from the Internet: URL:https://pubs.acs.org/doi/pdf/10.1021/acs.joc.2c00870> | 10,14,15 | |
| A | * Scheme 2 * | 1-9, 11-13 | |
| | ----- | | |
| | | | TECHNICAL FIELDS SEARCHED (IPC) |

1

The present search report has been drawn up for all claims

| Place of search | Date of completion of the search | Examiner |
|---|---|---|
| Munich | 12 December 2023 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KWON, T. et al.** *J. Am. Chem. Soc.*, 2022, vol. 144 (28), 12595-12601
- **SCHNITZER, T. et al.** *Angewandte Chemie*, 2022, vol. 134 (41)
- **KAABEL, S. et al.** *Angewandte Chemie International Edition*, 2019, vol. 58 (19), 6230-6234
- **KAABEL, S. et al.** *Israel Journal of Chemistry*, 2018, vol. 58 (3-4), 296-313
- **ANDERSEN, N. et al.** *Israel Journal of Chemistry*, 2018, vol. 58 (3-4), 435-448
- **ZENG, Q. et al.** *Org. Chem.*, 2021, vol. 17 (1), 2840-2847
- **WANG, L. et al.** *Tetrahedron Letters*, 2022, vol. 101, 153918
- **DE SIMONE et al.** *J. Org. Chem.*, 2022, vol. 87 (15), 9829-9838
- **WEISSTEIN, ERIC W.** *Necklace*
- **STOLLE, A. et al.** *Faraday Discuss.*, 2014, vol. 170, 267-286
- **SCHMIDT, R. et al.** *Org. Process Res. Dev.*, 2015, vol. 19, 427-436
- *Polym. Chem.*, 2019, vol. 10, 3674-3711
- *ACS Nano*, 2020, vol. 14 (10), 12491-12521
- **FUENTES DE ARRIBA, Á. L.** ; **SEISDEDOS, D. G.** ; **SIMÓN, L.** ; **ALCÁZAR, V.** ; **RAPOSO, C.** ; **MORÁN, J. R**. Synthesis of Monoacylated Derivatives of 1,2-Cyclohexanediamine. Evaluation of Their Catalytic Activity in the Preparation of Wieland-Miescher Ketone.. *J. Org. Chem.*, 2010, vol. 75 (23), 8303-8306, https://doi.org/10.1021/jo101723v
- **BECKE, A. D.** Density-Functional Thermochemistry. III. The Role of Exact Exchange.. *J. Chem. Phys.*, 1993, vol. 98 (7), 5648-5652, https://doi.org/10.1063/1.464913
- **LEE, C.** ; **YANG, W.** ; **PARR, R. G.** Development of the Colle-Salvetti Correlation-Energy Formula into a Functional of the Electron Density.. *Phys. Rev. B*, 1988, vol. 37 (2), 785-789, https://doi.org/10.1103/PhysRevB.37.785
- **VOSKO, S. H.** ; **WILK, L.** ; **NUSAIR, M.** Accurate Spin-Dependent Electron Liquid Correlation Energies for Local Spin Density Calculations: A Critical Analysis. *Con. J. Phys*, 1980, vol. 58 (8), 1200-1211, https://doi.org/10.1139/p80-159
- **STEPHENS, P. J.** ; **DEVLIN, F. J.** ; **CHABALOWSKI, C. F.** ; **FRISCH, M. J.** Ab Initio Calculation of Vibrational Absorption and Circular Dichroism Spectra Using Density Functional Force Fields.. *J. Phys. Chem.*, 1994, vol. 98 (45), 11623-11627, https://doi.org/10.1021/j100096a001
- **DEWAR, M. J. S.** ; **ZOEBISCH, E. G.** ; **HEALY, E. F.** ; **STEWART, J. J. P**. Development and Use of Quantum Mechanical Molecular Models. 76. AM1: A New General Purpose Quantum Mechanical Molecular Model.. *J. Am. Chem. Soc.*, 1985, vol. 107 (13), 3902-3909, https://doi.org/10.1021/ja00299a024
- **HARIHARAN, P. C.** ; **POPLE, J. A.** The Influence of Polarization Functions on Molecular Orbital Hydrogenation Energies. *Theor. Chim. Acta*, 1973, vol. 28 (3), 213-222, https://doi.org/10.1007/BF00533485
- **DITCHFIELD, R.** ; **HEHRE, W. J.** ; **POPLE, J. A.** Self-Consistent Molecular-Orbital Methods. IX. An Extended Gaussian-Type Basis for Molecular-Orbital Studies of Organic Molecules.. *J. Chem. Phys.*, 1971, vol. 54 (2), 724-728, https://doi.org/10.1063/1.1674902
- **HEHRE, W. J.** ; **DITCHFIELD, K.** ; **POPLE, J. A.** Self-Consistent Molecular Orbital Methods. XII. Further Extensions of Gaussian-Type Basis Sets for Use in Molecular Orbital Studies of Organic Molecules.. *J. Chem. Phys.*, 1972, vol. 56 (5), 2257-2261, https://doi.org/10.1063/1.1677527
- **GRIMME, S.** ; **EHRLICH, S.** ; **GOERIGK, L.** Effect of the Damping Function in Dispersion Corrected Density Functional Theory.. *J. Comput. Chem.*, 2011, vol. 32 (7), 1456-1465, https://doi.org/10.1002/jcc.21759
- **FRISCH, M. J.** ; **TRUCKS, G. W.** ; **SCHLEGEL, H. B.** ; **SCUSERIA, G. E.** ; **ROBB, M. A.** ; **CHEESEMAN, J. R.** ; **SCALMANI, G.** ; **BARONE, V.** ; **PETERSSON, G. A.** ; **NAKATSUJI, H.** *Gaussian 16 Rev. C.01*, 2016
- **SHELDRICK, G. M**. SHELXT - Integrated Space-Group and Crystal-Structure Determination. Acta Crystallogr. *Sect. Found. Adv.*, 2015, vol. 71 (1), 3-8, https://doi.org/10.1107/S2053273314026370

- **DOLOMANOV, O. V.** ; **BOURHIS, L. J.** ; **GILDEA, R. J.** ; **HOWARD, J. A. K.** ; **PUSCHMANN, H.** OLEX2: A Complete Structure Solution, Refinement and Analysis Program. *J. Appl. Crystallogr.*, 2009, vol. 42 (2), 339-341, https://doi.org/10.1107/S0021889808042726
- **SPACKMAN, M. A.** ; **JAYATILAKA, D.** Hirshfeld Surface Analysis. *CrystEngComm*, 2009, vol. 11 (1), 19-32, https://doi.org/10.1039/B818330A
- **SPACKMAN, P. R.** ; **TURNER, M. J.** ; **MCKINNON, J. J.** ; **WOLFF, S. K.** ; **GRIMWOOD, D. J.** ; **JAYATILAKA, D.** ; **SPACKMAN, M. A.** CrystalExplorer: A Program for Hirshfeld Surface Analysis, Visualization and Quantitative Analysis of Molecular Crystals.. *J. Appl. Crystallogr.*, 2021, vol. 54 (3), 1006-1011, https://doi.org/10.1107/S1600576721002910
- **SCHNITZER, T.** ; **PREUSS, M. D.** ; **VAN BASTEN, J.** ; **SCHOENMAKERS, S. M. C.** ; **SPIERING, A. J. H.** ; **VANTOMME, G.** ; **MEIJER, E. W.** How Subtle Changes Can Make a Difference: Reproducibility in Complex Supramolecular Systems.. *Angew. Chem.*, 2022, vol. 134 (41), e202206738, https://doi.org/10.1002/ange.202206738
- **YAMAOKI, Y.** ; **NAGATA, T.** ; **KONDO, K.** ; **SAKAMOTO, T.** ; **TAKAMI, S.** ; **KATAHIRA, M.** Shedding Light on the Base-Pair Opening Dynamics of Nucleic Acids in Living Human Cells.. *Nat. Commun.*, 2022, vol. 13 (1), 7143, https://doi.org/10.1038/s41467-022-34822-4
- **KWON, T.** ; **SONG, B.** ; **NAM, K. W.** ; **STODDART, J. F.** Mechanochemical Enhancement of the Structural Stability of Pseudorotaxane Intermediates in the Synthesis of Rotaxanes.. *J Am Chem Soc*, 2022, vol. 144 (28), 12595-12601, https://doi.org/10.1021/jacs.2c00515
- **KAABEL, S.** ; **AAV, R.** Templating Effects in the Dynamic Chemistry of Cucurbiturils and Hemicucurbiturils. *Isr. J. Chem.*, 2018, vol. 58 (3-4), 296-313, https://doi.org/10.1002/ijch.201700106
- **LEHN, J.-M.** Dynamic Combinatorial Chemistry and Virtual Combinatorial Libraries. *Chem. - Eur. J.*, 1999, vol. 5 (9), 2455-2463, https://doi.org/10.1002/(SICI)1521-3765(19990903)5:93.0.CO;2-H
- **CORBETT, P. T.** ; **LECLAIRE, J.** ; **VIAL, L.** ; **WEST, K. R.** ; **WIETOR, J.-L.** ; **SANDERS, J. K. M.** ; **OTTO, S.** Dynamic Combinatorial Chemistry.. *Chem. Rev.*, 2006, vol. 106 (9), 3652-3711, https://doi.org/10.1021/cr020452p
- **ANDERSEN, N. N.** ; **LISBJERG, M.** ; **ERIKSEN, K.** ; **PITTELKOW, M.** Hemicucurbit[n]Urils and Their Derivatives - Synthesis and Applications. *Isr. J. Chem.*, 2018, vol. 58 (3-4), 435-448, https://doi.org/10.1002/ijch.201700129
- **LISBJERG, M.** ; **JESSEN, B. M.** ; **RASMUSSEN, B.** ; **NIELSEN, B. E.** ; **MADSEN, A. Ø.** ; **PITTELKOW, M.** Discovery of a Cyclic 6 + 6 Hexamer of D-Biotin and Formaldehyde. *Chem. Sci.*, 2014, vol. 5 (7), 2647-2650, https://doi.org/10.1039/C4SC00990H
- **HAVEL, V.** ; **YAWER, M. A.** ; **SINDELAR, V.** Real-Time Analysis of Multiple Anion Mixtures in Aqueous Media Using a Single Receptor.. *Chem. Commun.*, 2015, vol. 51 (22), 4666-4669, https://doi.org/10.1039/C4CC10108A
- **YAWER, M. A.** ; **HAVEL, V.** ; **SINDELAR, V.** A Bambusuril Macrocycle That Binds Anions in Water with High Affinity and Selectivity.. *Angew. Chem. Int. Ed.*, 2015, vol. 54 (1), 276-279, https://doi.org/10.1002/anie.201409895
- **LANGERREITER, D.** ; **KOSTIAINEN, M. A.** ; **KAABEL, S.** ; **ANAYA-PLAZA, E.** A Greener Route to Blue: Solid-State Synthesis of Phthalocyanines.. *Angew. Chem. Int. Ed*, 2022, vol. 61 (42), e202209033, https://doi.org/10.1002/anie.202209033
- **PASCU, M.** ; **RUGGI, A.** ; **SCOPELLITI, R.** ; **SEVERIN, K.** Synthesis of Borasiloxane-Based Macrocycles by Multicomponent Condensation Reactions in Solution or in a Ball Mill.. *Chem. Commun.*, 2012, vol. 49 (1), 45-47, https://doi.org/10.1039/C2CC37538A
- **SIM Y.;** ; **SHI, Y. X.** ; **GANGULY, R.** ; **LI Y.;** ; **GARCIA, F.** Mechanochemical Synthesis of Phosphazane-Based Frameworks. *Chem. - Eur. J.*, 2017, vol. 23 (47), 11279-11285, https://doi.org/10.1002/chem.201701619
- **XI, H.-T.** ; **ZHAO, T.** ; **SUN, X.-Q.** ; **MIAO, C.-B.** ; **ZONG, T.** ; **MENG, Q.** Rapid and Efficient Solvent-Free Synthesis of Cyclophanes Based on Bipyridinium under Mechanical Ball Milling. *RSC Adv.*, 2012, vol. 3 (3), 691-694, https://doi.org/10.1039/C2RA22802E
- **SHY, H.** ; **MACKIN, P.** ; **ORVIETO, A. S.** ; **GHARBHARAN, D.** ; **PETERSON, G. R.** ; **BAMPOS, N.** ; **HAMILTON, T. D**. The Two-Step Mechanochemical Synthesis of Porphyrins. *Faraday Discuss.*, 2014, vol. 170 (0), 59-69, https://doi.org/10.1039/C3FD00140G
- **SU, Q.** ; **HAMILTON, T. D**. Extending Mechanochemical Porphyrin Synthesis to Bulkier Aromatics: Tetramesitylporphyrin. *Beilstein J. Org. Chem.*, 2019, vol. 15 (1), 1149-1153, https://doi.org/10.3762/bjoc.15.111
- **ZENG, Q.** ; **LONG, Q.** ; **LU, J.** ; **WANG, L.** ; **YOU, YUAN, X.** ; **ZHANG, Q.** ; **GE, Q.** ; **CONG, H.** ; **LIU, M.** Synthesis of a Novel Aminobenzene-Containing Hemicucurbituril and Its Fluorescence Spectral Properties with Ions. *Beilstein J. Org. Chem.*, 2021, vol. 17 (1), 2840-2847, https://doi.org/10.3762/bjoc.17.195
- **WANG, L.** ; **HAN, J.** ; **PAN, R.** ; **YUAN, X.** ; **YOU, CEN, X.** ; **ZHANG, Q.** ; **GE, Q.** ; **CONG, H.** ; **LIU, M.** Synthesis of Hybrid Thiohemicucurbiturils.. *Tetrahedron Lett.*, 2022, vol. 101, 153918, https://doi.org/10.1016/j.tetlet.2022.153918

- **MARTI-CENTELLES, V.** ; **PANDEY, M. D.** ; **BURGUETE, M. I.** ; **LUIS, S. V.** Macrocyclization Reactions: The Importance of Conformational, Configurational, and Template-Induced Preorganization.. *Chem. Rev.*, 2015, vol. 115 (16), 8736-8834, https://doi.org/10.1021/acs.chemrev.5b00056
- **BELENGUER, A. M.** ; **LAMPRONTI, G. I.** ; **DE MITRI, N.** ; **DRIVER, M.** ; **HUNTER, C. A.** ; **SANDERS, J. K. M.** Understanding the Influence of Surface Solvation and Structure on Polymorph Stability: A Combined Mechanochemical and Theoretical Approach.. *J. Am. Chem. Soc.*, 2018, vol. 140 (49), 17051-17059, https://doi.org/10.1021/jacs.8b08549
- **LIZAL, T.** ; **SINDELAR, V**. Bambusuril Anion Receptors. *Isr. J. Chem.*, 2018, vol. 58 (3-4), 326-333, https://doi.org/10.1002/ijch.201700111
- **MYERS, R. H.** ; **MONTGOMERY, D. C.** ; **ANDERSON-COOK, C. M.** Response Surface Methodology: Process and Product Optimization Using Designed Experiments. John Wiley & Sons, 2016
- **URBANSKY, E. T.** Perchlorate as an Environmental Contaminant.. *Environ. Sci. Pollut. Res.*, 2002, vol. 9 (3), 187-192, https://doi.org/10.1007/BF02987487
- **KUMARATHILAKA, P.** ; **OZE, C.** ; **INDRARATNE, S. P.** ; **VITHANAGE, M.** Perchlorate as an Emerging Contaminant in Soil, Water and Food. *Chemosphere*, 2016, vol. 150, 667-677
- **SIGEL, H.** ; **SCHELLER, K. H.** Metal Ion Complexes of D-Biotin in Solution. Stability of the Stereoselective Thioether Coordination.. *J. Inorg. Biochem.*, 1982, vol. 16 (4), 297-310, https://doi.org/10.1016/S0162-0134(00)80266-4
- **AOKI, K.** ; **SAENGER, W.** Interactions of Biotin with Metal Ions. X-Ray Crystal Structure of the Polymeric Biotin-Silver(i) Nitrate Complex: Metal Bonding to Thioether and Ureido Carbonyl Groups.. *J. Inorg. Biochem.*, 1983, vol. 19 (3), 269-273, https://doi.org/10.1016/0162-0134(83)85031-4
- **ALTAF, M.** ; **STOECKLI-EVANS, H.** Chiral One- and Two-Dimensional Silver(I)-Biotin Coordination Polymers. *Acta Crystallogr. C*, 2013, vol. 69 (2), 127-137, https://doi.org/10.1107/S0108270113000322
- **LEHN, J.-M.** Perspectives in Chemistry-Steps towards Complex Matter.. *Angew. Chem. Int. Ed.*, 2013, vol. 52 (10), 2836-2850, https://doi.org/10.1002/anie.201208397
- **MCELROY, C. R.** ; **CONSTANTINOU, A.** ; **JONES, L. C.** ; **SUMMERTON, L.** ; **CLARK, J. H**. Towards a Holistic Approach to Metrics for the 21st Century Pharmaceutical Industry.. *Green Chem.*, 2015, vol. 17 (5), 3111-3121, https://doi.org/10.1039/C5GC00340G
- **REANY, O.** ; **MOHITE, A.** ; **KEINAN, E.** Hetero-Bambusurils. *Isr. J. Chem*, 2018, vol. 58 (3-4), 449-460, https://doi.org/10.1002/ijch.201700138
- **LISBJERG, M.** ; **NIELSEN, B. E.** ; **MILHØJ, B. O.** ; **SAUER, S. P. A.** ; **PITTELKOW, M.** Anion Binding by Biotin[6]Uril in Water.. *Org. Biomol. Chem.*, 2014, vol. 13 (2), 369-373, https://doi.org/10.1039/-C4OB02211D
- **ANDERSEN, N. N.** ; **ERIKSEN, K.** ; **LISBJERG, M.** ; **OTTESEN, M. E.** ; **MILHØJ, B. O.** ; **SAUER, S. P. A.** ; **PITTELKOW, M.** Entropy/Enthalpy Compensation in Anion Binding: Biotin[6]Uril and Biotin-I-Sulfoxide[6] Uril Reveal Strong Solvent Dependency.. *J. Org. Chem.*, 2019, vol. 84 (5), 2577-2584, https://doi.org/10.1021/acs.joc.8b02797
- **ASSAF, K. I.** ; **NAU, W. M.** The Chaotropic Effect as an Assembly Motif in Chemistry.. *Angew. Chem. Int. Ed.*, 2018, vol. 57 (43), 13968-13981, https://doi.org/10.1002/anie.201804597
- **SPACKMAN, M. A.** ; **JAYATILAKA, D**. Hirshfeld Surface Analysis. *CrystEngComm*, 2009, vol. 11 (1), 19-32, https://doi.org/10.1039/B818330A
- **CALDERÓN, R.** ; **PALMA, P.** ; **ARANCIBIA-MIRANDA, N.** ; **KIM, U.-J.** ; **SILVA-MORENO, E.** ; **KANNAN, K.** Occurrence, Distribution and Dynamics of Perchlorate in Soil, Water, Fertilizers, Vegetables and Fruits and Associated Human Exposure in Chile. *Environ. Geochem. Health*, 2022, vol. 44 (2), 527-535, https://doi.org/10.1007/s10653-020-00680-6
- **CHEN, ZHU, Z.** ; **WU, X.** ; **ZHANG, D.** ; **TONG, J.** ; **LIN, YIN, L.** ; **LI, X.** ; **ZHENG, Q.** ; **LU, S.** A Nationwide Investigation of Perchlorate Levels in Staple Foods from China: Implications for Human Exposure and Risk Assessment.. *J. Hazard. Mater.*, 2022, vol. 439, 129629, https://doi.org/10.1016/j.jhazmat.2022.129629
- **DAVILA, A. F.** ; **WILLSON, D.** ; **COATES, J. D.** ; **MCKAY, C. P.** Perchlorate on Mars: A Chemical Hazard and a Resource for Humans. *Int. J. Astrobiol.*, 2013, vol. 12 (4), 321-325, https://doi.org/10.1017/S1473550413000189
- **GAYEN, P.** ; **SANKARASUBRAMANIAN, S.** ; **RAMANI, V. K.** Fuel and Oxygen Harvesting from Martian Regolithic Brine.. *Proc. Natl. Acad. Sci.*, 2020, vol. 117 (50), 31685-31689, https://doi.org/10.1073/pnas.2008613117
- **TABAKCI, M.** Immobilization of Calix[6]Arene Bearing Carboxylic Acid and Amide Groups on Aminopropyl Silica Gel and Its Sorption Properties for Cr(VI).. *J. Incl. Phenom. Macrocycl. Chem.*, 2008, vol. 61 (1), 53-60, https://doi.org/10.1007/s10847-007-9392-2
- **CANNON, K. M.** ; **BRITT, D. T.** ; **SMITH, T. M.** ; **FRITSCHE, R. F.** ; **BATCHELDOR, D.** Mars Global Simulant MGS-1: A Rocknest-Based Open Standard for Basaltic Martian Regolith Simulants. *Icarus*, 2019, vol. 317, 470-478, https://doi.org/10.1016/j.icarus.2018.08.019